# EUROPEAN PATENT APPLICATION

(11) **EP 4 556 047 A1**
(43) Date of publication of application: **21.05.2025**
(21) Application number: 23838872.2
(22) Date of filing: 07.07.2023
(51) Int. Cl.: A61M 25/01

(54) **CATHETER IMPLANT SYSTEM, AND INCLUDED ASSOCIATED DEVICE, PROSTHETIC HEART VALVES AND METHOD**

(30) Priority: 13.07.2022 CN 202210828221; 13.07.2022 CN 202210828216; 02.12.2022 US 202263429560 P; 03.03.2023 CN 202310226501; 06.03.2023 CN 202310229828; 07.04.2023 CN 202310391496; 16.06.2023 CN 202310722355
(71) Applicant: Venus MedTech (HangZhou) Inc., Hangzhou, Zhejiang 310051 (CN)
(72) Inventor: WANG, Xiang, Hangzhou, Zhejiang 310051 (CN); WANG, Jian, Hangzhou, Zhejiang 310051 (CN); ZENG, Min Frank, Hangzhou, Zhejiang 310051 (CN); ZHOU, Bin, Hangzhou, Zhejiang 310051 (CN); YANG, Lingfeng, Hangzhou, Zhejiang 310051 (CN); HU, Xiang, Hangzhou, Zhejiang 310051 (CN); YANG, Yuanfeng, Hangzhou, Zhejiang 310051 (CN); QI, Jesse Jun, Hangzhou, Zhejiang 310051 (CN); PHAM, Trinh Van, Hangzhou, Zhejiang 310051 (CN); JIN, Wenchao, Hangzhou, Zhejiang 310051 (CN); DING, Zhenjun, Hangzhou, Zhejiang 310051 (CN); HOU, Zhiyuan, Hangzhou, Zhejiang 310051 (CN); DONG, Qun, Hangzhou, Zhejiang 310051 (CN)
(74) Representative: Zaboliene, Reda
(86) International application number: PCT/CN2023/106397
(87) International publication number: WO 2024/012380

(57) **Abstract**

The present disclosure relates to the technical field of medical devices, and in particular to catheter implant systems and included associated device, prosthetic heart valves and method. The interventional delivery system included includes a catheter assembly and a control handle connected to the catheter assembly, the catheter assembly has opposing distal and proximal ends and includes an inner sheath and an outer sheath slidably engaged with each other, the outer sheath includes a loading section and a sheath section in sequence from the distal end to the proximal end, the loading section is configured to enclose and keep the interventional device in a compressed state; the control handle includes a main handle and an auxiliary handle slidably fitted over the inner sheath, the proximal end of the inner sheath is connected to the main handle and the proximal end of the outer sheath is connected to the auxiliary handle.

## Description

### TECHNICAL FIELD

The present application relates to the technical field of medical devices, and in particular to catheter implant systems and included associated device, prosthetic heart valves and method.

### DESCRIPTION OF THE PRIOR ART

In the prior art, a transcatheter implant system mainly includes a prosthetic implant and an interventional delivery system for delivering the prosthetic implant into the body of a subject. Common prosthetic implants include prosthetic heart valves, which may be prosthetic aortic valves, prosthetic pulmonary valves, prosthetic mitral valves, prosthetic tricuspid valves, etc., depending on their specific application sites. The prosthetic heart valve is usually compressed to a smaller diameter outside the body and then delivered to the intended position using an interventional delivery system.

The existing interventional delivery system mainly includes a control handle, a catheter assembly, a locking wire structure, a deflection assembly and other functional components. The control handle operates the catheter assembly to deliver the prosthetic implant loaded in the catheter assembly to the lesion site. The deployment of the prosthetic implant can be achieved by self-expansion or by expanding the prosthetic implant outward from within using a balloon catheter. The interventional delivery system includes a plurality of controlled components, and the distal ends of the controlled components cooperate with each other to manipulate the prosthetic heart valve, such as releasing, retrieving, locking in position, and adjusting spatial orientation.

In the balloon expansion method, precise fine-tuning of the movement of the outer sheath is not strictly required. However, improvements in the control handle are needed to enhance the flexibility of outer sheath operation. Additionally, in some balloon-expansion procedures, the prosthetic implant may experience displacement or misalignment during the early stages of deployment, posing potential safety risks. Some existing solutions in the prior art have attempted to address these issues, but their effectiveness remains suboptimal.

### SUMMARY OF THE DISCLOSURE

The present disclosure provides an interventional delivery system that enhances the flexibility of outer sheath operation.

The present disclosure provides an interventional delivery system, including a catheter assembly and a control handle connected to the catheter assembly, wherein the catheter assembly has a distal end and a proximal end opposite to each other and includes an inner sheath and an outer sheath slidably engaged with each other, the outer sheath includes a loading section and a sheath section sequentially from a distal end to a proximal end, and the loading section is configured to enclose a prosthetic implant and keep the prosthetic implant in a compressed state;
The control handle includes:
a main handle, to which a proximal end of the inner sheath is connected; and
an auxiliary handle, slidably fitted over the inner sheath, to which the proximal end of the outer sheath is connected.

The following provides several optional implementations, which are not additional limitations to the overall solution but serve as further supplements or optimizations. Provided there are no technical or logical conflicts, each optional implementation can be independently combined with the overall solution or combined with other optional implementations.

Optionally, the auxiliary handle includes:
a housing having an axial direction, with a distal end and a proximal end opposite to each other in the axial direction, the housing having an axially through threading channel;
a connecting sleeve fixed in the housing and arranged around the threading channel, with a distal end of the connecting sleeve configured for insertion of the outer sheath;
a positioning member movably mounted in the housing, the positioning member having a locked position for restricting the threading channel and an unlocked position for releasing the threading channel; and
a driving member which is in transmission cooperation with the positioning member to drive the positioning member to switch positions.

Optionally, the housing is formed by separate pieces in a snap-fit structure, with an engagement and positioning structure provided between the inner wall of the housing and the outer circumference of the connecting sleeve.

Optionally, the housing includes two half-shells snapped with each other in the radial direction of the connecting sleeve, and end caps respectively located at the axial ends of the two half-shells. The end caps are fixed to the two half-shells via either a snap-fit or a threaded connection.

Optionally, the sliding range of the auxiliary handle relative to the main handle is in the range of 10 to 40 cm.

Optionally, a side wall of the connecting sleeve defines a first vent hole in communication with the threading channel, a first pipe joint is fixedly connected to the first vent hole, the first vent hole and the first pipe joint define a first vent channel, and a one-way valve core is provided in the first vent channel.

Optionally, a first annular seat is arranged around the first vent hole, and the first pipe joint is inserted and mated with the first annular seat, with an anti-rotation structure provided between the first pipe joint and the first annular seat.

Optionally, a first protective sleeve arranged around the first pipe joint is embedded in the housing, and a pipeline insertion gap is defined between an outer wall of the first pipe joint and an inner wall of the first protective sleeve.

Optionally, the anti-rotation structure includes:
a slot extending in an insertion direction of the first pipe joint, wherein the slot is provided on a side wall of one of the first pipe joint and the first annular seat; and
a positioning rib engaging with the slot, wherein the positioning rib is provided on a side wall of the other of the first pipe joint and the first annular seat.

The present disclosure further provides a vent structure on a control handle. The control handle is provided with a first vent hole, a first pipe joint is fixedly connected to the first vent hole, the first vent hole and the first pipe joint define a first vent channel, and a one-way valve core is provided in the first vent channel.

The one-way valve core can be applied to the auxiliary handle. For example, optionally, the one-way valve core has a sealed state and an open state, and the one-way valve core specifically includes:
a limiting rod which is slidably mounted in an inner cavity of the first pipe joint, with a fluid gap between an outer periphery of the limiting rod and an inner wall of the first pipe joint; and
a sealing sheet connected to the limiting rod, which closes the fluid gap in the sealed state and opens the fluid gap in the open state.

Optionally, the inner wall of the first pipe joint is provided with a retaining ring surrounding the outer periphery of the limiting rod, and the fluid gap is located between an outer periphery of the retaining ring and the inner wall of the first pipe joint;
The end of the limiting rod away from the sealing sheet passes through the retaining ring and has a head; in the open state, the head abuts against the retaining ring, and the sealing sheet is away from the retaining ring and opens the fluid gap; in the sealed state, the head is away from the retaining ring, and the sealing sheet abuts against the retaining ring and closes the fluid gap.

Optionally, the inner wall of the first pipe joint is provided with a retaining ring surrounding the outer periphery of the limiting rod, and the fluid gap is located between an outer periphery of the retaining ring and the inner wall of the first pipe joint;
The end of the limiting rod away from the sealing sheet passes through the retaining ring and has a head restricted by the retaining ring, the head and the sealing sheet are fixed on two opposite sides of the retaining ring, and an edge of the sealing sheet is elastically deformable to open or close the fluid gap.

Optionally, the positioning member includes an elastic claw, one end of the elastic claw is fixed to the connecting sleeve, and the other end is a free end that is capable of swinging in a radial direction of the connecting sleeve.

Optionally, the elastic claw and the connecting sleeve are formed in one piece.

Optionally, there are two to six (for example, four) elastic claws distributed in a circumferential direction of the connecting sleeve.

Optionally, the driving member is cylindrical and slidably fitted over the connecting sleeve, and the inner wall of the driving member acts on the elastic claws.

Optionally, in the radial direction of the connecting sleeve, an outer side of the elastic claw is provided with a guide slope, and an inner wall of the driving member acts on the guide slope.

Optionally, an elastic liner is provided inside the connecting sleeve, and the elastic claw acts on an outer wall of the elastic liner to restrict the threading channel by compressing the elastic liner.

Optionally, a distal end of the elastic liner extends out of the connecting sleeve, and the extended portion is provided with a radially outwardly protruding positioning step; in the axial direction, the positioning step is axially positioned and matched with an end face of the connecting sleeve and/or an inner wall of the housing.

Optionally, a guiding structure is provided between an inner wall of the driving member and an outer wall of the connecting sleeve.

The guiding structure includes:
a slide groove defined on one of the inner wall of the driving member and the outer wall of the connecting sleeve; and
a guide rib engaging with the slide groove and provided on the other of the inner wall of the driving member and the outer wall of the connecting sleeve.

Optionally, an operation button is provided on the outer wall of the driving member, and a first clearance opening corresponding to the position of the operation button is opened on the housing.

Optionally, the side wall of the driving member defines a second clearance opening. The side wall of the connecting sleeve defines a vent hole that corresponds to the threading channel. **In** the axial direction of the connecting sleeve, the position of the second clearance opening corresponds to the position of the vent hole.

Optionally, the catheter assembly further includes a balloon catheter, and the balloon catheter includes:
a balloon body provided at a distal end of the inner sheath and configured to switch between an inflated state and a folded state under an action of fluid, the prosthetic implant in a loaded state being located around the balloon body; and
a catheter body fitted over the inner sheath or arranged in parallel with the inner sheath, wherein a distal end of the catheter body is in communication with the balloon body, a proximal end of the catheter body is connected to the main handle, and the catheter body contains a fluid channel

Optionally, a fluid guide member is provided in the balloon body and is located around the inner sheath, and the fluid guide member defines a diversion channel that communicates a distal end and a proximal end of the balloon body.

Optionally, the fluid guide member is tubular, and the diversion channel is positioned in at least one of the following ways relative to the fluid guide member:
on an outer wall of the fluid guide member;
inside the fluid guide member; and
in a side wall interlayer or hollow area of the fluid guide member.

Optionally, the diversion channel includes a diversion groove opened on an outer circumferential wall of the fluid guide member.

Optionally, the distal side and the proximal side of the fluid guide member are adjacent to the distal side and the proximal side of the balloon body, respectively.

Optionally, there are a plurality of diversion grooves, which are distributed in a circumferential direction of the fluid guide member, and each diversion groove extends in an axial direction of the fluid guide member or in a helical pattern.

Optionally, in the circumferential direction of the fluid guide member, a wrap angle of each diversion groove is in a range of 0 to 180 degrees.

Optionally, a communication groove is defined between two adjacent diversion grooves.

Optionally, a plurality of communication grooves arranged at intervals are provided, while the communication grooves at a same axial position form an annular groove.

Optionally, a fluid guide member is provided around the inner sheath, the fluid guide member is tubular and is located inside the balloon body, a tube wall of the fluid guide member defines a hollow area, and the hollow area extends continuously or intermittently from a distal side of the fluid guide member to a proximal side of the fluid guide member.

Optionally, there is a radial gap between the fluid guide member and the inner sheath and serves as the diversion channel.

When the fluid guide member is tubular, it is a flow guide tube located around the inner sheath, the radial gap between the flow guide tube and the inner sheath serves as a fluid channel for delivering fluid to inflate the balloon body.

In view of the related improvements of the flow guide tube, the present disclosure further provides an interventional delivery device based on balloon expansion deployment, which has opposite distal and proximal ends and includes an inner shaft and a balloon catheter located around the inner shaft, wherein the balloon catheter includes a balloon body and a catheter body in communication with a proximal end of the balloon body, the balloon body includes a first part, a middle part and a second part from a distal end to a proximal end, the middle part is configured for loading and fixing a prosthetic implant, and the catheter body has a main flow channel;
A flow guide tube is provided in a radial gap between the inner shaft and the balloon body, and at least a part of fluid output from the main flow channel to the balloon body passes through the second part and the middle part through the flow guide tube and then enters and inflates the first part.

The inner shaft can generally be a tube, and the interior of the tube can be used for a guide wire to pass through during intervention. The tube can also be called a core tube or an inner sheath. When the interventional delivery device is used based on balloon expansion, it is equivalent to a balloon device.

The present disclosure further provides an interventional delivery device based on balloon expansion deployment, which has opposite distal and proximal ends and includes an inner shaft and a balloon catheter located around the inner shaft, wherein the balloon catheter includes a balloon body and a catheter body in communication with a proximal end of the balloon body, the balloon body includes a first part, a middle part and a second part from a distal end to a proximal end, and the middle part is configured for loading and fixing a prosthetic implant;
A flow guide tube is arranged in a radial gap between the inner shaft and the balloon body, a distal end of the flow guide tube is fixed to a distal end of the inner shaft, while the remaining part of the flow guide tube is suspended between the inner shaft and the balloon catheter;
At least a part of fluid output from the catheter body to the balloon body passes through the second part and the middle part via the flow guide tube and then enters and inflates the first part.

Optionally, a radial gap between the inner shaft and the catheter body serves as a main flow channel, and the main flow channel is a single flow channel.

Optionally, a part of the fluid output from the main flow channel to the balloon body enters and inflates the second part, while the other part enters the flow guide tube, passes through the second part and the middle part, and then enters and inflates the first part.

Optionally, a proximal end of the flow guide tube has an open port serving as a fluid inlet.

Optionally, an axial position of the fluid inlet is adjacent to a junction of the balloon body and the catheter body.

Optionally, in a loaded state, the prosthetic implant is radially compressed and encloses the middle part, while the first part and the second part are exposed from both ends of the prosthetic implant; a proximal end of the flow guide tube has a fluid inlet, and the fluid inlet is located in the second part.

Optionally, at a proximal port of the flow guide tube, there is a first gap with a radial span of L1 between the inner shaft and the flow guide tube, and there is a second gap with a radial span of L2 between the inner shaft and the catheter body, and wherein L1 : L2 = 1 : 1.25 to 1.6.

Optionally, the balloon body is in an inflated state after being infused with fluid, and in the inflated state, the fluid inlet is located at a position where a cross-sectional area of a flow channel of the second part changes abruptly relative to the catheter body.

Optionally, the flow guide tube includes a distal section, a middle section and a proximal section from a distal end to a proximal end corresponding to different parts of the balloon body respectively, and the distal section of the flow guide tube has a first fluid outlet in communication with the first part, and wherein the first fluid outlet is located on a tube wall of the distal section and/or an end face of the distal section.

Optionally, a plurality of first fluid outlets are provided on the tube wall of the distal section.

Optionally, the plurality of first fluid outlets are distributed in a circumferential direction of the flow guide tube.

Optionally, the plurality of first fluid outlets are located in a middle of the distal section in a length direction of the flow guide tube.

Optionally, a proximal end of the flow guide tube and a distal end of the catheter body are interconnected and integrated into one piece, and a tube wall of the proximal section is provided with a second fluid outlet in communication with the second part.

Optionally, a proximal end of the flow guide tube has a tapered section with a gradually converging shape, and at least a part of the tapered section extends into a distal end of the catheter body.

Optionally, the tapered section has one or two beveled surfaces.

Optionally, an inner lumen of the flow guide tube is open to the beveled surface and is simultaneously in communication with an inner lumen of the catheter body and an inner cavity of the second part.

Optionally, a spacer is provided between the inner shaft and the flow guide tube in a radial direction to maintain a radial gap between them.

Optionally, the spacer includes a rib located on an inner wall of the flow guide tube.

Optionally, the spacer is a hollow structural component fixed between the inner shaft and the flow guide tube in the radial direction.

Optionally, a guide head is fixed to a distal end of the inner shaft, and a distal end of the flow guide tube is fixed to a proximal side of the guide head.

Optionally, the distal end of the flow guide tube is closed by the guide head.

Optionally, the proximal side of the guide head is provided with a positioning structure adapted to the distal end of the flow guide tube, and the positioning structure is a coupling groove for inserting the flow guide tube or a coupling column for inserting the flow guide tube.

The interventional delivery device of the present disclosure improves the delivery and diversion method of the fluid for the balloon expansion method. During the deployment process of the prosthetic implant, axial slippage can be avoided to ensure the positioning effect.

The present disclosure provides a method for driving a balloon catheter with fluid, wherein the balloon catheter has a distal end and a proximal end opposite to each other and includes a balloon body and a catheter body, a distal end of the catheter body is connected to a proximal end of the balloon body, the balloon body includes a first part, a middle part and a second part from a distal end to a proximal end, the middle part is configured for loading and fixing a prosthetic implant, the catheter body has a main flow channel, and the method includes steps of:
injecting fluid into the main flow channel from a proximal end of the catheter body;
diverting the fluid output from the main flow channel to the balloon body, allowing only a part of the fluid to enter and inflate the second part; and
directing the other part of the fluid to pass through the second part and the middle part, and then enter and inflate the first part

Optionally, all fluid for inflating the balloon body is supplied from the main flow channel before being diverted.

Optionally, the diverting step includes:
splitting the fluid into two streams at a junction of the catheter body and the second part, one of which enters and inflates the second part, and the other enters and inflates the first part after passing through the second part and the middle part through an independent flow channel.

Optionally, all the fluid enters the independent flow channel from the main flow channel, and the independent flow channel is in communication with a branch flow channel, wherein a part of the fluid enters and inflates the second part through the branch flow channel when flowing through the second part, and the other part enters and inflates the first part after passing through the second part and the middle part through the independent flow channel.

Optionally, the method further includes:
controlling the fluid so that the first part and the second part are inflated before the middle part, and the balloon body enters a transitional state in which both ends are inflated while the middle part is relatively contracted; and continuously injecting the fluid into the catheter body until the balloon body is fully inflated and the prosthetic implant is radially expanded.

Optionally, the fluid is split into two streams, with each stream including one or more sub-streams.

Optionally, the sub-streams of the same stream are radially distributed.

Optionally, the prosthetic implant is a cylindrical structure, and the fluid entering the independent flow channel passes through the middle part through an interior of the cylindrical structure.

Optionally, the balloon body has an axial direction extending between the distal end and the proximal end and corresponding radial and circumferential directions, and the step of inflating the first part includes:
outputting the fluid in the radial direction of the balloon body through the independent flow channel and inflating the first part; and/or
output the fluid in the axial direction of the balloon body through the independent flow channel and inflating the first part.

Optionally, the catheter assembly further includes a deflectable tube and a traction member for driving the deflectable tube, the deflectable tube is fitted over the inner sheath, distal ends of the deflectable tube and the traction member are fixedly connected, and proximal ends of the deflectable tube and the traction member are connected to the main handle and are in relative sliding fit.

Optionally, the deflectable tube and the traction member are both located on an outer periphery of the catheter body.

Optionally, the catheter assembly further includes a deflectable tube and a traction member for driving the deflectable tube, the deflectable tube is fitted over the inner sheath, distal ends of the deflectable tube and the traction member are fixedly connected, and proximal ends of the deflectable tube and the traction member are connected to the main handle and are in relative sliding fit.

Optionally, the traction member is arranged in the following manner:
the traction member is a traction tube and is located inside or outside the deflectable tube; or
the traction member is a traction wire, and is located inside, outside or embedded within a wall of the deflectable tube.

Optionally, a wall of the deflectable tube has an interlayer structure, within which a lining tube is fixed, and the traction member is movably arranged in the lining tube.

Optionally, the main handle includes:
a support body having an axial direction, wherein the proximal end of the deflectable tube is fixedly connected to the support body, and the inner sheath and the catheter body of the balloon catheter are both extended and connected to the proximal side of the support body;
a first sliding seat axially slidably engaged with the support body, wherein the proximal end of the traction member is fixed to the first sliding seat;
a first driving sleeve, which is rotatably fitted over the support body and is located around the first sliding seat, wherein the first driving sleeve and the first sliding seat are engaged in a threaded transmission fit;
a housing fixedly fitted over the support body, with at least a portion being an indicating section located outside the first driving sleeve; and
an identification member which is axially slidably installed on the indicating section and is located on an outer periphery of the first driving sleeve, wherein the first driving sleeve and the identification member are engaged in a threaded transmission fit.

Optionally, the first driving sleeve has an internal thread and an external thread, the first sliding seat has an external tooth that matches with the internal thread, and the identification member has an internal tooth that matches with the external thread.

Optionally, the identification member and the first sliding seat are configured to move synchronously under an action of the first driving sleeve.

Optionally, a view window corresponding to the identification member in position is provided on a side wall of the indicating section.

Optionally, an installation groove extending axially is provided in the support body, a guide cylinder is fixed in the installation groove, the first sliding seat is slidably fitted over the guide cylinder, and a guiding structure is provided between the first sliding seat and an outer wall of the guide cylinder to guide the axial movement.

Optionally, the proximal end of the deflectable tube is inserted into and fixed to the guide cylinder.

Optionally, when the auxiliary handle slides proximally to an extreme position, the distal end of the deflectable tube is exposed from the outer sheath, with an exposure length ranging from 0 to 20 cm.

The present disclosure further provides a vent structure on a control handle. The control handle is provided with a second vent hole, a second pipe joint is fixedly connected to the second vent hole, the second vent hole and the second pipe joint define a second vent channel, and a one-way valve core is provided in the second vent channel.

The one-way valve core can be applied to the main handle. For example, optionally, a second vent hole is defined on a side wall of the guide cylinder, a radial gap between the deflectable tube and the catheter body is in communication with the second vent hole, and a proximal end of the catheter body passes through the proximal end of the deflectable tube and further extends through the guide cylinder;
A second pipe joint is fixedly connected at the second vent hole, the second vent hole and the second pipe joint define a second vent channel, and a one-way valve core is provided in the second vent channel.

Optionally, the one-way valve core has a sealed state and an open state, and the one-way valve core specifically includes:
a limiting rod which is slidably mounted in an inner cavity of the second pipe joint, with a fluid gap between an outer periphery of the limiting rod and an inner wall of the second pipe joint; and
a sealing sheet connected to the limiting rod, which closes the fluid gap in the sealed state and opens the fluid gap in the open state.

Optionally, a second annular seat is provided around the second vent hole, and the second pipe joint is inserted and mated with the second annular seat, with an anti-rotation structure provided between the second pipe joint and the second annular seat.

Optionally, a second protective sleeve is provided around the second pipe joint, and a pipeline insertion gap is defined between an outer wall of the second pipe joint and an inner wall of the second protective sleeve.

Optionally, the anti-rotation structure includes:
a slot extending in an insertion direction of the second pipe joint, wherein the slot is provided on a side wall of one of the second pipe joint and the second annular seat; and
a positioning rib engaging with the slot, wherein the positioning rib is provided on a side wall of the other of the second pipe joint and the second annular seat.

Optionally, the inner wall of the second pipe joint is provided with a retaining ring surrounding the outer periphery of the limiting rod, and the fluid gap is located between an outer periphery of the retaining ring and the inner wall of the second pipe joint;
The end of the limiting rod away from the sealing sheet passes through the retaining ring and has a head restricted by the retaining ring, the head and the sealing sheet are fixed on two opposite sides of the retaining ring, and an edge of the sealing sheet is elastically deformable to open or close the fluid gap.

Optionally, the main handle includes:
a support body having an axial direction, wherein the inner sheath and the catheter body of the balloon catheter both extend to and are connected with a proximal side of the support body;
a second sliding seat axially slidably engaged with the support body;
a second driving sleeve rotatably fitted over the support body and located around the second sliding seat, wherein the second driving sleeve and the second sliding seat are engaged in a threaded transmission fit; and
a multi-way connector connected to a proximal side of the second sliding seat, wherein proximal ends of both the balloon catheter and the inner sheath are connected to the multi-way connector, an interior of the inner sheath is a through guidewire channel, and the multi-way connector has at least interfaces respectively connected to the guidewire channel and the fluid channel.

Optionally, the multi-way connector and the proximal side of the second sliding seat are rotatably engaged with each other and axially limited relative to each other.

Optionally, the second sliding seat is generally cylindrical and at least partially housed within the second driving sleeve, an outer wall of the second sliding seat is in threaded fit with an inner wall of the second driving sleeve, and the support body has a guide groove for guiding the second sliding seat to move axially.

Optionally, the distal end of the multi-way connector and a proximal end of the second sliding seat are inserted into and rotatably engaged with each other, with an axial limiting structure between the multi-way connector and the second sliding seat, and the axial limiting structure includes:
a latching tooth, fixed to one of the multi-way connector and the second sliding seat and protruding radially toward the other; and
a latching groove, which is provided on the other of the multi-way connector and the second sliding seat and receives the latching tooth.

Optionally, the interventional delivery system may also be combined with a prosthetic implant to form a transcatheter implant system, and the prosthetic implant is radially compressed and then enclosed by an outer sheath.

The present disclosure further provides an improved prosthetic implant, the prosthetic implant includes a stent and leaflets, the stent has a cell structure, two ends of the stent are respectively an inflow side and an outflow side, and an interior of the stent is a blood flow channel; The leaflets are located in the blood flow channel and cooperate with each other to open or close the blood flow channel, and an edge of the leaflet includes a fixed edge fixed to the stent and a free edge that cooperates with other leaflets to control the blood flow channel.

The stent is connected with an anti-paravalvular leakage component, which is formed in one piece and includes a base inside the stent and anti-paravalvular leakage elements fixed to an outside of the base, and the anti-paravalvular leakage elements are spaced apart and block-shaped and aligned with hollow areas of the stent's cells.

Optionally, the base is made of PET material, and the anti-paravalvular leakage element is made of PU material.

Optionally, depending on different axial positions of corresponding cells, the anti-paravalvular leakage elements include a first row of anti-paravalvular leakage elements and at least one of a second row of anti-paravalvular leakage elements and a third row of anti-paravalvular leakage elements, wherein the first row of anti-paravalvular leakage elements cover entire corresponding cells;
The second row of anti-paravalvular leakage elements are adjacent to an inflow side of the first row of anti-paravalvular leakage elements and cover partial areas of corresponding cells;
The third row of anti-paravalvular leakage elements are adjacent to an outflow side of the first row of anti-paravalvular leakage elements and cover partial areas of corresponding cells.

Optionally, axial lengths of the second row of anti-paravalvular leakage elements and the third row of anti-paravalvular leakage elements are only half a length of a cell respectively, and are adjacent to the first row of anti-paravalvular leakage elements.

Optionally, each anti-paravalvular leakage element, from an outflow side thereof to an inflow side thereof, gradually increases in thickness, and then gradually decreases in thickness after reaching a maximum outward protrusion.

Optionally, for each anti-paravalvular leakage element, the maximum outward protrusion is closer to an inflow side of a corresponding cell.

Optionally, for each anti-paravalvular leakage element, a distance from the maximum outward protrusion to the inflow side of the corresponding cell is S1, and a distance from the maximum outward protrusion to an outflow side of the corresponding cell is S2, wherein S1 : S2 is in a range of 0.2 to 0.8.

Optionally, a diameter of the loading section is larger than a diameter of the sheath section.

Optionally, a distal end of the inner sheath is provided with a guide head, an outer circumference of the guide head is provided with an annular step, and a distal end surface of the loading section abuts against the annular step for position limitation.

The interventional delivery system of the present disclosure separates the movement of the outer sheath from other internal tubes, while also releasing the restriction on the length of the main handle, making the operation of the outer sheath more flexible.

The present disclosure provides a limiting mechanism, namely a blocking mechanism, which provides a relatively long-lasting blocking effect during the initial stage of interventional delivery and deployment of the prosthetic heart valve.

The present disclosure provides a limiting mechanism for an interventional delivery system, including:
a coupling portion for connecting with the interventional delivery system, the coupling portion having an axial direction and corresponding circumferential and radial directions; and
a deformable portion including a plurality of elongated rods arranged in sequence in the circumferential direction, each of which has:
   a first end connected to the coupling portion; and
   a second end located on one axial side of the coupling portion relative to the first end; wherein the rods are elastically deformable, and have a compressed state suitable for interventional delivery and an expanded state, and wherein the second ends of the rods diverge outward in the radial direction of the coupling portion in the expanded state relative to the compressed state.

Optionally, the coupling portion is in a straight cylindrical shape, and the rods are distributed radially, with the first ends of the rods converging at one axial end of the coupling portion.

Optionally, the coupling portion is a radially deformable annular structure.

Optionally, the coupling portion is an axially undulating wave structure with opposite peaks and valleys, and the first ends of the rods are connected to corresponding peaks.

Optionally, the coupling portion and the deformable portion are formed in one piece.

Optionally, the coupling portion and the deformable portion are formed in one piece by cutting a tube.

Optionally, the second ends of the rods are configured to move independently.

Optionally, the rods tend to extend helically around an axis of the coupling portion.

Optionally, the rods have a number in a range of 4 to 10.

Optionally, the second end of the rod has a smooth outer contour and/or is coated with a protective layer.

Optionally, a portion of the rod close to the second end is bent radially inwardly.

Optionally, a portion of the rod close to the second end has a lower radial stiffness than the rest of the rod.

The present disclosure provides another limiting mechanism for an interventional delivery system, which is configured to be installed in a balloon body, wherein the balloon body has a folded state and an inflated state, and in the folded state, the balloon body has a plurality of folds; the limiting mechanism includes:
a coupling portion for connecting with the interventional delivery system, the coupling portion having an axial direction and corresponding circumferential and radial directions; and
a deformable portion including a plurality of rods arranged in sequence in the circumferential direction, wherein the rods are elastically deformable, and have a compressed state suitable for interventional delivery and an expanded state, and in the compressed state, the rods are placed in corresponding folds.

Optionally, the balloon body is wrapped in a first direction in the circumferential direction in the folded state, and a helical direction of the rods is the same as the first direction.

Optionally, when the balloon body is in the folded state, a second end of at least one rod is not lower than a prosthetic implant in the radial direction.

The present disclosure provides another limiting mechanism for an interventional delivery system, including:
a coupling portion for connecting with the interventional delivery system, the coupling portion having an axial direction and corresponding circumferential and radial directions; and
a deformable portion including a plurality of rods arranged in sequence in the circumferential direction, each of which has:
   a first end connected to the coupling portion; and
   a second end located on one axial side of the coupling portion relative to the first end; wherein the rods are elastically deformable, and have a compressed state suitable for interventional delivery and an expanded state, and the second ends of the rods diverge outward in the radial direction of the coupling portion in the expanded state relative to the compressed state; the rods are made of memory alloy, distances from the second end of the rod to an axis of the coupling portion in the expanded and compressed states are R1 and R2 respectively, and R1 : R2 = 2 - 20 : 1.

The present disclosure provides a balloon device for delivering a prosthetic implant, including an inner shaft and a balloon body located around the inner shaft, wherein the inner shaft is installed with any of the above-mentioned limiting mechanisms located inside the balloon body.

The inner shaft can generally be a tube, and the interior of the tube can be used for a guide wire to pass through during intervention. The tube can also be called a core tube or an inner sheath.

In view of the various limiting mechanisms provided in the present disclosure, the present disclosure provides an interventional delivery system based on balloon expansion for delivering a prosthetic implant. The interventional delivery system includes a balloon device and an outer sheath, the balloon device includes an inner shaft and a balloon body located around the inner shaft, a plurality of rods arranged in sequence in a circumferential direction are provided at a distal end of the inner shaft, the rods are elastically deformable and have a compressed state suitable for interventional delivery and an expanded state, and each rod has:
a first end connected to the inner shaft; and
a second end which is located at one side of the first end and is suspended relative to the inner shaft, wherein the second ends of the rods diverge outward in a radial direction in the expanded state relative to the compressed state;
wherein the prosthetic implant is configured to be radially compressed and installed on the balloon body in a loaded state and located at a proximal end of the rods, and the rods act on the balloon body so that an outer peripheral surface of the balloon body is not lower than an outer peripheral surface of a distal end of the prosthetic implant.

Optionally, the rods act on the balloon body so that the outer peripheral surface of the balloon body is roughly flush with the outer peripheral surface of the distal end of the prosthetic implant.

In view of the various limiting mechanisms provided in the present disclosure, the present disclosure provides another interventional delivery system based on balloon expansion for delivering a prosthetic implant, wherein the interventional delivery system has opposite distal and proximal ends and an axial direction extending between the proximal end and the distal end, and the interventional delivery system includes:
a balloon device, including an inner shaft and a balloon body located around the inner shaft, wherein a first limiting mechanism (the limiting mechanism described in the present disclosure can be used) located inside the balloon body is installed at a distal end of the inner shaft;
an intermediate shaft, which is located outside the inner shaft, wherein a second limiting mechanism outside the balloon body is installed at a distal end of the intermediate shaft, the second limiting mechanism is located proximal to the first limiting mechanism, the prosthetic implant is configured to be radially compressed and installed on the balloon body in a loaded state and located between the first limiting mechanism and the second limiting mechanism, and the two limiting mechanisms are configured to limit axial movement of the prosthetic implant relative to the balloon body; and
an outer sheath which is slidably fitted over the balloon device, wherein the outer sheath has an extreme position of proximal movement on its sliding path, and in this extreme position, the first limiting mechanism and the prosthetic implant are exposed outside the outer sheath, while the second limiting mechanism remains inside the outer sheath.

In view of the various limiting mechanisms provided in the present disclosure, the present disclosure provides another interventional delivery system based on balloon expansion for delivering a prosthetic implant, the interventional delivery system including:
a balloon device, including an inner shaft and a balloon body located around the inner shaft, wherein a plurality of rods arranged in sequence in a circumferential direction inside the balloon body are installed at a distal end of the inner shaft, the rods are elastically deformable and have a compressed state suitable for interventional delivery and an expanded state, each rod has a first end connected to the inner shaft and a second end located on one side of the first end and suspended relative to the inner shaft, the second ends of the rods diverge outward in a radial direction in the expanded state relative to the compressed state, and the prosthetic implant is configured to be radially compressed and installed on the balloon body in a loaded state and is located at a proximal end of the rods;
an adjustment wire for releasably fixing the prosthetic implant on the balloon body, wherein one end of the adjustment wire is fixed to the balloon body, while the other end passes through the prosthetic implant and has a locking eyelet; and
a locking wire having a locked state and an unlocked state, wherein in the locked state, the locking wire passes through the locking eyelet to restrict the prosthetic implant, and in the unlocked state, the locking wire is disengaged from the locking eyelet to release the prosthetic implant.

Optionally, the first end of the rod is connected to a coupling portion connected to the inner shaft, and all the rods constitute a deformable portion.

Optionally, the interventional delivery system further includes a control handle, wherein the control handle is connected to and controls a balloon device, and the balloon device adopts the above-mentioned balloon device.

Optionally, a fluid guide member is provided in the balloon body and is located around the inner shaft, and the fluid guide member defines a diversion channel that communicates a distal end and a proximal end of the balloon body.

The rods of the limiting mechanism of the present disclosure are relatively independent. On the one hand, the prosthetic implant can be deformed and expanded during the expansion process to extend the blocking time and better limit the axial displacement of the prosthetic implant. On the other hand, the traction between the rods in the radial direction is reduced, further ensuring the positioning effect of the prosthetic implant in an eccentric state.

The present disclosure provides a balloon device for delivering a prosthetic heart valve, which provides a relatively long-lasting blocking effect during the initial stage of the interventional delivery and deployment of the prosthetic heart valve.

The present disclosure provides a balloon device for delivering a prosthetic heart valve, including:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body; and
a limiting mechanism which is configured to limit movement of the prosthetic heart valve in the axial direction at least when the prosthetic heart valve is mounted on the balloon body in a radially compressed state, wherein the limiting mechanism specifically includes:
   a limiting body including a plurality of rods, which generally extend from a first end in the axial direction to a second end in the axial direction, converging at the first end and the second end respectively to form a hollow cage-like structure.

Optionally, the plurality of rods are configured to form a side wall of the cage-like structure, and gaps between two adjacent rods on the side wall form hollow areas.

Optionally, the hollow areas include main hollow areas and auxiliary hollow areas, and a span of the main hollow area in the axial direction is greater than a span of the auxiliary hollow area in the axial direction.

Optionally, the main hollow area spans the first end and the second end.

Optionally, the cage-like structure has an outward expansion part, the outward expansion part has a maximum outer diameter of the cage-like structure, and the main hollow area extends across the outward expansion part.

Optionally, the main hollow areas and the auxiliary hollow areas are distributed in sequence at intervals in a circumferential direction.

Optionally, the limiting mechanism is located inside or outside the balloon body.

Optionally, at least one of the catheter body and the balloon body is directly fixed to the limiting mechanism, or is indirectly fixed to the limiting mechanism through an intermediate piece.

Optionally, the balloon device further includes an inner shaft (as the intermediate piece) which extends through the catheter body and the balloon body; the limiting mechanism further includes:
at least one coupling member which is fixed to the inner shaft and connected to the converging portion of the limiting body.

Optionally, the coupling member is located inside or outside the limiting body.

Optionally, the coupling member is located at at least one end of the limiting body in the axial direction.

Optionally, the outer diameter ratio of the outward expansion part to the coupling member is in a range of 2 - 4 : 1, preferably 3 : 1.

The present disclosure further provides a balloon device for delivering a prosthetic heart valve, including:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body; and
a limiting mechanism which is configured to limit movement of the prosthetic heart valve in the axial direction at least when the prosthetic heart valve is mounted on the balloon body in a radially compressed state, wherein the limiting mechanism specifically includes:
   a limiting body which is generally a cage-like structure, wherein a part of the cage-like structure is an outward expansion part, and the outward expansion part has the largest radial expansion relative to other parts of the cage-like structure; in the axial direction, one end of the limiting body is a first end facing the prosthetic heart valve when in use, and the other end is an opposite second end, and the outward expansion part is adjacent to the first end of the limiting body.

Optionally, the balloon device further includes an inner shaft which extends through the catheter body and the balloon body; the limiting mechanism further includes a coupling member fixed to the inner shaft and connected to the limiting body.

Optionally, a concave area for accommodating an end of the prosthetic heart valve is defined between a side of the limiting body on the first end and the coupling member on that side.

The present disclosure further provides a balloon device for delivering a prosthetic heart valve, including:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body; and
a limiting mechanism which is configured to limit movement of the prosthetic heart valve in the axial direction at least when the prosthetic heart valve is mounted on the balloon body in a radially compressed state, wherein the limiting mechanism specifically includes:
   a limiting body including a plurality of rods that generally form a cage-like structure, wherein a part of the cage-like structure is an outward expansion part, and the outward expansion part has the largest radial expansion relative to other parts of the cage-like structure; on a cross-section of the outward expansion part, the rods are arranged at intervals in a circumferential direction of the limiting mechanism, enclosing an area that approximates a polygon.

Optionally, the polygon is a regular polygon with 4 to 12 sides.

Optionally, the regular polygon has 6 to 8 sides.

Optionally, one end of the limiting body is the first end facing the prosthetic heart valve when in use, and the other end is the opposite second end, and both ends of the limiting body are converged toward a central axis of the limiting body.

Optionally, the rods extend from the first end to the second end to form a sphere or an ellipsoid.

Optionally, a cross-section of the ellipsoid is an elliptical surface, and a major axis of the elliptical surface is consistent with the axial direction.

Optionally, the cage-like structure is formed by a plurality of rods, and the rods extend from the first end to the second end to form a cone.

Optionally, the cone includes a first cone and a second cone.

Optionally, the first cone and the second cone are located on both sides or on the same side of the outward expansion part.

Optionally, when the first cone and the second cone are located on both sides of the outward expansion part, the first cone and the second cone gradually converge in shape from the outward expansion part, with different converging trends.

Optionally, one end of the limiting body is the first end facing the prosthetic heart valve when in use, and the other end is the opposite second end, wherein the first cone is connected to the first end, and the first cone has a steeper converging trend.

Optionally, the first cone and the second cone intersect at the outward expansion part, and an angle at the intersection is in a range of 20 to 120 degrees.

Optionally, when the first cone and the second cone are on the same side of the outward expansion part, one end of the limiting body is the first end facing the prosthetic heart valve when in use, and the other end is the opposite second end, wherein the first cone is connected to the first end, and the first cone is converged in a direction away from the first end.

Optionally, the balloon device further includes an inner shaft, which extends through the catheter body and the balloon body; the limiting mechanism further includes a coupling member, which is connected to the inner shaft, and the coupling member is in a radially compressible tubular shape.

Optionally, the coupling member has a wave structure that undulates in the axial direction or has a deformable mesh structure.

Optionally, there are two coupling members, the cage-like structure is formed by a plurality of rods, all the rods define a side wall of the cage-like structure, and all the rods extend from one coupling member to the other coupling member and have at least one branch on their extension path, or intersect with adjacent rods.

Optionally, a plurality of junction points of two rods are distributed at intervals in a circumferential direction of the outward expansion part.

Optionally, the rods are arranged in pairs and extend side by side from one of the coupling members.

Optionally, the main hollow areas have a number in a range of 4 to 12.

Optionally, the number of the main hollow areas is in a range of 6 to 8.

Optionally, the main hollow areas have the same shape and are evenly arranged in a circumferential direction.

Optionally, the main hollow area is elongated.

Optionally, in the axial direction, a length of the main hollow area is at least 40% of a total length of the limiting body.

Optionally, the length of the main hollow area is at least 60% of the total length of the limiting body.

Optionally, a length of the main hollow area is 75% to 100% of a total length of the limiting body.

Optionally, in the axial direction, the main hollow area extends to both sides of the outward expansion part, and the extended length is at least 20% of a total length of the limiting body.

Optionally, the main hollow area is diamond-shaped.

Optionally, the hollow areas of the cage-like structure include the main hollow areas and the corresponding auxiliary hollow areas, wherein the auxiliary hollow areas avoid the outward expansion part.

Optionally, the limiting mechanism is formed in one piece by cutting a pipe.

Optionally, the tube has an initial outer diameter D1, the coupling member has an outer diameter D2, and the outer diameter D2 of at least one coupling member is smaller than D1.

The present disclosure further provides a balloon device for delivering a prosthetic heart valve, having a distal end and a proximal end opposite to each other, including:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body;
an inner shaft which extends through the catheter body and the balloon body; and
a limiting mechanism which is configured to limit movement of the prosthetic heart valve in the axial direction at least when the prosthetic heart valve is mounted on the balloon body in a radially compressed state, wherein the limiting mechanism specifically includes:
   a limiting body which is generally a cage-like structure;
   a guide tube connected to the limiting body, wherein the guide tube and the limiting body are formed in one piece by cutting a tube made of shape memory alloy; and
   a coupling member connected to the inner shaft and at least one of the limiting body and the guide tube.

Optionally, the guide tube is connected to a proximal side of the limiting body.

Optionally, there are two limiting bodies, which are respectively connected to a proximal side and a distal side of the guide tube.

Optionally, the cage-like structures of the two limiting bodies are independent of each other.

Optionally, the balloon body includes a first part, a middle part and a second part from a distal end to a proximal end, and the middle part is configured for loading and fixing the prosthetic heart valve;
The guide tube is arranged in a radial gap between the inner shaft and the balloon body, and a guide channel for fluid to pass through is defined between the guide tube and the inner shaft.

Optionally, a first coupling member and a second coupling member located at a distal end of the first coupling member are provided and located around the inner shaft, at least one coupling member is fixed to the inner shaft, and a distal part of the balloon body wraps the second coupling member.

Optionally, the second coupling member is located at a proximal end of the guide tube and is adjacent to a connection of the balloon body and the catheter body.

Optionally, a proximal end of the guide tube has a diameter-reduced section and is connected to the corresponding coupling member through the diameter-reduced section.

Optionally, the guide tube has a fluid inlet, and the fluid inlet is defined on the diameter-reduced section and/or a circumferential wall of the guide tube.

Optionally, a circumferential wall of the guide tube has a hollowed-out gap.

Optionally, the limiting mechanism is formed in one piece by cutting a tube.

Optionally, the tube has an initial outer diameter D1, the guide tube has an outer diameter D1, the coupling member has an outer diameter D2, and the outer diameter D2 of at least one coupling member is smaller than D1.

The present disclosure further provides a transcatheter implant intervention system, including the balloon device described in the present disclosure and a prosthetic implant. For example, the balloon device includes:
a catheter body;
a balloon body in communication with the catheter body; and
a limiting mechanism, wherein the prosthetic implant is radially compressed and installed on the balloon body in a loaded state and is blocked by the limiting mechanism in an axial direction.

Optionally, the transcatheter implant intervention system further includes an outer sheath that is slidably fitted over the balloon device, and the limiting body has a loaded state, an intermediate state, and an expanded state;
wherein in the loaded state, the limiting body is located inside the balloon body and the outer sheath, and is subjected to radial forces from both the balloon body and the outer sheath;
in the intermediate state, the limiting body is free from a radial constraint from the outer sheath and is subjected to the radial force from the balloon body; and
in the expanded state, the balloon body is inflated and the limiting body is expanded.

Optionally, the outer sheath is a catheter sheath.

Optionally, the prosthetic implant is a prosthetic heart valve.

The present disclosure improves the limiting mechanism to make it more suitable for interventional delivery of the prosthetic implant and for cooperation with the deployment based on balloon expansion.

The present disclosure provides a prosthetic heart valve, including a first stent and a plurality of leaflets, wherein the first stent is a radially deformable cylindrical structure, an interior of the first stent is a blood flow channel, and the leaflets cooperate with each other to control the blood flow channel;
the first stent has a cell structure formed by struts, the first stent is in a straight cylindrical shape, the cell structure includes diamond-shaped cells, an end of the first stent has an eyelet structure protruding from the end, and the eyelet structure is formed by struts at the end; the first stent has an expanded state and a compressed state, wherein in the compressed state, the eyelet structure protrudes from the end by a distance of **L1,** and in the expanded state, the eyelet structure protrudes from the end by a distance of L2, and L1 > L2 ≥ 0.

Optionally, the cell structure of the first stent forms a plurality of nodes at the end; in the expanded state, the eyelet structure is basically flush with surrounding nodes, and in the compressed state, the eyelet structure is higher than the surrounding nodes.

Optionally, L1 : L2 is in a range of 1.2 to 1.6 : 1.

Optionally, L1 is in a range of 0.6 to 0.8 mm, and L2 is in a range of 0.4 to 0.6 mm.

Optionally, in the expanded state, the eyelet structure is generally an arc-shaped structure, and in the compressed state, the eyelet structure approximates a circular structure.

Optionally, the prosthetic heart valve is a prosthetic pulmonary valve or a prosthetic aortic valve.

Optionally, in the expanded state, the struts at the end are bent into an arc shape and define an edge of the eyelet structure.

Optionally, the first stent has eyelet structures at both axial ends, and circumferential positions of the eyelet structures at both ends are the same or staggered.

Optionally, a cell with the eyelet structure is a first cell, a cell adjacent to the first cell in a circumferential direction is a second cell, and within the first cell, the struts around the eyelet structure include:
an arc segment defining an edge of the eyelet structure; and
connecting segments extending from two ends of the arc segment in opposite directions to nodes on both sides of the first cell in the circumferential direction.

Optionally, an angle between the connecting segments on both sides of the arc segment is A1, an inner angle of a node located at an axial end of the second cell is A2, and A1 is greater than A2 in the expanded state.

Optionally, in the expanded state, a central angle corresponding to the arc segment is in a range of 150 to 210 degrees.

Optionally, in the expanded state, a span of the arc segment in the circumferential direction of the first stent accounts for 1/4 to 1/2 of a span of the first cell, for example, about 1/3.

Optionally, in the first cell, a strength of the struts around the eyelet structure is less than that of other struts of the first cell.

Optionally, the cell structure of the first stent includes a plurality of rows of cells, and two axially adjacent rows are staggered in a circumferential direction and share some struts; for example, 3 to 6 rows, and for another example, 5 rows. **In** the expanded state, an inner angle of a node of each cell is in a range of 75 to 105 degrees, for example, 90 degrees, in which case the cell is basically a square.

Optionally, the prosthetic heart valve further includes a skirt connected to and surrounded by an inner wall of the first stent, and one axial side of the skirt is connected to the leaflets.

Optionally, the prosthetic heart valve further includes a sealing member arranged in a circumferential direction of the first stent, the sealing member is fixed to the skirt and protrudes from a radial inner side of the first stent to a radial outer side of the first stent via corresponding cells.

Optionally, the skirt and the sealing member are formed in one piece or separate pieces which are fixed by bonding, and both the skirt and the sealing member are made of PU material.

Optionally, the sealing member includes:
a plurality of sealing blocks arranged in the circumferential direction of the first stent, wherein the sealing blocks are made of foam material and embedded in corresponding cells.

Optionally, the sealing member further includes:
an inner lining film, wherein the plurality of sealing blocks are fixed on one side of the inner lining film, and the inner lining film and the plurality of sealing blocks are made of a same material.

Optionally, the skirt is made of biological pericardial material, and the inner lining film is sutured to the skirt.

To improve the connection of the leaflets, the present disclosure further provides a prosthetic heart valve, including a stent (i.e., the first stent) and a plurality of leaflets connected to the stent, wherein the stent is a cylindrical structure and has a blood flow channel inside;
at least parts of the stent are configured as support bars, in a circumferential direction of the stent, at least one side of the support bar is provided with a recessed area, the leaflets are fixed to the support bars by sutures, and all knots of the sutures are located in the corresponding recessed areas.

Optionally, the leaflet includes:
a leaflet body having a transverse direction and a longitudinal direction in a flattened state, wherein an outer edge of the leaflet body includes a free edge and a fixed edge distributed on two opposite sides in the longitudinal direction, the plurality of leaflets are fixed to the stent through their respective fixed edges, and the free edges of the plurality of leaflets cooperate with each other to control the blood flow channel in the prosthetic heart valve; and
commissure tabs arranged at two opposite sides of the leaflet body in the transverse direction, wherein the free edge and the fixed edge meet at the commissure tabs on corresponding sides;
wherein commissure tabs between two adjacent leaflets are connected to each other through first sutures, and are also connected to the corresponding support bars through second sutures and third sutures, wherein all knots of the second sutures and the third sutures are located in corresponding recessed areas.

Optionally, a joint of the commissure tabs between two adjacent leaflets connected through the first sutures contacts a radial inner side of the support bar.

Optionally, the commissure tabs between two adjacent leaflets cooperate with each other to wrap the support bar.

Optionally, the knots of the second sutures are wrapped by the commissure tabs, and the knots of the third sutures are exposed from the commissure tabs.

Optionally, two axial sides of the stent are opposite inflow and outflow sides, and a row of hexagonal cells is arranged circumferentially on the outflow side, with the support bar serving as a common side between two adjacent cells.

Optionally, an extension direction of the support bar is consistent with an axial direction of the stent.

The present disclosure further provides a transcatheter implant system, including a balloon device and a prosthetic heart valve. The balloon device and the prosthetic heart valve can respectively adopt the various solutions described in the present disclosure. As an improved solution, the balloon device includes:
a catheter body;
a balloon body in communication with the catheter body, wherein the balloon body is configured to receive fluid from the catheter body to transition from a folded state to an inflated state, thereby enabling the prosthetic heart valve to expand from a compressed state to an expanded state based on changes of the balloon body; and
an inner shaft which extends through the catheter body and the balloon body, and is provided with a guide head at a distal end thereof;
wherein the transcatheter implant system further includes a locking wire structure for limiting an axial position of the prosthetic heart valve relative to the balloon body, and the locking wire is configured to pass through the eyelet structure of the first stent.

Optionally, the locking wire structure includes:
a first adjustment wire for releasably fixing the prosthetic implant on the balloon body, wherein one end of the first adjustment wire is fixed to the catheter body, and the other end passes through a corresponding eyelet structure and has a first locking eyelet;
a second adjustment wire, one end of which is connected to the guide head and the other end of which passes through a corresponding eyelet structure and has a second locking eyelet; and
a locking wire having a locked state and an unlocked state, wherein in the locked state, the locking wire passes through the locking eyelets to restrict the prosthetic implant, and in the unlocked state, the locking wire is disengaged from the locking eyelets to release the prosthetic implant;
wherein the guide head is provided with an insertion hole, and an end of the locking wire extends into the insertion hole in the locked state.

Optionally, there are at least two junctions between the adjustment wires and the locking wire, at least one junction limits movement of the prosthetic implant in a distal direction, and at least one junction limits movement of the prosthetic implant in a proximal direction.

Optionally, the first adjustment wire and the second adjustment wire are matched in axial length so that the first stent is maintained in a middle of the balloon body.

The present disclosure further provides a transcatheter implant system, including a balloon device and a prosthetic heart valve as described above, wherein the balloon device includes:
a catheter body;
a balloon body in communication with the catheter body, wherein the balloon body is configured to receive fluid from the catheter body to transition from a folded state to an inflated state, thereby enabling the prosthetic heart valve to expand from a compressed state to an expanded state based on changes of the balloon body; and
a limiting mechanism which is configured to limit the movement of the prosthetic heart valve in the axial direction at least when the prosthetic heart valve is installed on the balloon body in a compressed state, wherein the limiting mechanism specifically includes a limiting body, and the limiting body can adopt any of the limiting bodies described above, such as including a plurality of rods to form a hollow cage-like structure.

Optionally, the limiting mechanism includes at least one limiting body, and at least the distal end of the first stent is blocked by the limiting body.

Optionally, the distal end of the first stent has an eyelet structure protruding from the end, and when the first stent is in the compressed state, the eyelet structure is in contact with the limiting body.

Optionally, both ends of the first stent each have an eyelet structure protruding from the corresponding end, and the limiting mechanism includes at least two limiting bodies respectively located at the proximal and distal ends of the balloon body. When the first stent is in a compressed state, the eyelet structures are respectively in contact with the corresponding limiting bodies.

Optionally, the balloon device is further configured with a locking wire structure for axial limiting for the first stent.
Optionally, the limiting mechanism includes a limiting body, and at least the distal end of the first stent is blocked by the limiting body;
The locking wire structure includes:
   a first adjustment wire, used for releasably fixing the prosthetic heart valve to the balloon body, one end of the first adjustment wire can remain fixed to the catheter body, and the other end can pass through the eyelet structure and has a first locking eyelet;
   a second adjustment wire, one end of which is connected to the guide head and the other end of which can pass through the eyelet structure and has a second locking eyelet; and
   a locking wire having a locked state and an unlocked state. In the locked state, the locking wire passes through the locking eyelets to restrict the prosthetic heart valve. In the unlocked state, the locking wire is disengaged from the locking eyelets to release the prosthetic heart valve. The guide head is provided with an insertion hole. In the locked state, the end of the locking wire extends into the insertion hole.

The present disclosure further provides a prosthetic heart valve assembly, including:
a prosthetic heart valve (such as a prosthetic pulmonary valve, etc.);
an anchoring stent, which is fitted over a radial outer side of the prosthetic heart valve after assembly, wherein the prosthetic heart valve and the anchoring stent both have an expanded state and a compressed state, and in the expanded state, the prosthetic heart valve and the anchoring stent contact each other;
wherein the anchoring stent has a cell structure formed by struts, and the cell structure includes in an axial direction:
   a middle part including two rows of fishbone-shaped cells; and
   end parts including two rows of diamond-shaped cells, which are respectively located on both sides of the middle part in the axial direction;
   wherein the anchoring stent also has a pre-expanded state before assembly, the anchoring stent has a first diameter in the pre-expanded state, and has a second diameter in the expanded state, and the second diameter is greater than the first diameter.

Optionally, in the expanded state, an axial length of the anchoring stent is greater than a length of the first stent.

Optionally, the first stent is located in a middle of the anchoring stent in the axial direction.

Optionally, the fishbone-shaped cell is a concave hexagon with opposite convex tips and concave tails, the concave tails of two rows of fishbone-shaped cells are symmetrical and face each other, while the convex tips face outward;
the two rows of diamond-shaped cells are respectively located on two sides of the middle part in the axial direction, and each diamond-shaped cell is located between the convex tips of two adjacent fishbone-shaped cells.

Optionally, the first stent does not axially exceed the middle part.

Optionally, the first stent has a denser cell than that of the anchoring stent.

Optionally, a number of circumferential cells of the first stent is 1.2 to 2 times a number of circumferential cells of the anchoring stent.

Optionally, within a diamond-shaped cell at an end of the anchoring stent, two struts facing away from the middle part have lower strength than two struts shared with the middle part.

Optionally, the prosthetic heart valve and the anchoring stent are both configured to be deployed based on balloon expansion.

Optionally, the prosthetic heart valve is configured to be deployed based on balloon expansion, and the anchoring stent is configured to be deployed based on self-expansion.

The present disclosure further provides a transcatheter implant system, including:
a prosthetic heart valve assembly as described above; and
a first delivery system having a first balloon body for delivering and expanding the prosthetic heart valve.

Optionally, the transcatheter implant system further includes a second delivery system for expanding the anchoring stent.

Optionally, the first delivery system and the second delivery system may use the same or different interventional paths to implement interventional delivery.

Optionally, the first delivery system further includes a second balloon body for expanding the anchoring stent.

Optionally, the first balloon body and the second balloon body are respectively configured with fluid channels (to avoid mutual interference), and the two balloon bodies are arranged axially and respectively loaded with a prosthetic heart valve and an anchoring stent, and the anchoring stent to be deployed first is located at the distal end of the first stent.

Optionally, the distal end or both ends of the anchoring stent are each provided with a limiting mechanism in the second delivery system.

Optionally, the second balloon body is configured with a limiting mechanism(s) for the distal end or both ends of the anchoring stent.

Optionally, the limiting mechanism is configured to limit movement of the anchoring stent in an axial direction at least when the anchoring stent is installed on the corresponding balloon body in a radially compressed state, and the limiting mechanism specifically includes a limiting body, and the limiting body can adopt any of the limiting bodies described above, such as including a plurality of rods to form a hollow cage-like structure.

Optionally, a limiting mechanism(s) is configured in the first balloon body for the distal end or both ends of the prosthetic heart valve.

Optionally, the limiting mechanism is configured to limit the axial movement of the prosthetic heart valve at least when the prosthetic heart valve is mounted on the first balloon body in a radially compressed state, and the limiting mechanism specifically includes a limiting body, and the limiting body can adopt any of the limiting bodies described above, such as including a plurality of rods to form a hollow cage-like structure.

Optionally, in the second delivery system, a locking wire structure for axial limiting is configured for two ends of the anchoring stent.

Optionally, the end of the first stent and/or the anchoring stent has an eyelet structure protruding from the end.

Optionally, the locking wire structure includes:
a first adjustment wire, used to releasably fix the prosthetic heart valve or the anchoring stent to the corresponding delivery system, one end of the first adjustment wire can remain fixed to the catheter body, and the other end can pass through the eyelet structure and has a first locking eyelet;
a second adjustment wire, one end of which is connected to the guide head and the other end of which can pass through the eyelet structure and has a second locking eyelet; and
a first locking wire having a locked state and an unlocked state. In the locked state, the locking wire passes through the locking eyelets to restrict the anchoring stent. In the unlocked state, the locking wire is disengaged from the locking eyelets to release the prosthetic heart valve or the anchoring stent.

Optionally, when the transcatheter implant system includes two delivery systems, two locking wire structures are configured for the two delivery systems.

Optionally, when the first delivery system includes a first balloon body and a second balloon body, the locking wire structure includes:
a third adjustment wire and a fourth adjustment wire used to releasably fix the prosthetic heart valve on the first balloon body, with locking eyelets that can pass through the corresponding eyelet structures;
a fifth adjustment wire and a sixth adjustment wire used to releasably fix the anchoring stent on the second balloon body, with locking eyelets that can pass through the anchoring stent; and
a second locking wire which has a locked state and an unlocked state; in the locked state, the second locking wire passes through the locking eyelets to restrict the prosthetic heart valve and the anchoring stent; in the unlocked state, the second locking wire is disengaged from the locking eyelets to release the prosthetic heart valve and the anchoring stent.

Optionally, the locking wire includes a first locking wire and a third locking wire. In the locked state, the first locking wire passes through the corresponding locking eyelets to restrict the prosthetic heart valve, and the third locking wire passes through the corresponding locking eyelets to restrict the anchoring stent.

Optionally, the anchoring stent includes an eyelet structure, and the second locking wire or the third locking wire passes through the eyelet structure of the anchoring stent.

Optionally, a locking wire structure for axial limitation is configured at two ends of the prosthetic heart valve in the first delivery system.

Optionally, a locking wire structure is configured for the anchoring stent in the transcatheter implant system.

Optionally, the transcatheter implant system is provided with a locking wire structure for the prosthetic heart valve and the anchoring stent.

Optionally, the first delivery system further includes an outer sheath, the anchoring stent is accommodated in the outer sheath in the compressed state, and the anchoring stent is configured to be deployed based on self-expansion.

The present disclosure further provides a releasing method for a prosthetic heart valve assembly from an interventional delivery system, wherein the prosthetic heart valve assembly includes a prosthetic heart valve and an anchoring stent, both of which have an expanded state and a compressed state;
the interventional delivery system includes at least an inner shaft for interventional delivery, and both the prosthetic heart valve and the anchoring stent are connected to the inner shaft in a compressed state;
the releasing method includes steps of:
   step S100, releasing a connection between the anchoring stent and the inner shaft, allowing the anchoring stent to enter the expanded state;
   step S200, delivering the prosthetic heart valve to an interior of the anchoring stent; and
   step S300, releasing a connection between the prosthetic heart valve and the inner shaft, allowing the prosthetic heart valve to enter the expanded state and fit tightly against an inner wall of the anchoring stent.

Optionally, the interventional delivery system further includes an outer sheath, the outer sheath is slidably mounted around the inner shaft, and the anchoring stent is connected to the inner shaft and enclosed by the outer sheath;
the step of releasing the connection between the anchoring stent and the inner shaft includes first moving the outer sheath and the inner shaft relative to each other to expose the anchoring stent from the outer sheath, thereby allowing the anchoring stent to enter the expanded state via self-expansion.

Optionally, one inner shaft is provided, and the interventional delivery system further includes:
two balloon bodies arranged in sequence along the inner shaft, wherein the prosthetic heart valve and the anchoring stent are respectively connected to outer peripheries of the corresponding balloon bodies; and
a locking wire for simultaneously limiting axial positions of the prosthetic heart valve and the anchoring stent relative to the corresponding balloon bodies;
wherein the step of releasing the connection between the anchoring stent and the inner shaft further includes moving the locking wire before or after inflating the balloon body corresponding to the anchoring stent to release axial limitation on the anchoring stent while maintaining axial limitation on the prosthetic heart valve; and
the step of releasing the connection between the prosthetic heart valve and the inner shaft further includes moving the locking wire along the original movement direction before or after inflating the balloon body corresponding to the prosthetic heart valve to release axial limitation on the prosthetic heart valve.

Optionally, the interventional delivery system further includes a deflection assembly, and the deflection assembly can at least act on the inner shaft to change an orientation of a distal end of the inner shaft;
before the prosthetic heart valve assembly is separated from the inner shaft, the inner shaft, under an action of the deflection assembly, has a smooth bent section or at least two smooth bent sections, and the two bent sections have opposite curvatures (taking S as an example).

The portion of the inner shaft corresponding to the bent section is made of a metal cutting tube.

The present disclosure improves the prosthetic heart valve and the interventional delivery system, and the two cooperate with each other during the interventional delivery and deployment process of the prosthetic heart valve, resulting in a more ideal operating effect.

### BRIEF DESCRIPTION OF DRAWINGS

FIG. 1 is a schematic view of a delivery system according to an embodiment of the present disclosure, with the auxiliary handle being away from the main handle;
FIG. 2 is a schematic view of the delivery system in FIG. 1, with the auxiliary handle being close to the main handle;
FIG. 3a is a schematic view of the distal part of the outer sheath in FIG. 1 in cooperation with the inner sheath;
FIG. 3b is a schematic view of the distal part being bent of a delivery system according to an embodiment of the present disclosure;
FIG. 4 is a perspective view of an auxiliary handle according to an embodiment of the present disclosure;
FIG. 5 is an exploded view of the housing and end caps of the auxiliary handle in FIG. 4;
FIG. 6 is an assembly view of the housing, the first end cap and the second end cap of the auxiliary handle in FIG. 4;
FIG. 7 is an assembly view of FIG. 6 from another perspective;
FIG. 8 is an exploded view of the remaining parts of the auxiliary handle except the housing and the end caps according to an embodiment of the present disclosure;
FIG. 9 is a structural view of an auxiliary handle according to an embodiment of the present disclosure;
FIG. 10a is an enlarged view of portion A in FIG. 9 (the sealing sheet closes the fluid gap);
FIG. 10b is an enlarged view of FIG. 10a showing the sealing sheet opening the fluid gap;
FIG. 11 is an exploded view of a first protective sleeve and a connecting sleeve according to an embodiment of the present disclosure;
FIG. 12 is a perspective view of a driving member sliding and locking according to an embodiment of the present disclosure;
FIG. 13 is a structural view of a connecting sleeve according to an embodiment of the present disclosure;
FIG. 14 is a perspective view of the auxiliary handle with the operation button sliding and unlocking according to an embodiment of the present disclosure;
FIG. 15a is a perspective view of the auxiliary handle in FIG. 14 with the operation button sliding and locking;

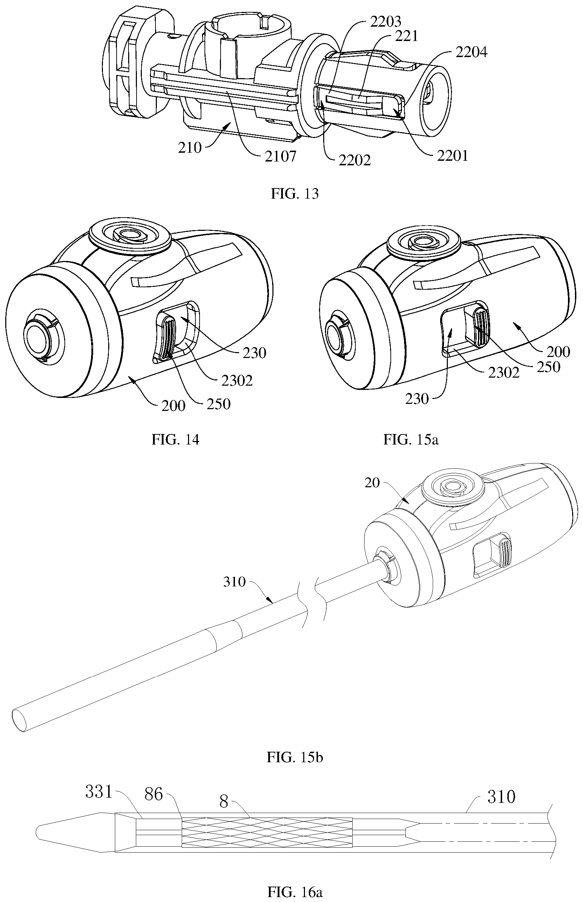

FIG. 15 b is a perspective view of the auxiliary handle and the outer sheath;

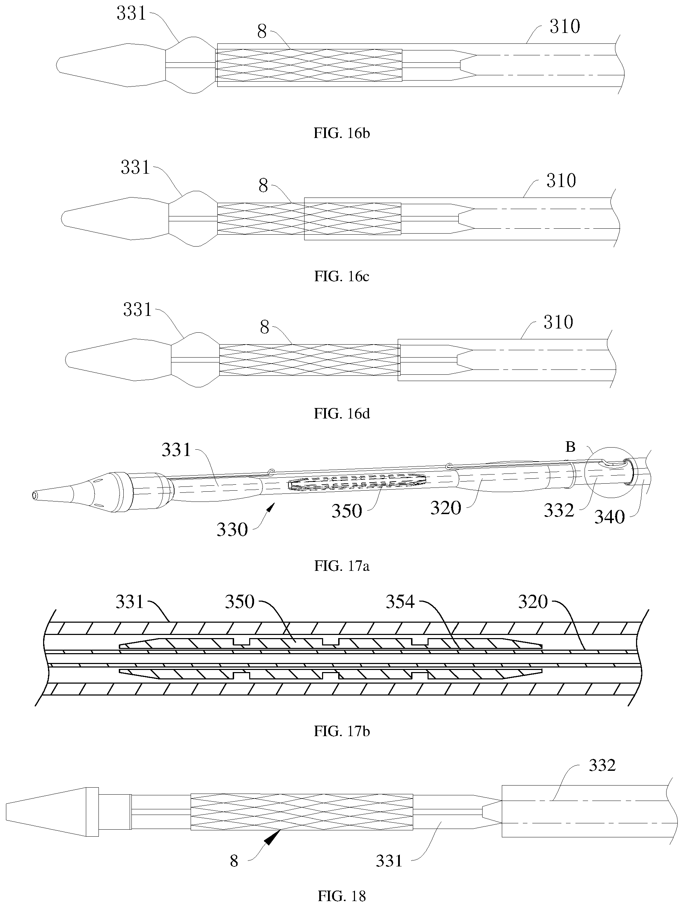

FIGS. 16a to 16d are schematic views illustrating the positioning principle of the distal end of the balloon body being inflated when the outer sheath is at different positions;
FIG. 17a is a structural view of the distal end of a catheter assembly according to an embodiment of the present disclosure;
FIG. 17b is a partial cross-sectional enlarged view of the fluid guide member in FIG. 17a;
FIG. 18 is a schematic view of a balloon body loaded with a prosthetic implant and in a folded state according to an embodiment of the present disclosure;

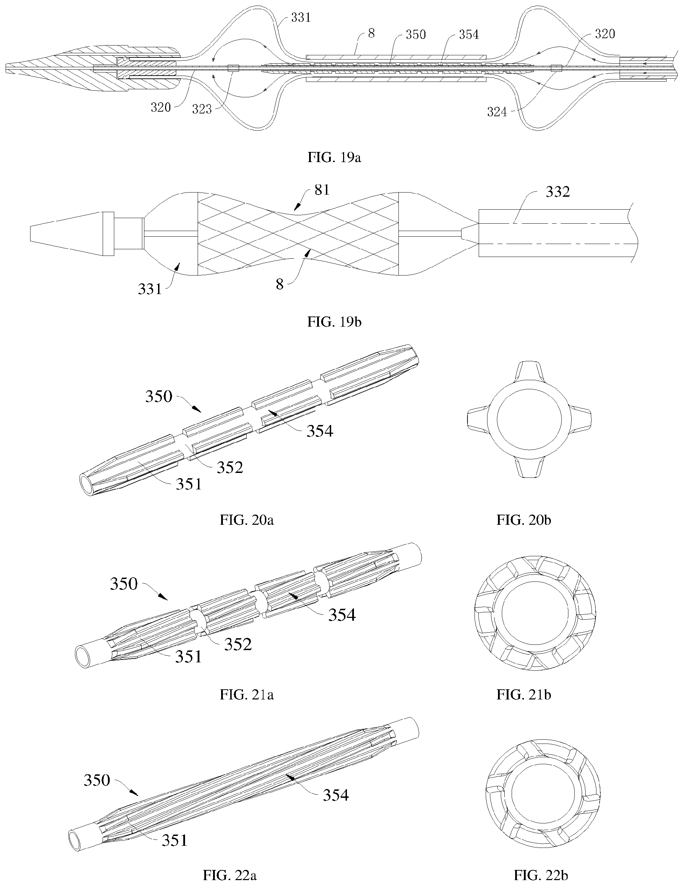

FIG. 19a is a schematic view showing the flow path of the fluid when the balloon body is filled with water and inflated according to an embodiment of the present disclosure;
FIG. 19b is a schematic view showing the balloon body in FIG. 18 being inflated with water;
FIG. 20a is a perspective view of a fluid guide member according to an embodiment of the present disclosure;
FIG. 20b is a front view of the fluid guide member in FIG. 20a;
FIG. 21a is a perspective view of a fluid guide member according to another embodiment of the present disclosure;
FIG. 21b is a front view of the fluid guide member in FIG. 21a;
FIG. 22a is a perspective view of a fluid guide member according to another embodiment of the present disclosure;
FIG. 22b is a front view of the fluid guide member in FIG. 22a;

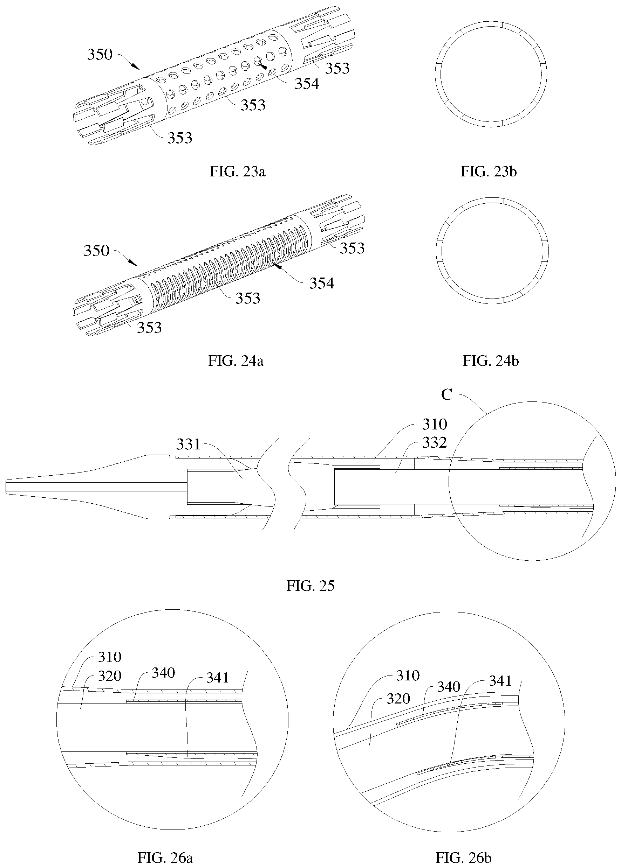

FIG. 23a is a perspective view of a fluid guide member according to another embodiment of the present disclosure;
FIG. 23b is a front view of the fluid guide member in FIG. 23a;
FIG. 24a is a perspective view of a fluid guide member according to another embodiment of the present disclosure;
FIG. 24b is a front view of the fluid guide member in FIG. 24a;
FIG. 25 is a schematic view showing the interior of a balloon body according to an embodiment of the present disclosure;
FIG. 26a is an enlarged view of one example of the portion C in FIG. 25;
FIG. 26b is a schematic view of FIG. 26a when being bent;

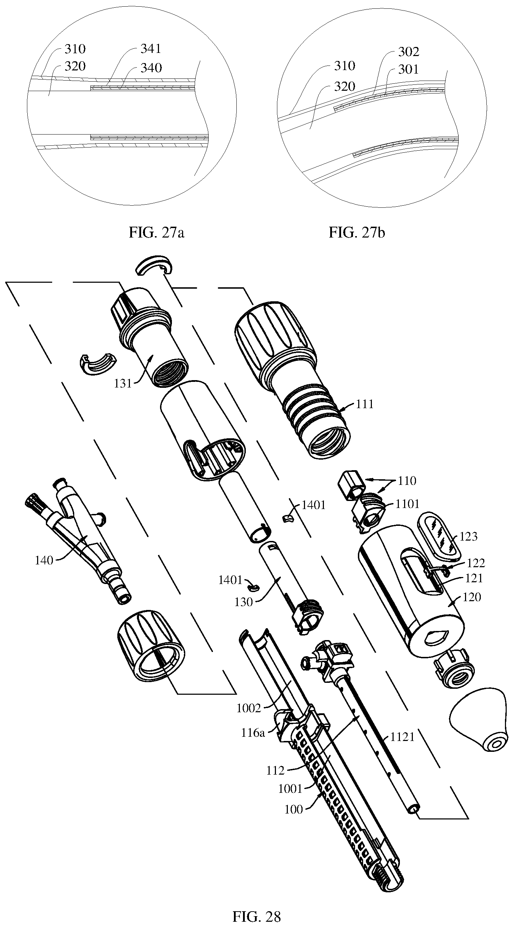

FIG. 27a is an enlarged view of another example of portion C in FIG. 25;
FIG. 27b is a schematic view of FIG. 27a when being bent;
FIG. 28 is an exploded view of a main handle according to an embodiment of the present disclosure;

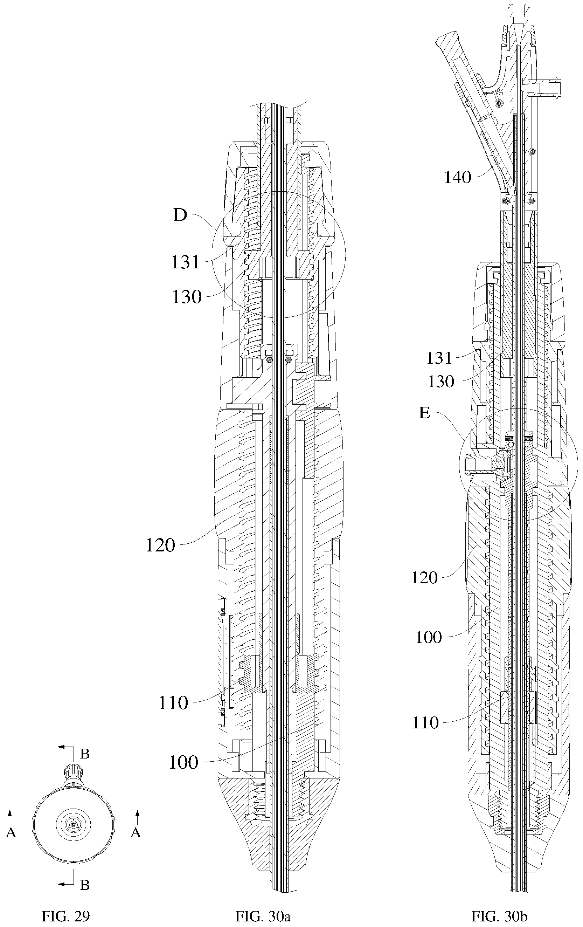

FIG. 29 is a front view of a main handle according to an embodiment of the present disclosure;
FIG. 30a is a cross-sectional view along A-A in FIG. 29;
FIG. 30b is a cross-sectional view along B-B in FIG. 29;

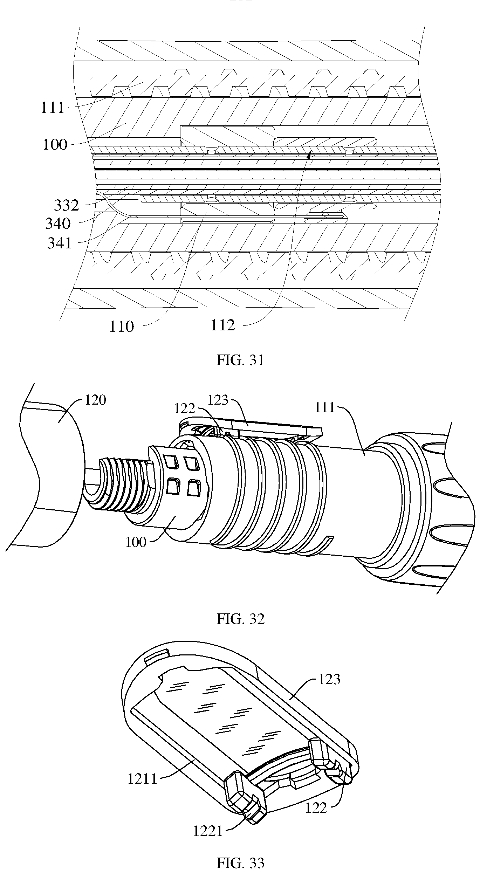

FIG. 31 is a partial enlarged view of the first sliding seat in FIG. 30b;
FIG. 32 is a partial exploded view of the identification member according to an embodiment of the present disclosure;
FIG. 33 is a schematic view showing the identification member engaging with the indicating section according to an embodiment of the present disclosure;

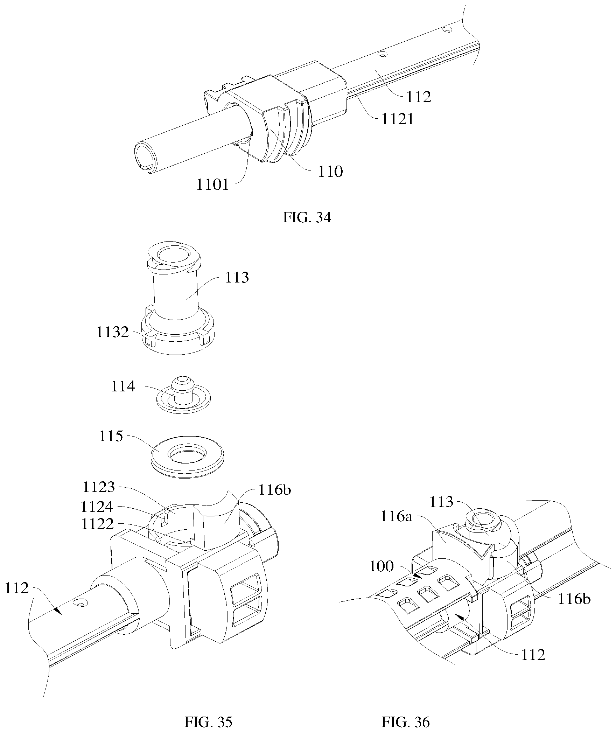

FIG. 34 is a schematic view showing the first sliding seat engaging with the guide cylinder according to an embodiment of the present disclosure;
FIG. 35 is an exploded view at a second pipe joint according to an embodiment of the present disclosure;
FIG. 36 is an assembled view of FIG. 35;

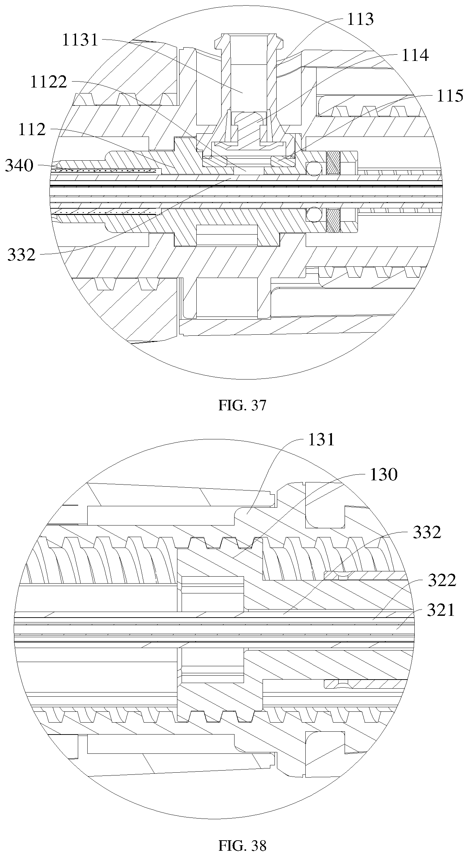

FIG. 37 is an enlarged view of portion E in FIG. 30b;
FIG. 38 is an enlarged view of portion D in FIG. 30a;

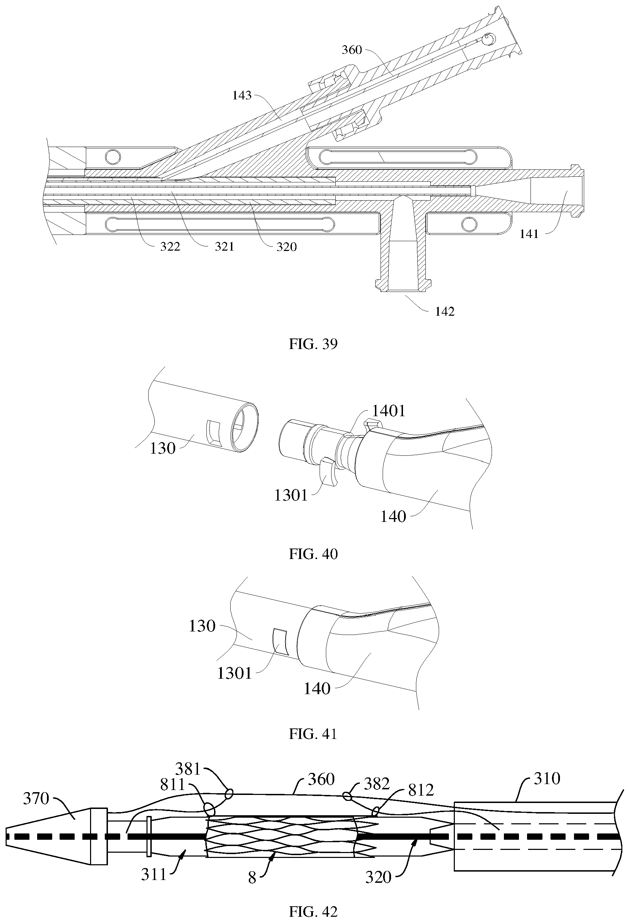

FIG. 39 is a partial cross-sectional view of the multi-way connector in FIG. 30b;
FIG. 40 is an exploded view of a multi-way connector and a second sliding seat according to an embodiment of the present disclosure;
FIG. 41 is an assembled view of FIG. 40;
FIG. 42 is a schematic view of the distal end of a delivery system with a locking wire according to an embodiment of the present disclosure;

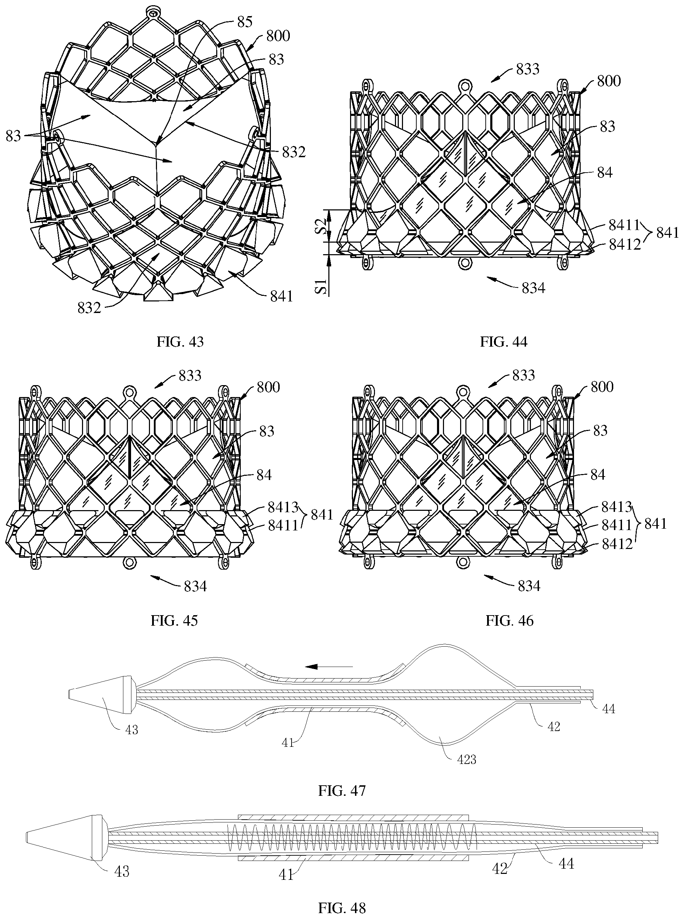

FIG. 43 is a perspective view of a prosthetic implant according to an embodiment of the present disclosure;
FIG. 44 is a front view of the prosthetic implant in FIG. 43;
FIG. 45 is a front view of a prosthetic implant according to another embodiment of the present disclosure;
FIG. 46 is a front view of a prosthetic implant according to another embodiment of the present disclosure;
FIGS. 47 to 48 are schematic views of balloon catheters in the prior art;

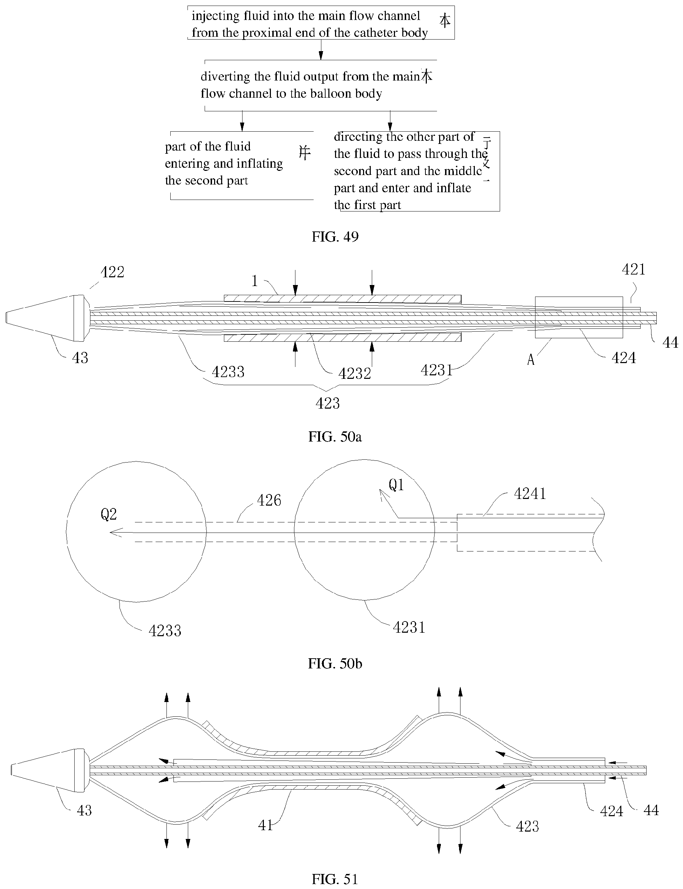

FIG. 49 is a flowchart of a method for driving a balloon catheter with fluid according to an embodiment of the present disclosure;
FIG. 50a is a schematic view of a balloon catheter before fluid is injected according to an embodiment of the present disclosure;
FIG. 50b is a schematic view showing the principle of a method for driving a balloon catheter with fluid according to an embodiment of the present disclosure;
FIG. 51 is a schematic view of the balloon catheter in the transitional state after fluid is injected into it;

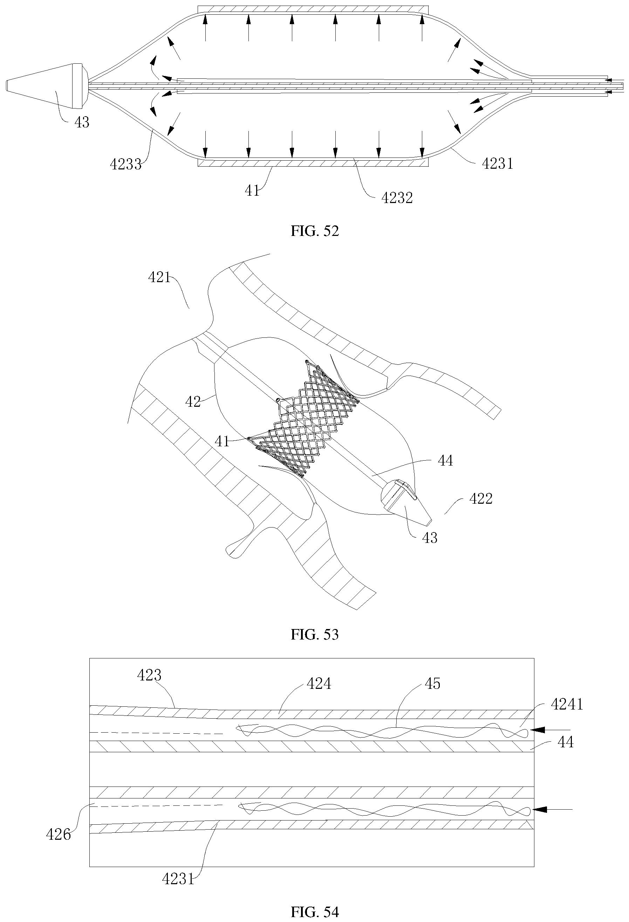

FIG. 52 is a schematic view of a balloon catheter after being fully inflated;
FIG. 53 is a schematic view of a prosthetic implant being placed in the human body (taking the aortic valve as an example);
FIG. 54 is an enlarged view of the portion A in FIG. 50a, wherein the fluid has not yet begun to divert;

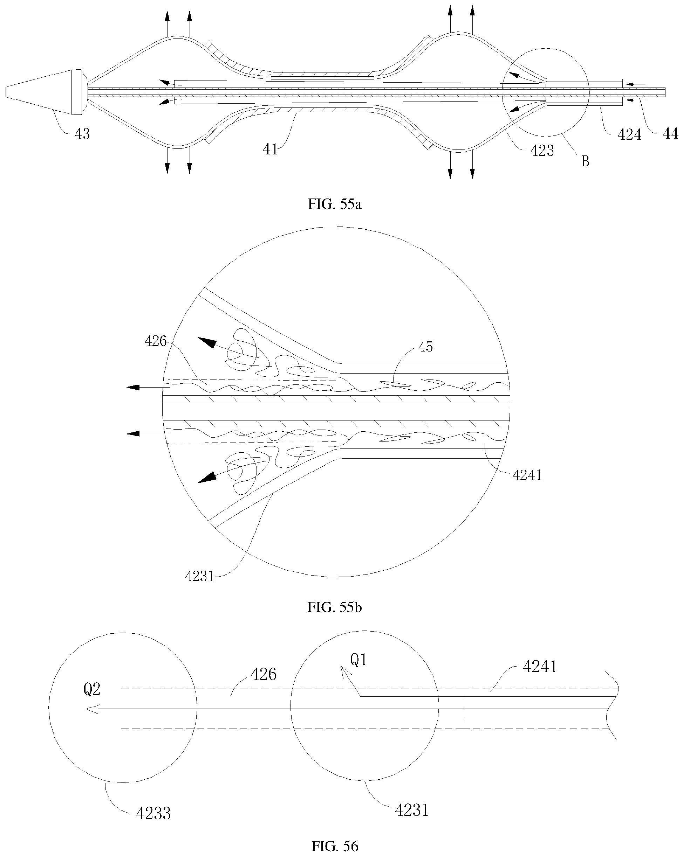

FIG. 55a is a schematic view illustrating a first fluid diversion method;
FIG. 55b is an enlarged view of the portion B in FIG. 55a, wherein the fluid is diverted according to the first diversion method;
FIG. 56 is a schematic view illustrating the principle of a second fluid diversion method;

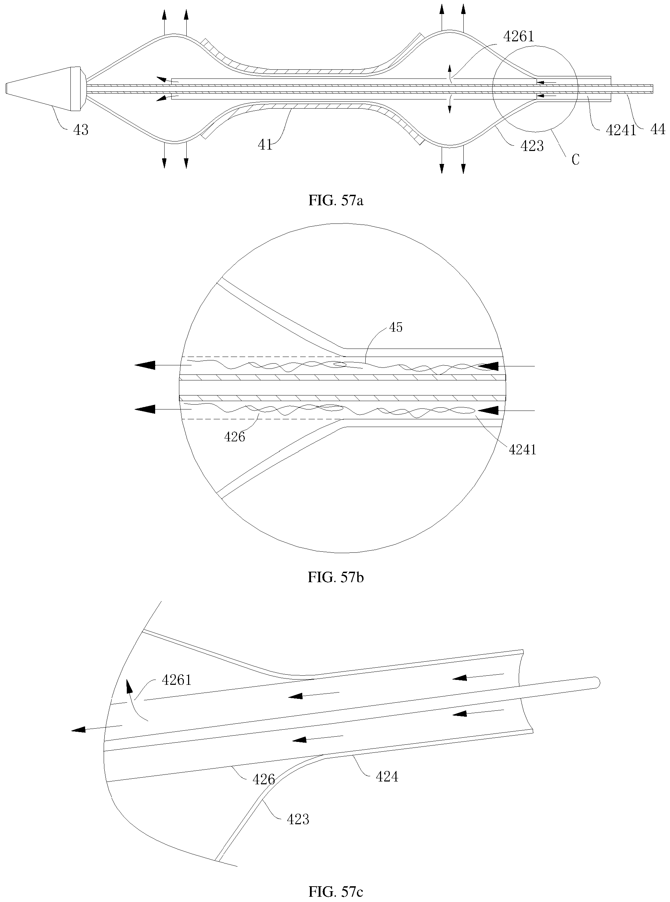

FIG. 57a is a schematic view illustrating a second fluid diversion method;
FIG. 57b is an enlarged view of the portion C in FIG. 57a, wherein the fluid has not yet begun to divert;
FIG. 57c is a schematic view illustrating the flow direction of the fluid in FIG. 57a after being diverted;

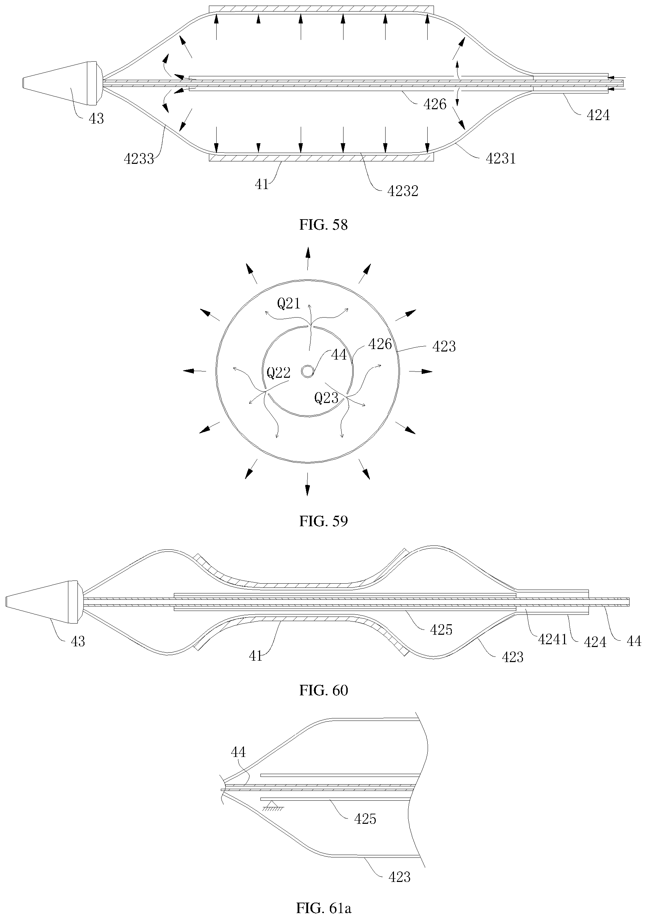

FIG. 58 is a schematic view of a fully inflated balloon catheter provided in the present disclosure;
FIG. 59 is a schematic cross-sectional view of the flow channel of a balloon catheter provided in the present disclosure;
FIG. 60 is a schematic view of an embodiment of a delivery device for interventional device provided in the present disclosure;
FIG. 61a is a schematic view illustrating the principle of fixing the distal end of the flow guide tube relative to the core tube in the delivery device for interventional device provided in the present disclosure;

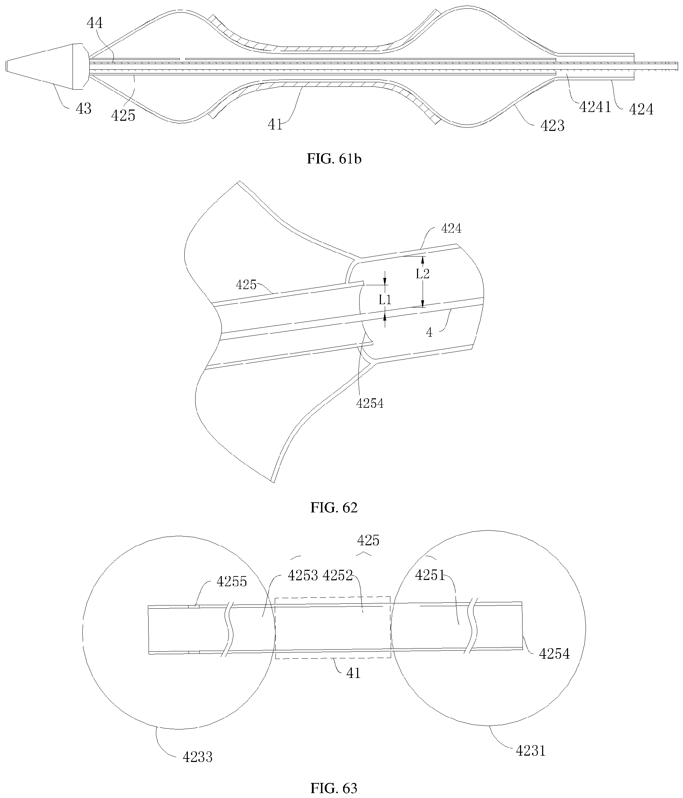

FIG. 61b is a schematic view showing the distal end of the flow guide tube being fixed relative to the core tube in the delivery device for interventional device provided in the present disclosure;
FIG. 62 is a partial cross-sectional view of a flow guide tube and a catheter in the delivery device for interventional device provided in the present disclosure;
FIG. 63 is a schematic view of the flow guide tube in the delivery device for interventional device provided in the present disclosure;

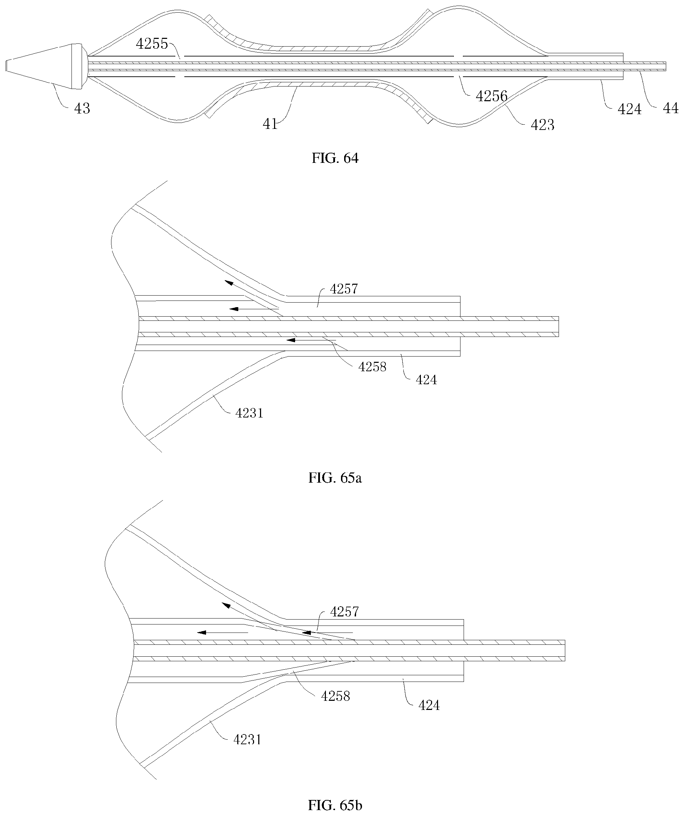

FIG. 64 is a schematic view of another embodiment of a delivery device for interventional device provided in the present disclosure;
FIG. 65a is a partial cross-sectional view of a tapered section of an embodiment of a delivery device for interventional device provided in the present disclosure;
FIG. 65b is a partial cross-sectional view of a tapered section of another embodiment of a delivery device for interventional device provided in the present disclosure;

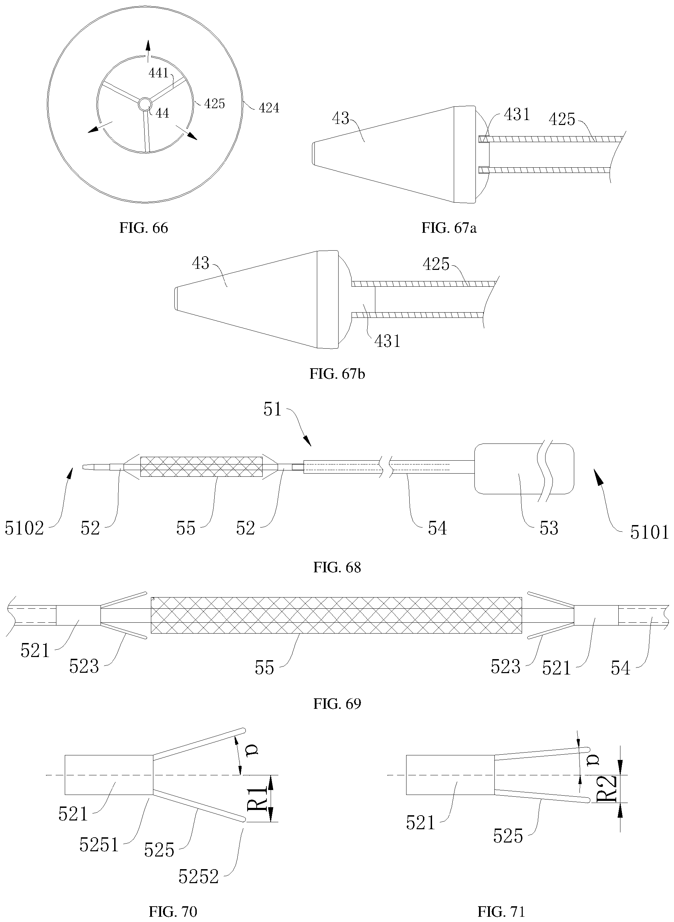

FIG. 66 is a schematic view of a spacer in a delivery device for interventional device provided in the present disclosure;
FIG. 67a is a schematic view of a positioning structure of an embodiment of a delivery device for interventional device provided in the present disclosure;
FIG. 67b is a schematic view of a positioning structure of another embodiment of a delivery device for interventional device provided in the present disclosure;
FIG. 68 is a schematic view of an interventional delivery system according to an embodiment of the present disclosure;
FIG. 69 is a schematic view of the distal part in FIG. 68;
FIG. 70 is a schematic view of the rods of the limiting mechanism in FIG. 69 in an expanded state;
FIG. 71 is a schematic view of the rods of the limiting mechanism in FIG. 70 in a compressed state;

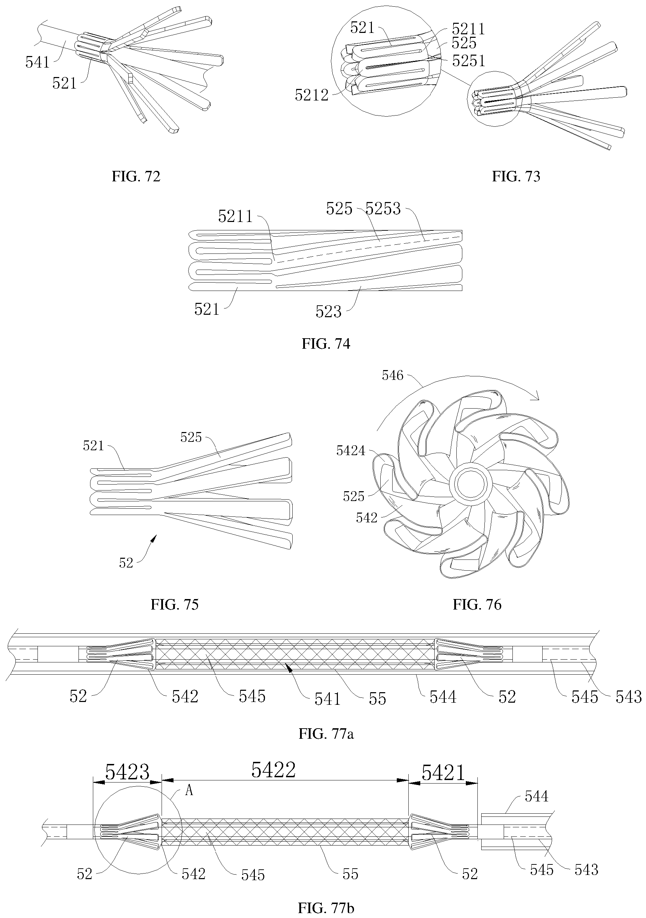

FIG. 72 is a schematic view of the limiting mechanism matching the inner shaft according to an embodiment of the present disclosure;
FIG. 73 is a schematic view of the limiting mechanism in FIG. 72;
FIG. 74 is a schematic view of the limiting mechanism in FIG. 72 in a straight cylindrical shape;
FIG. 75 is a front view of the limiting mechanism in FIG. 72 in a compressed state or an intermediate state;
FIG. 76 is a side view of the balloon body in a folded state cooperating with the limiting mechanism in FIG. 75;
FIG. 77a is a schematic view of a distal end (with a balloon device) of a delivery system with a sheath according to another embodiment of the present disclosure;
FIG. 77b is a schematic view of FIG. 77a after the sheath moves to one end;

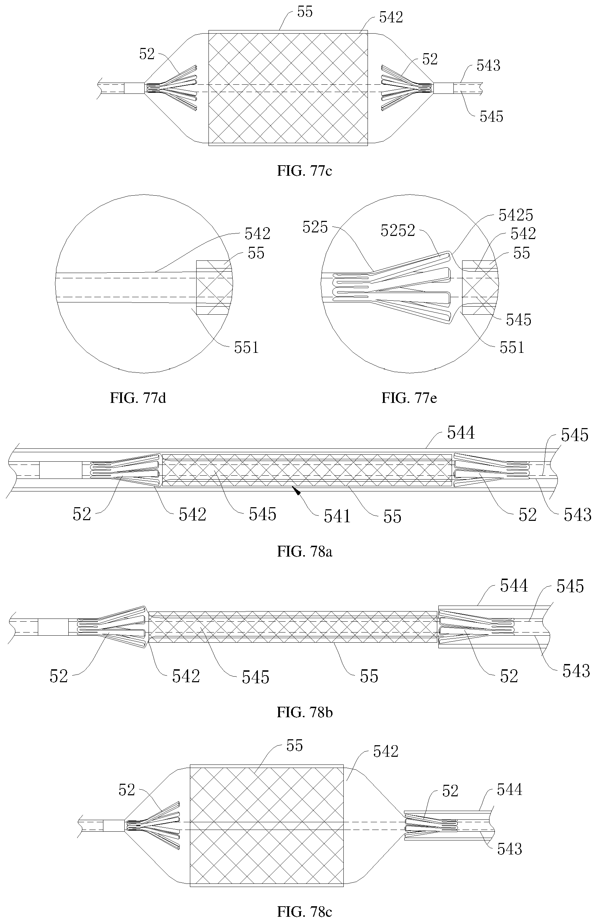

FIG. 77c is a schematic view after the balloon body in FIG. 77a is inflated;
FIG. 77d is an enlarged view showing a step created when a conventional prosthetic implant is installed on a balloon body;
FIG. 77e is an enlarged view of portion A in FIG. 77b;
FIG. 78a is a schematic view of the distal end of a delivery system according to another embodiment of the present disclosure;
FIG. 78b is a schematic view showing the sheath moving and covering the proximal limiting mechanism in FIG. 78a;
FIG. 78c is a schematic view after the balloon body in FIG. 78b is inflated;

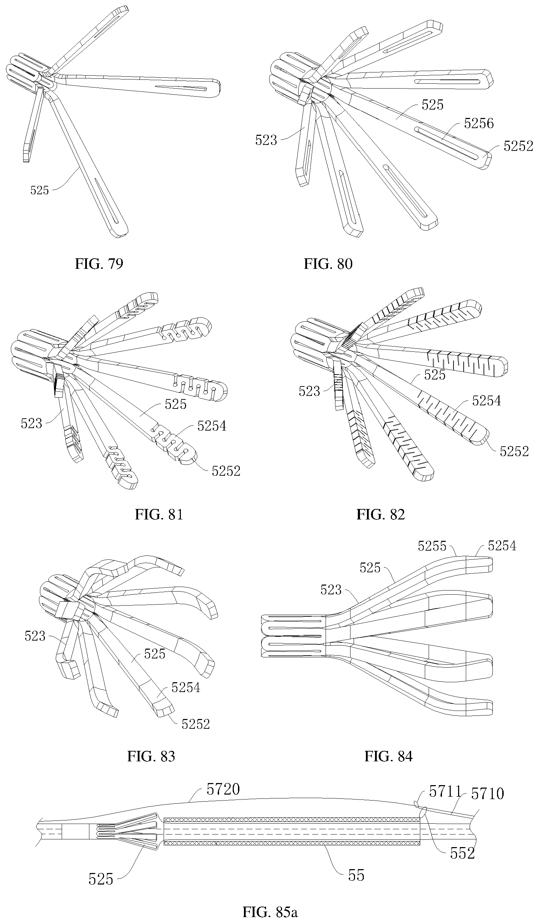

FIGS. 79 to 83 are schematic views of other embodiments of the limiting mechanisms of the present disclosure;
FIG. 84 is a front view of the limiting mechanism of FIG. 83;
FIGS. 85a to 85c are schematic views of different embodiments of the distal end of the delivery system of the present disclosure;

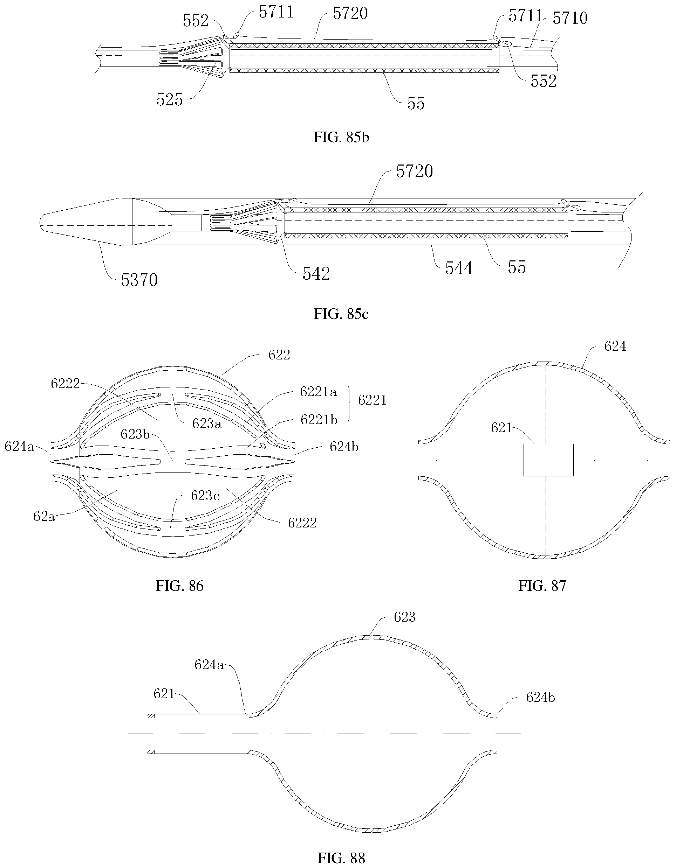

FIG. 86 is a schematic view of a limiting mechanism according to an embodiment of the present disclosure;
FIGS. 87 to 89 are schematic views showing the distribution of the coupling member according to an embodiment of the present disclosure;

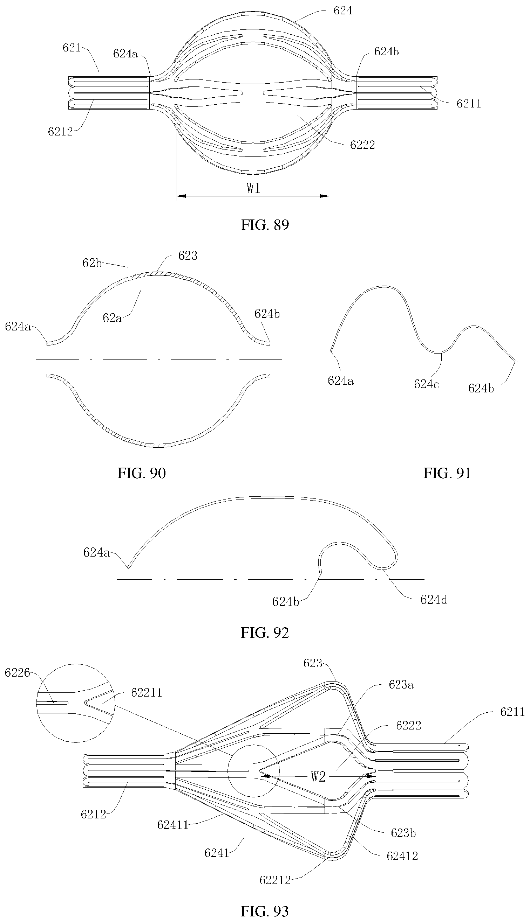

FIG. 90 is a schematic longitudinal cross-sectional view of the limiting mechanism in FIG. 86;
FIGS. 91 and 92 are schematic views showing the converging portions of the cage-like structures according to some embodiments of the present disclosure;
FIG. 93 is a schematic view of a limiting mechanism according to another embodiment of the present disclosure;

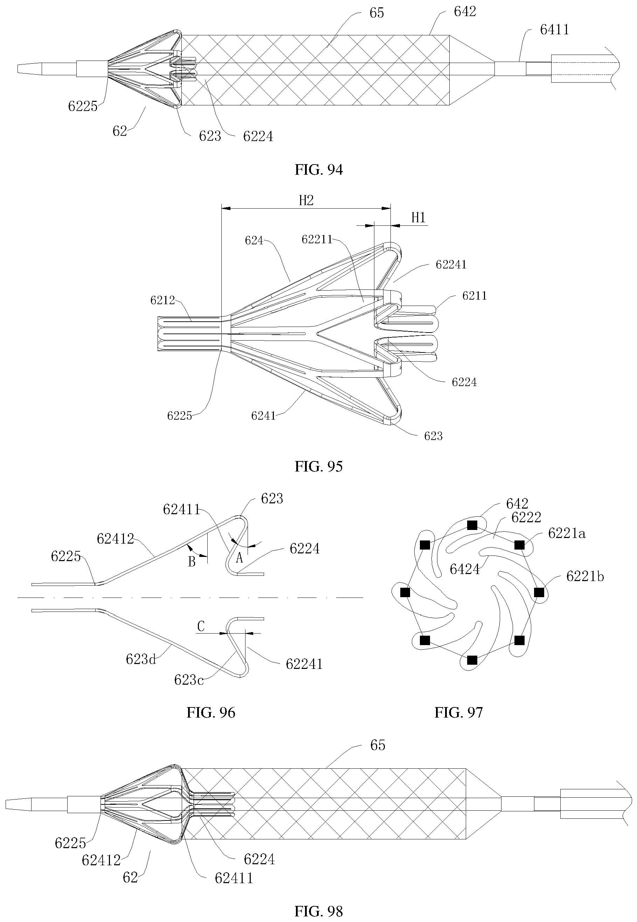

FIG. 94 is a schematic view of a balloon device according to an embodiment of the present disclosure;
FIG. 95 is a schematic view of the limiting mechanism in FIG. 94;
FIG. 96 is a schematic longitudinal cross-sectional view of the limiting mechanism in FIG. 95;
FIG. 97 is a schematic transverse cross-sectional view of the limiting mechanism in FIG. 86;
FIG. 98 is a schematic view of a balloon device according to another embodiment of the present disclosure;

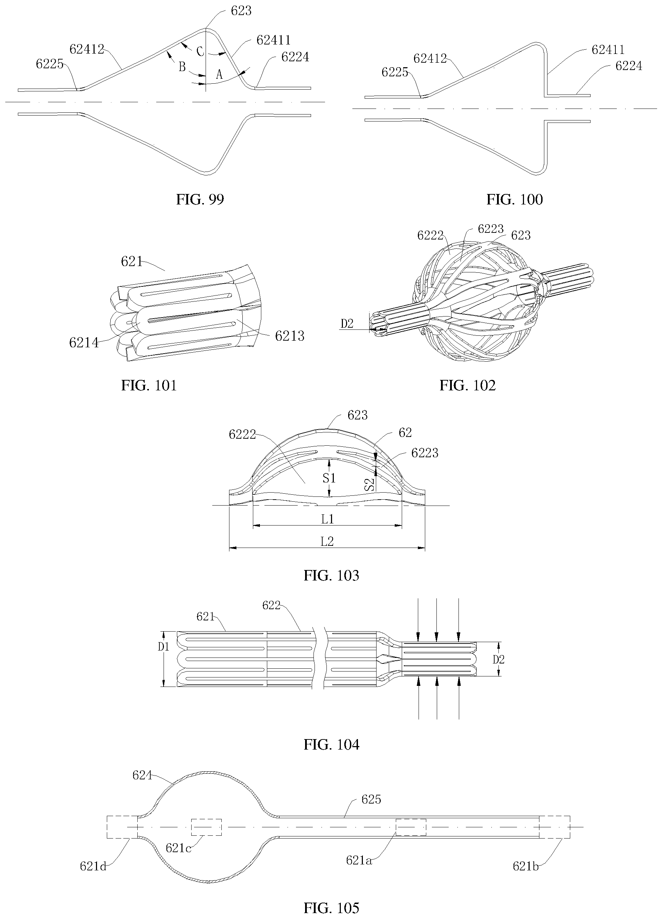

FIG. 99 is a schematic view of the limiting mechanism in FIG. 98;
FIG. 100 is a schematic longitudinal cross-sectional view of a limiting mechanism according to another embodiment of the present disclosure;
FIG. 101 is a schematic view of a coupling member according to another embodiment of the present disclosure;
FIG. 102 is a perspective view of a blocking member according to another embodiment of the present disclosure;
FIG. 103 is a partial cross-sectional view of the limiting body in FIG. 102;
FIG. 104 is a schematic view of the limiting mechanism in FIG. 102 formed in one piece by cutting a tube;
FIGS. 105 to 106 are schematic views of a limiting mechanism according to another embodiment of the present disclosure;

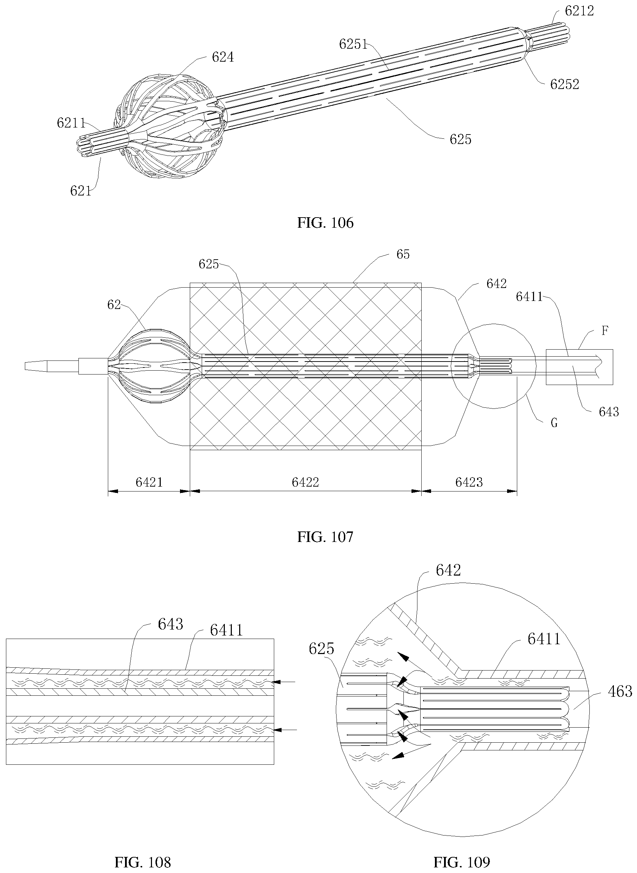

FIG. 107 is a schematic view of an inflated balloon device with the limiting mechanism in FIG. 106;
FIG. 108 is a partial enlarged view of portion F in FIG. 107;
FIG. 109 is a partial enlarged view of portion G in FIG. 107;

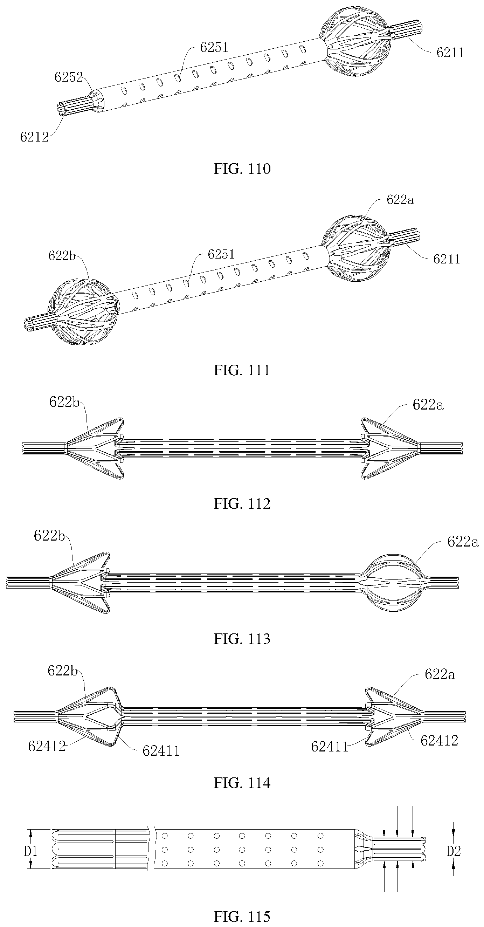

FIG. 110 is a perspective view of a blocking member according to another embodiment of the present disclosure;
FIG. 111 is a schematic view of a blocking member according to another embodiment of the present disclosure;
FIG. 112 is a schematic view of a blocking member according to another embodiment of the present disclosure;
FIG. 113 is a schematic view of a blocking member according to another embodiment of the present disclosure;
FIG. 114 is a schematic view of a blocking member according to another embodiment of the present disclosure;
FIG. 115 is a schematic view of the limiting mechanism in FIG. 110 formed in one piece by cutting a tube;

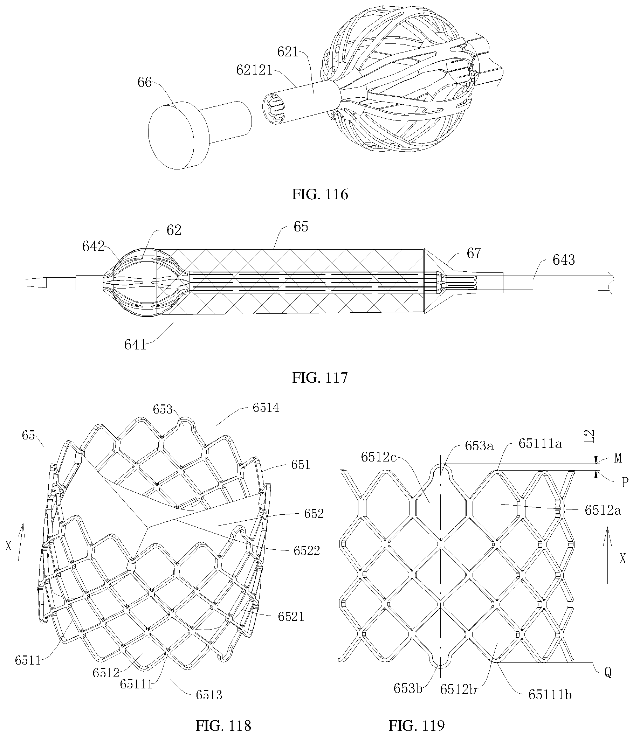

FIG. 116 is a schematic view of the coupling member mating with the tubular end according to an embodiment of the present disclosure;
FIG. 117 is a schematic view of a transcatheter implant system according to an embodiment of the present disclosure;
FIG. 118 is a schematic view of a prosthetic heart valve according to an embodiment of the present disclosure;
FIG. 119 is a schematic partial view of the first stent in FIG. 118;

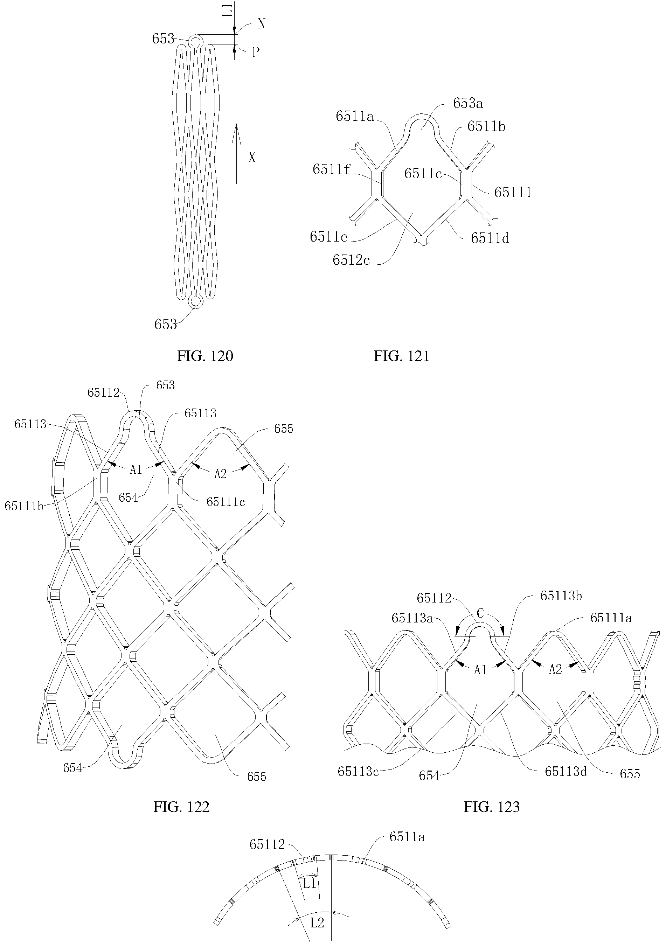

FIG. 120 is a schematic partial view of the first stent in FIG. 118 after being radially compressed;
FIG. 121 is a schematic view showing the eyelet structure of the first stent in FIG. 118;
FIG. 122 is another schematic partial view of the first stent in FIG. 118;
FIG. 123 is a schematic view showing the eyelet structure in FIG. 122;

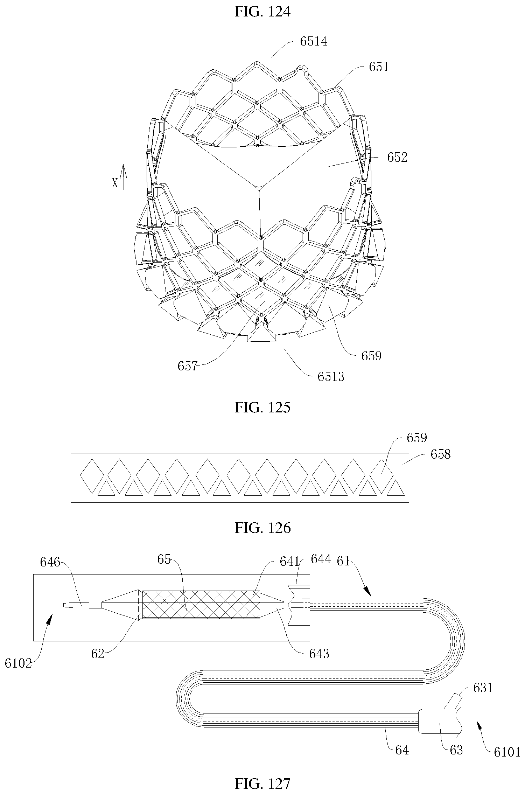

FIG. 124 is a schematic view of the first stent in FIG. 123 from another perspective;
FIG. 125 is a schematic view of a prosthetic heart valve according to another embodiment of the present disclosure;
FIG. 126 is a schematic view of the sealing member in FIG. 125 formed in one piece;
FIG. 127 is a schematic view of a delivery system of the present disclosure;

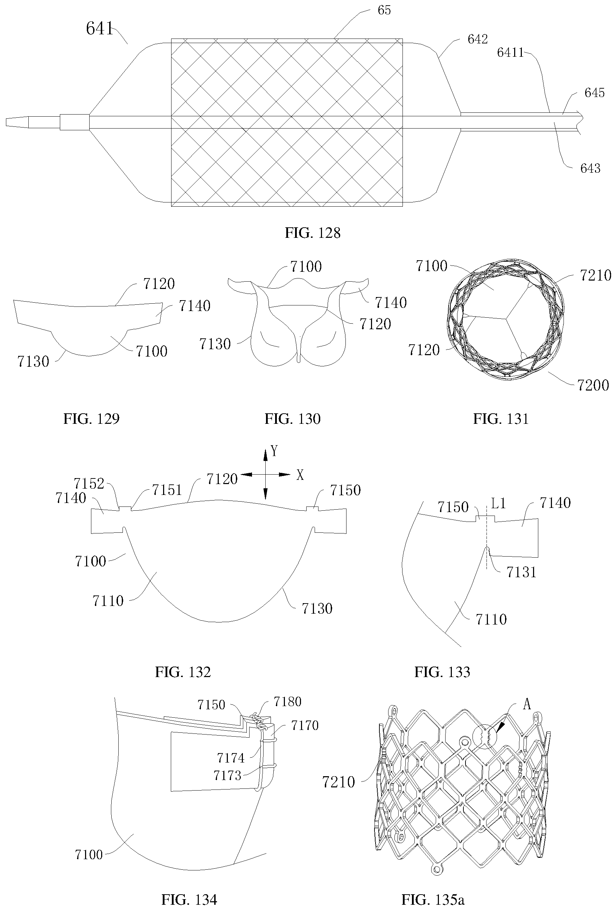

FIG. 128 is a schematic view of the balloon body in FIG. 127 in the inflated state;
FIG. 129 is a schematic view of a flattened leaflet in the prior art;
FIG. 130 is a schematic view of multiple leaflets after suturing in FIG. 129;
FIG. 131 is a schematic view of a prosthetic heart valve in the prior art;
FIG. 132 is a schematic view of a leaflet of a prosthetic heart valve in a flattened state according to an embodiment of the present disclosure;
FIG. 133 is a schematic view of the outward-protruding portion and the opening area in FIG. 132;
FIG. 134 is a schematic view showing the joints sutured using the first suture;
FIG. 135a is a schematic view of a stent according to an embodiment of the present disclosure;

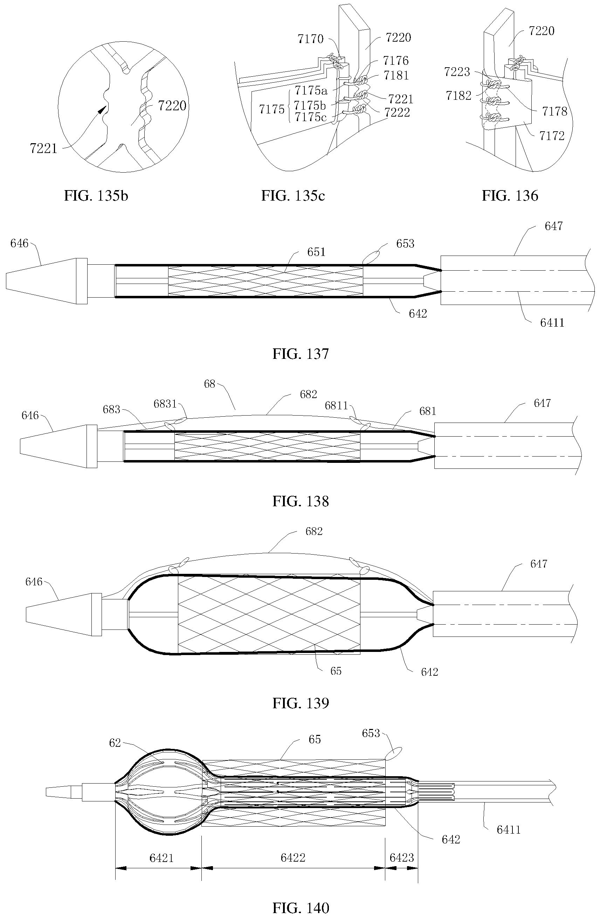

FIG. 135b is an enlarged view of portion A in FIG. 135a;
FIG. 135c is a schematic view showing two joints and a support bar sutured using a plurality of second sutures;
FIG. 136 is a schematic view showing the commissure tabs of the leaflets wrapping around the support bar and being sutured using a plurality of third sutures;
FIGS. 137 to 139 are schematic views of a transcatheter implant system with a locking wire structure according to an embodiment of the present disclosure;
FIG. 140 is a schematic view of a transcatheter implant system according to an embodiment of the present disclosure;

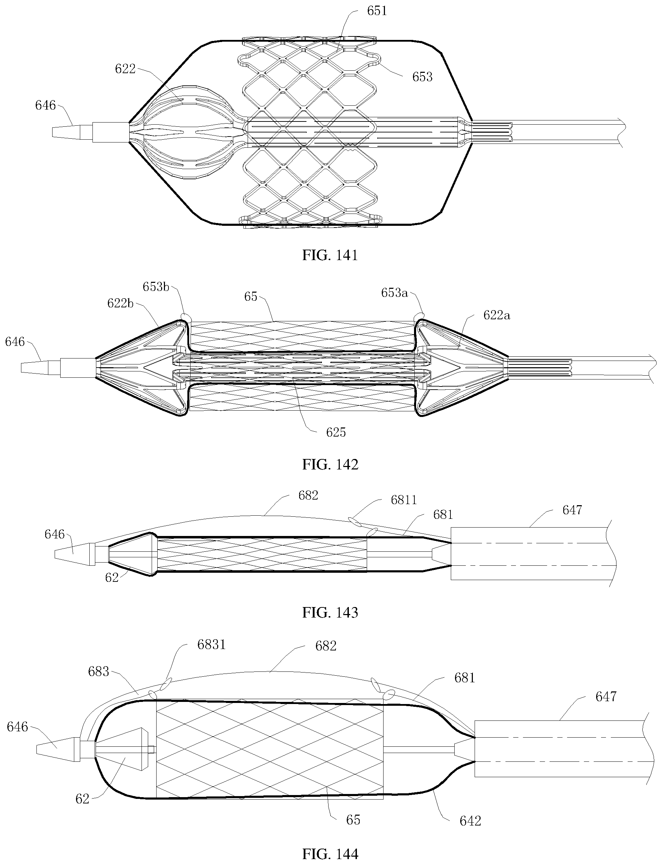

FIG. 141 is a schematic view of the balloon body in FIG. 140 in an inflated state;
FIG. 142 is a schematic view of a transcatheter implant system according to another embodiment of the present disclosure;
FIGS. 143 to 144 are schematic views of a transcatheter implant system with a locking wire structure and a limiting mechanism according to an embodiment of the present disclosure;

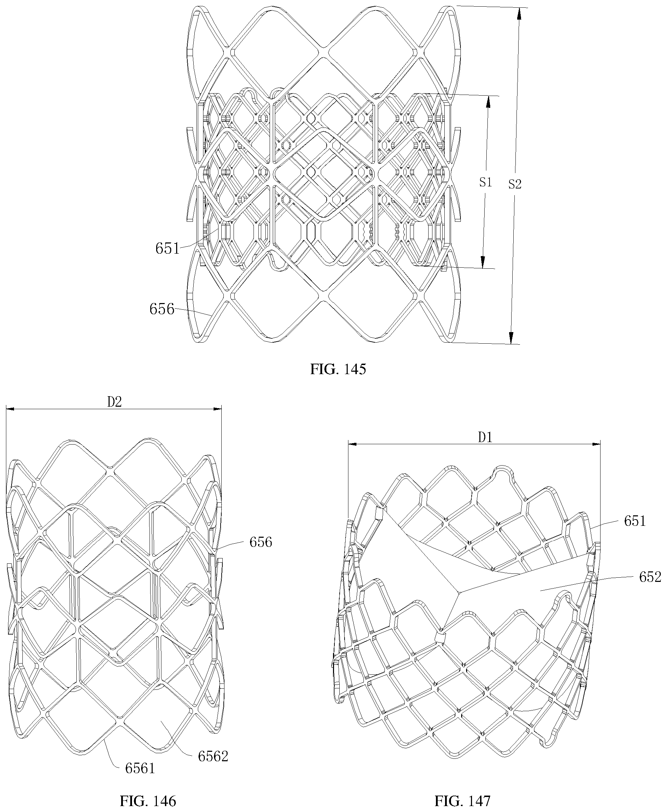

FIG. 145 is a schematic view of a prosthetic heart valve according to another embodiment of the present disclosure;
FIG. 146 is a schematic view of the anchoring stent in FIG. 145;
FIG. 147 is a schematic view of the first stent in FIG. 145;

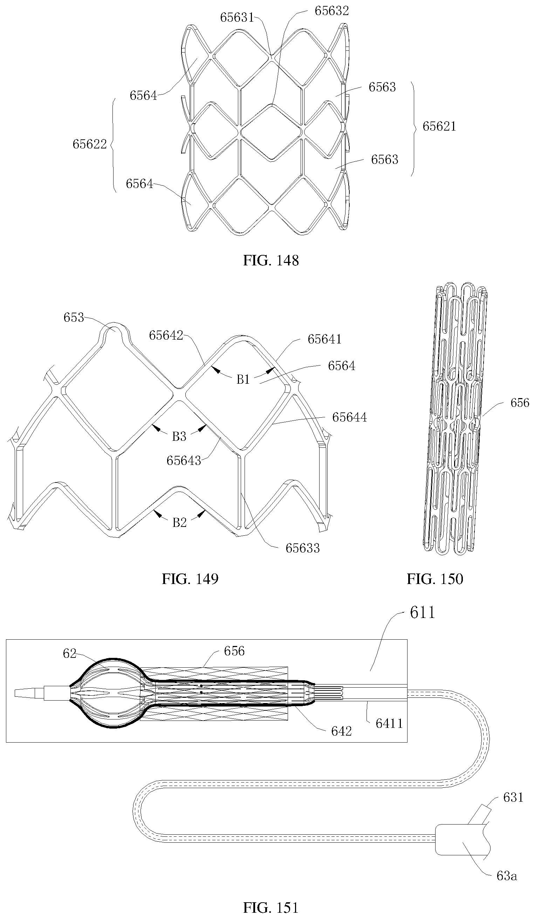

FIG. 148 is a schematic partial view of FIG. 146;
FIG. 149 is a schematic view showing an eyelet structure for the anchoring stent in FIG. 148;
FIG. 150 is a schematic view of the anchoring stent in FIG. 146 being radially compressed;
FIGS. 151 to 156 are schematic views of transcatheter implant systems according to other embodiments of the present disclosure;

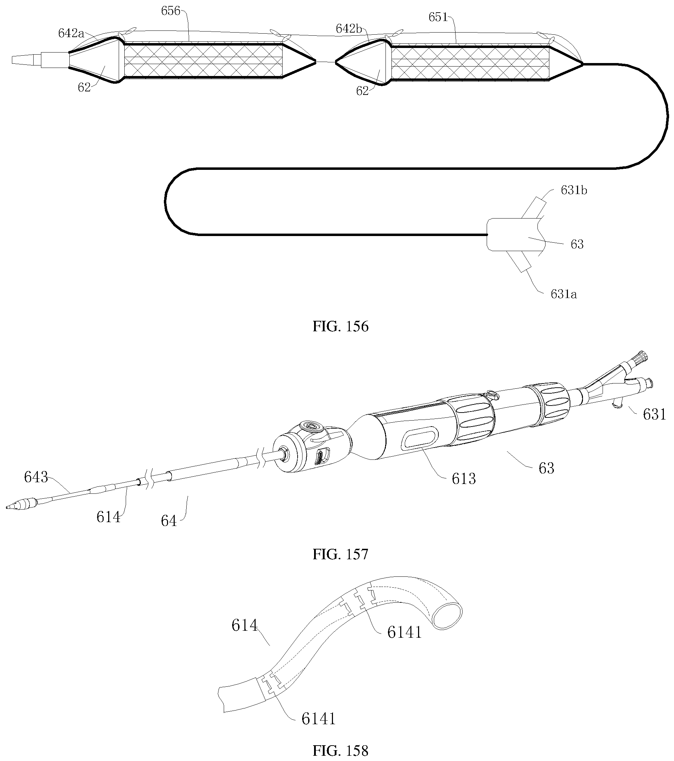

FIG. 157 is a schematic view of a transcatheter implant system with a deflection assembly according to other embodiments of the present disclosure; and
FIG. 158 is a schematic view of the deflectable tube in FIG. 156 in a bent state.

### List of reference numerals in the figures:

1, interventional delivery system;
8, prosthetic implant; 800, stent; 811, first eyelet; 812, second eyelet; 82, waist-like structure; 83, leaflet; 831, fixed edge; 832, free edge; 833, outflow side; 834, inflow side; 84, base; 841, anti-paravalvular leakage element; 8411, first row of anti-paravalvular leakage element; 8412, second row of anti-paravalvular leakage element; 8413, third row of anti-paravalvular leakage element; 85, blood flow channel; 86, protruding step;
9, control handle; 10, main handle; 100, support body; 1001, installation groove; 1002, guide groove;
110, first sliding seat; 1101, slide groove; 111, first driving sleeve;
112, guide cylinder; 1121, guide strip; 1122, second vent hole; 1123, second annular seat; 1124, slot;
113, second pipe joint; 1131, second vent channel; 1132, positioning rib;
114, one-way valve core; 115, sealing ring; 116, second protective sleeve; 116a, first part; 116b, second part;
120, housing; 121, frame; 1211, slide groove; 122, identification member; 1221, inner tooth; 123, transparent cover plate;
130, second sliding seat; 1301, latching tooth; 131, second driving sleeve;
140, multi-way connector; 1401, latching groove; 141, interface; 142, interface;
20, auxiliary handle; 200, housing; 201, threading channel; 202, half-shell; 2021, screwing-in latching groove; 2022, plug-in latching groove; 2023, positioning groove;
203, end cap; 203a, first end cap; 203b, second end cap; 2031, through hole; 2032, latching tooth; 2033, elastic snap;
210, connecting sleeve; 2101, positioning shoulder; 2102, first vent hole; 2103, first vent channel; 2104, first annular seat; 2105, positioning rib; 2106, reinforcement hole; 2107, slide groove;
211, first pipe joint; 2111, slot; 2112, retaining ring; 212, first protective sleeve; 213, sealing ring; 214, fluid gap;
220, positioning member; 2201, free end; 2202, fixed end; 2203, guide slope; 2204, hollow area; 221, elastic claw;
230, driving member; 2301, guide rib; 2302, first clearance opening; 2303, second clearance opening; 240, elastic liner; 2401, positioning step; 250, operation button;
26, one-way valve core; 261, limiting rod; 2611, head; 262, sealing sheet;
30, catheter assembly; 310, outer sheath; 3101, insertion section; 3102, loading section; 3103, sheath section; 320, inner sheath; 321, guidewire channel; 322, fluid channel; 323, second radiopaque ring; 324, third radiopaque ring;
330, balloon catheter; 331, balloon body; 332, catheter body;
340, deflectable tube; 341, traction member;
350, fluid guide member; 351, diversion groove; 352, communication groove; 353, hollow area; 354, diversion channel;
360, locking wire; 370, guide head; 3701, annular step; 381, first pull ring; 382, second pull ring,
41, prosthetic implant; 42, balloon catheter; 421, proximal end; 422, distal end; 423, balloon body; 4231, second part; 4232, middle part; 4233, first part; 424, catheter body; 4241, main flow channel; 425, flow guide tube; 4251, proximal section; 42511, fluid outlet; 4252, middle section; 4253, distal section; 4254, fluid inlet; 4255, first fluid outlet; 4256, second fluid outlet; 4257, tapered section; 4258, beveled surface; 426, independent flow channel; 43, guide head; 431, positioning structure; 44, inner shaft; 441, spacer; 45, fluid,
51, interventional delivery system; 5101, proximal end; 5102, distal end;
52, limiting mechanism; 521, coupling portion; 5211, peak; 5212, valley;
523, deformable portion; 525, rod; 5251, first end; 5252, second end; 5253, helical path; 5254, abutment portion; 5255, end portion; 5256, slot;
53, control handle; 54, catheter assembly; 541, balloon device; 542, balloon body; 5421, proximal section; 5422, middle section; 5423, distal section; 5424, fold; 5425, protrusion; 5425, distal part; 5426, proximal part; 543, intermediate shaft; 544, outer sheath; 545, inner shaft; 546, first direction;
55, prosthetic implant; 551, step; 552, eyelet;
5710, adjustment wire; 5711, locking eyelet; 5720, locking wire;
61, transcatheter implant system; 6101, proximal end; 6102, distal end; 611, first delivery system; 612, second delivery system; 613, deflection module; 614, deflectable tube; 6141, bent section;
62, limiting mechanism; 62a, internal space; 62b, external space; 621, coupling member; 621a, coupling member; 621b, coupling member; 621c, coupling member; 621d, coupling member; 6211, first coupling member; 6212, second coupling member; 62121, sleeve; 6213, peak; 6214, valley; 622, limiting body; 622a, limiting body; 622b, limiting body; 6221, rod; 62211, branch; 62212, junction point; 6222, main hollow area; 6223, auxiliary hollow area; 6224, first end; 62241, concave area; 6225, second end; 6226, slit; 623, outward expansion part; 623a, rod; 623b, rod; 623c, rod; 623d, rod; 623e, rod; 624, cage-like structure; 624a, converging portion; 624b, converging portion; 624c, converging portion; 624d, converging portion; 6241, cone; 62411, first cone; 62412, second cone; 625, guide tube; 6251, fluid inlet; 6252, diameter-reduced section;
63, control handle; 63a, control handle; 63b, control handle; 631, interface; 631a, interface; 631b, interface;
64, catheter assembly; 641, balloon device; 6411, catheter body; 642, balloon body; 642a, second balloon body; 642b, first balloon body; 6421, first part; 6422, middle part; 6423, second part; 6424, folded portion; 643, inner shaft; 644, outer sheath; 645, fluid inlet; 646, guide head;
65, prosthetic heart valve; 65a, prosthetic implant; 651, first stent; 6511, strut; 6511a, strut; 6511b, strut; 6511c, strut; 6511d, strut; 6511e, strut; 6511f, strut; 65111, node; 65111a, node; 65111b, node; 65111c, node; 65112, arc segment; 65113, connecting segment; 65113a, connecting segment; 65113b, connecting segment; 65113c, connecting segment; 65113d, connecting segment; 6512, cell; 6512a, cell; 6512b, cell; 6512c, cell; 6513, inflow side; 6514, outflow side; 652, leaflet; 6521, fixed edge; 6522, free edge; 653a, eyelet structure; 653b, eyelet structure; 654, first cell; 655, second cell;
656, anchoring stent; 6561, strut; 6562, cell; 65621, middle part; 65622, end part; 6563, fishbone-shaped cell; 65631, convex tip; 65632, concave tail; 65633, strut; 6564, diamond-shaped cell; 65641, strut; 65642, strut; 65643, strut; 65644, strut;
657, skirt; 658, inner lining film; 659, sealing member;
66, tool; 67, blocking member;
68, locking wire structure; 681, first adjustment wire; 6811, first locking eyelet; 682, locking wire; 6821, first locking wire; 6822, second locking wire; 683, second adjustment wire; 6831, second locking eyelet; 684, third adjustment wire; 685, fourth adjustment wire; 686, fifth adjustment wire; 687, sixth adjustment wire,
7100, leaflet; 7110, leaflet body; 7120, free edge; 7130, fixed edge; 7131, opening area; 7140, commissure tab; 7150, outward-protruding portion; 7151, inner edge; 7152, outer edge; 7170, joint; 7171, first folded portion; 7172, second folded portion; 7173, suture hole; 7174, first suture; 7175, suture hole; 7175a, suture hole; 7175b, suture hole; 7175c, suture hole; 7176, second suture; 7178, third suture; 7180, knot; 7181, knot; 7182, knot;
7200, prosthetic heart valve; 7210, stent; 7220, support bar; 7221, recessed area; 7222, first recessed area; 7223, second recessed area.

### DESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this disclosure.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by those skilled in the art. The terms in the description of the present disclosure are used to describe specific embodiments, and not to limit the present disclosure. The term "and/or" used herein includes one or more of the listed options in any combinations, or the combination of all of the listed options.

In the present disclosure, the terms "first", "second" and the like are used for descriptive purposes only and are not to be understood as indicating or implying the relative importance or the number or order of the technical features referred. Thus, features defined with "first", "second" can explicitly or implicitly include one or more of such features. In the description of the present disclosure, "plurality" means at least two, such as two, three, etc., unless explicitly and specifically defined otherwise.

For ease of understanding of the present disclosure, the "distal end" and "proximal end" mentioned in the following embodiments are commonly used terms in the field of interventional medical devices. When used to indicate direction, the "distal end" refers to the end away from the operator during surgery, and the "proximal end" refers to the end close to the operator during surgery. When used to refer to a structure, the term "end" in this disclosure refers to the endpoint of the structure, or a point or area on that side, or a specific structure connected to that point or area.

In the embodiments of the present disclosure, the axial direction refers, unless otherwise specified, to the direction along the straight line between the proximal and distal ends that is theoretically determined when the catheter assembly and the control handle are completely straightened. Correspondingly, the radial direction perpendicular to the axial direction and the circumferential direction around the axial direction can be determined.

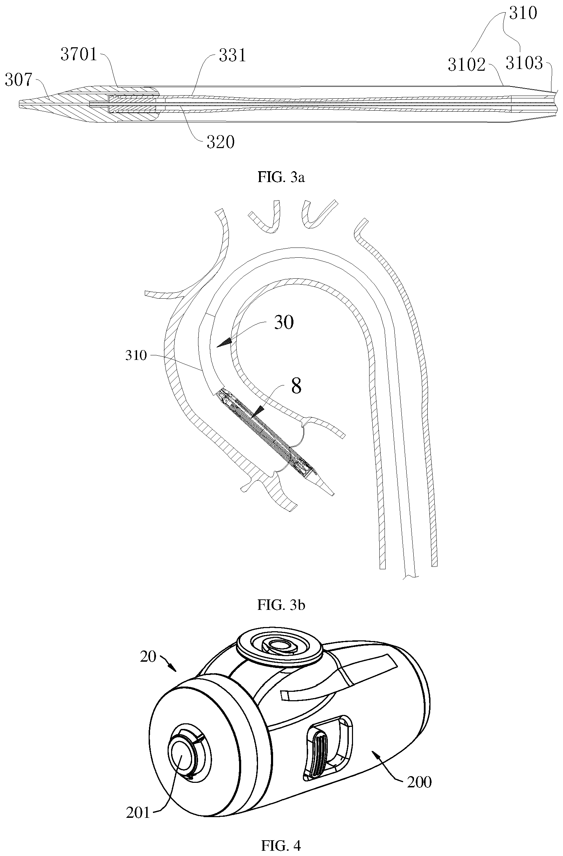

FIG. 3b schematically shows the orientation of the device delivered into the aortic arch during cardiac interventional surgery. In order to adapt to the physiological structure of this area, the distal end of the catheter assembly 30 is obviously bent. The orientation of the distal end of the catheter assembly is usually controlled by bending. After the prosthetic implant is generally positioned, regardless of whether self-expandable or balloon-expandable, the prosthetic implant is exposed by withdrawing the outer sheath, thus facilitating its complete deployment.

The proximal end of the catheter assembly is connected to and controlled by the control handle. The relative movement range of its tubes is limited by the size (length) of the control handle. Therefore, during the withdrawal of the outer sheath, the distal end of the outer sheath generally remains in the aortic arch. For the catheter assembly as a whole, the stiffness varies significantly between the parts exposed outside the outer sheath and those still inside the outer sheath. This results in unstable spatial orientation, potentially requiring readjustment, which brings unnecessary cumbersome operations to the surgery. Especially in the case of deploying the prosthetic implant via the balloon expansion method, it is expected to withdraw the outer sheath quickly to improve efficiency.

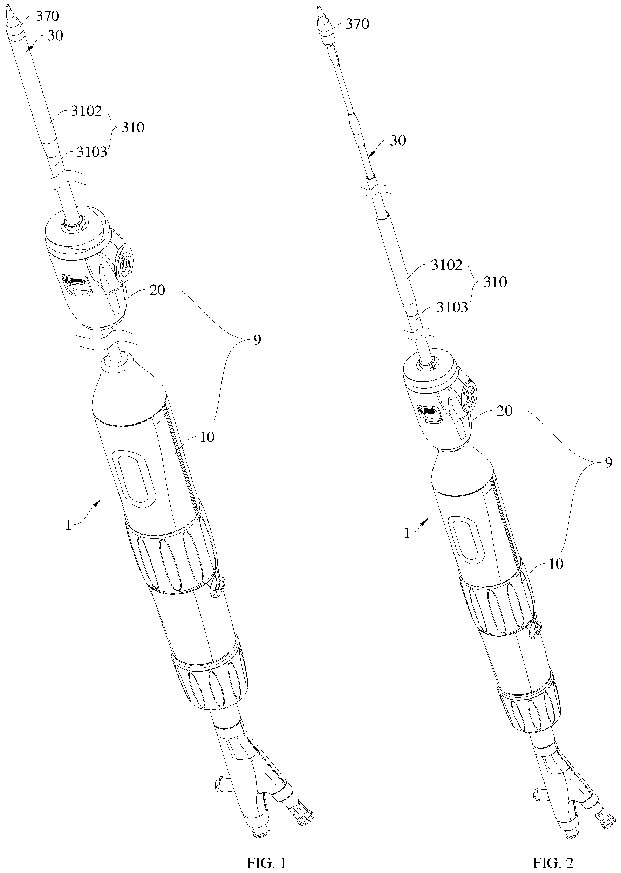

Referring to FIGS. 1 to 3a, the present disclosure provides an interventional delivery system **1,** including a catheter assembly 30 and a control handle 9 connected to the catheter assembly 30. The catheter assembly 30 has a distal end and an opposite proximal end and includes an inner sheath 320 and an outer sheath 310 which are slidably engaged with each other. The outer sheath 310 includes, from distal to proximal, a loading section 3102 and a sheath section 3103. The loading section 3102 is used to enclose the prosthetic implant and maintain the prosthetic implant in a compressed state.

The control handle 9 includes:
a main handle 10, with the proximal end of the inner sheath 320 connected to the main handle 10; and
an auxiliary handle 20, slidably fitted around the inner sheath 320, with the proximal end of the outer sheath 310 connected to the auxiliary handle 20.

In the present disclosure, the auxiliary handle 20 is independently configured for the outer sheath of the catheter assembly. The entire auxiliary handle 20 has a threading channel, which, on the one hand, allows for the insertion and connection of the outer sheath, and on the other hand, facilitates the passage of other tube(s) of the catheter assembly through the auxiliary handle 20 after extending proximally from the outer sheath. For the entire interventional delivery system, the outer sheath is controlled by the auxiliary handle, and the remaining tube(s) are controlled by the main handle (where the terms "main" and "auxiliary" are understood as relative concepts).

By configuring the auxiliary handle and the main handle separately, the flexibility of controlling the outer sheath can be further improved. More importantly, the travel of the outer sheath is no longer limited by the length of the main handle. When retracting the outer sheath, the distal portion of the outer sheath can also be withdrawn from the aortic arch, thereby minimizing its impact on the spatial orientation of the other tube(s).

Depending on the loading and deployment methods of the prosthetic implant, the inner sheath can be directly inserted into the outer sheath (i.e. the two are directly adjacent in the radial direction) or other tube(s) can be arranged between the two radially. The prosthetic implant itself can adopt conventional technologies. Taking a prosthetic heart valve as an example, it generally includes a radially deformable stent and multiple leaflets connected to the stent. The stent has a tubular structure with hollow cells. The interior of the stent is a blood flow channel, and the leaflets cooperate with each other to control the opening and closing of the blood flow channel.

Taking a self-expandable prosthetic implant as an example, the inner sheath has a mounting head near the distal end, and the radially compressed prosthetic implant is connected to the mounting head. The distal end of the outer sheath is an enlarged loading section, which surrounds the prosthetic implant and restricts the prosthetic implant in the compressed state. When the outer sheath is withdrawn proximally, the prosthetic implant is gradually exposed and released.

In the case of a balloon-expandable prosthetic implant, a balloon catheter is further arranged between the inner sheath and the outer sheath. The radially compressed prosthetic implant surrounds the balloon body 331 and is enclosed by the loading section of the outer sheath. When the outer sheath is withdrawn proximally to expose the prosthetic implant, the balloon catheter is inflated by injecting fluid, thereby radially expanding the prosthetic implant to complete the deployment.

A deflectable tube or the like can also be arranged between the inner sheath and the outer sheath as needed for specific operations, which will be further described below. To facilitate the surgery and obtain the position and orientation of the catheter assembly in the human body in real time, radiopaque markers can also be configured on each tube. For example, a first radiopaque ring can be provided at the distal end of the loading section, and a second radiopaque ring 323 and a third radiopaque ring 324 can be provided on the inner sheath corresponding to the balloon body (see FIG. 19a).

Conventional materials can be used for the materials of the tubes. For example, the loading section 3102 of the outer sheath 310 can be made of Pebax, while the other sections such as the sheath section 3103 can be made of PTFE. The deflectable tube can be made of a metallic Hypotube, with slightly varying cutting densities at different parts-for instance, a higher density at the distal end and a lower density at the proximal end-providing greater flexibility at the distal end compared to the proximal end.

The diameter of the loading section 3102 is larger than that of the sheath section 3103 to accommodate prosthetic implants of different specifications. During loading, the prosthetic implant is radially compressed into a tubular shape and is housed within the loading section 3102. In order to facilitate the interventional delivery of the loading section 3102 into the human body, a catheter sheath can be used. The catheter sheath can create a temporary channel in the blood vessel for the catheter assembly 30 to pass through. The wall of the catheter sheath is preferably of an expandable structure to adapt to a slightly larger-diameter loading section 3102. For example, from the perspective of the cross-section of the catheter sheath wall, the catheter sheath wall can have a folded structure. The folded structure can deform and return to its original shape, allowing the temporary channel to expand where the larger-diameter loading section 3102 passes through and then contract back after it passes.

To limit the distal extreme position of the outer sheath 310, the distal end of the inner sheath 320 is provided with a guide head 370. The outer circumference of the guide head 370 defines an annular step 3701, and the distal end surface of the loading section 3102 abuts against the annular step 3701 to achieve axial position limitation. The outer circumference of the guide head 370 is flush with the outer circumference of the loading section 3102, reducing the potential safety hazard of scratching.

Referring to FIGS. 1 to 5, the auxiliary handle 20 includes:
a housing 200 having an axial direction, with a distal end and a proximal end opposite to each other in the axial direction, the housing 200 having an axially through threading channel 201;
a connecting sleeve 210 fixed in the housing 200 and arranged around the threading channel 201, with the distal end of the connecting sleeve 210 configured for insertion of the outer sheath 310;
a positioning member 220 movably installed in the housing 200, the positioning member 220 having a locked position for restricting the threading channel 201 and an unlocked position for releasing the threading channel 201; and
a driving member 230, which is in transmission cooperation with the positioning member 220 to drive the positioning member 220 to switch positions.

The positioning member 220 and the driving member 230 are primarily configured to adjust and lock the relative position of the auxiliary handle 20 and the main handle 10, preventing the outer sheath 310 from slipping and misaligning relative to other tube(s), thereby reducing potential safety risks.

When in use, all tubes except the outer sheath 310 are located within the threading channel 201. When the positioning member 220 restricts the threading channel 201, it can exert force on the other tube(s), thereby locking the relative position of the auxiliary handle 20 and the other tube(s). Similarly, releasing the threading channel 201 means reducing or completely removing the force exerted on the other tube(s), allowing the auxiliary handle 20 to drive the outer sheath 310 to slide relative to the other tube(s). The driving member 230 and the positioning member 220 can be formed in one or separate pieces that are fixed together or in transmission cooperation, so as to drive the positioning member 220 to switch between the locked position and the unlocked position.

In this embodiment, the movement direction of the positioning member and the driving member relative to the auxiliary handle is not strictly limited. The positioning member has at least a movement component in the radial direction of the threading channel to change the force exerted on other tube(s) or the radial relative position.

The force exerted by the positioning member on other tube(s) can be a friction force generated by radial abutment. It can also be achieved through structures that cooperate with each other to generate an axial limiting force, such as latching teeth or axial blocking elements. The driving member can move in ways like axial sliding, radial pressing, or circumferential rotation.

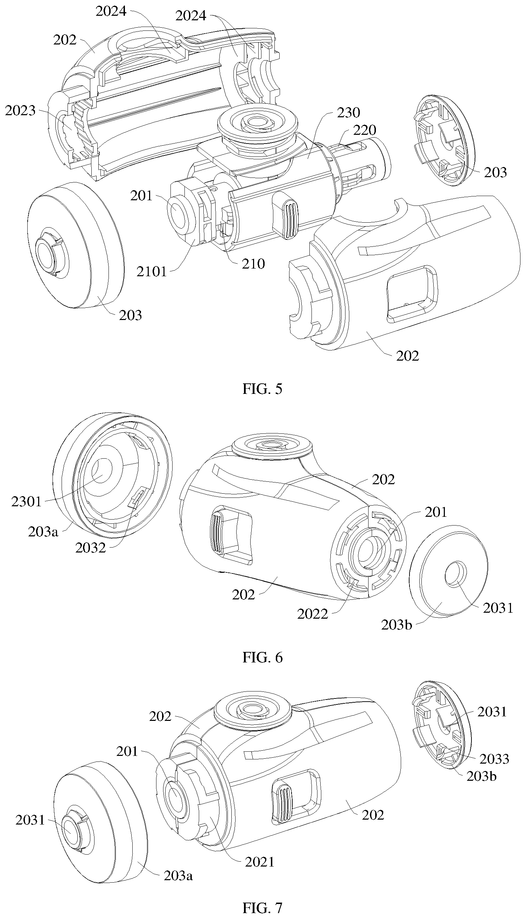

The housing 200 provides a space for installing and accommodating other components, and is generally held directly during operation. For ease of assembly, referring to FIGS. 4 and 5, in one embodiment, the housing 200 is formed by separate pieces in a snap-fit structure, with an engagement and positioning structure provided between the inner wall of the housing 200 and the outer circumference of the connecting sleeve 210.

The snap-fit structure mentioned above may consist of separate pieces snapped with each other in the radial direction, or further in combination with an axially segmented design. **In** this embodiment, the housing 200 includes two half-shells 202 snapped with each other in the radial direction of the connecting sleeve 210, and end caps 203 respectively located at the axial ends of the two half-shells 202. The end caps 203 are fixed to the two half-shells 202 via either a snap-fit or a threaded connection.

Referring to FIGS. 6 and 7, the engagement parts of the two half-shells 202 abut against or engage with each other. The end caps 203 at both ends hoop the two half-shells 202 together and fix them to each other. In addition, they can be fixed by bonding, fasteners, snaps, etc. Each end cap has a through hole 2031 to avoid interference with the threading channel 201. The inner circumference of the first end cap 203a has latching teeth 2032, and the outer circumference of the half-shells 202 defines screwing-in latching grooves 2021 that engage with the latching teeth 2032. The second end cap 203b at the other end is provided with elastic snaps 2033 on one axial end face, and the corresponding axial end face of the half-shells 202 defines plug-in latching grooves 2022 that engage with the elastic snaps 2033.

The engagement and positioning structure between the housing 200 and the connecting sleeve 210 includes a positioning groove 2023 defined on the inner circumference of the half-shells, and a positioning shoulder 2101 fixed to the outer circumference of the connecting sleeve. After the two half-shells 202 are snapped together, the positioning shoulder 2101 is inserted into the positioning groove 2023 to achieve axial positioning, while also limiting the displacement of the connecting sleeve in the radial direction.

In order to ensure the positioning effect, a plurality of ribs 2024 can be provided on the inner walls of the half-shells 202. The ribs 2024 surround and support the outer circumference of the connecting sleeve 210 to assist in positioning. In the axial direction of the connecting sleeve, the positioning shoulder 2101 and the ribs 2024 are respectively located at both ends of the connecting sleeve 210.

There is a radial gap between the outer sheath 310 and the internal tube(s). Before surgery, it is necessary to expel the air within this radial gap to reduce safety risks. For example, physiological saline can be injected into the radial gap from the proximal end of the outer sheath 310, forcing the air out through the distal end. In conventional technologies, a pipeline connected to the radial gap is configured, with an additional control valve installed on the pipeline. The present disclosure provides an improved approach in one embodiment.

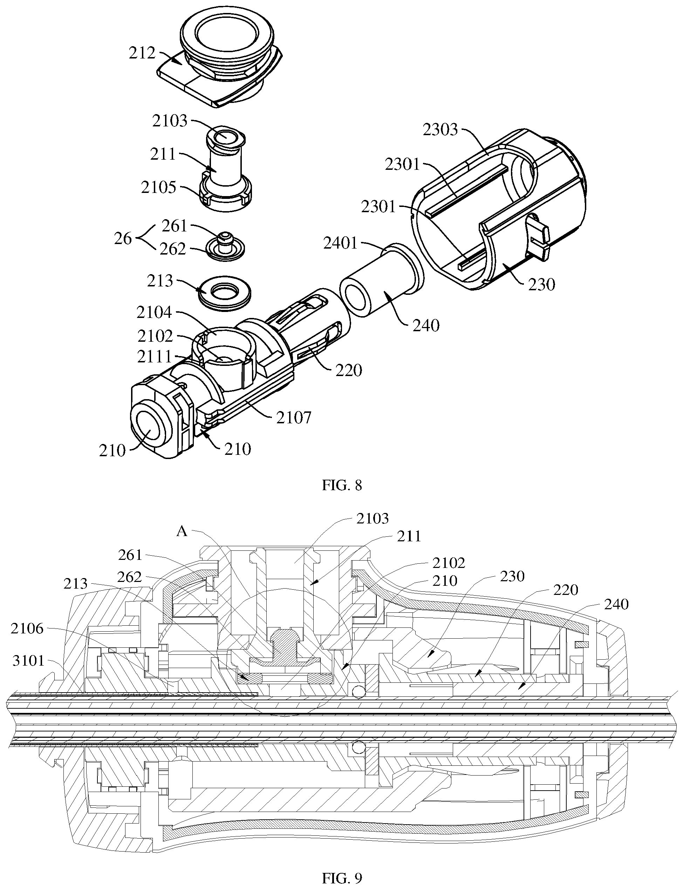

Referring to FIGS. 8 and 9, the side wall of the connecting sleeve 210 defines a first vent hole 2102 communicating with the threading channel 201. A first pipe joint 211 is fixedly connected to the first vent hole 2102. The first vent hole 2102 and the first pipe joint 211 define a first vent channel 2103, and a one-way valve core 26 is provided in the first vent channel 2103.

From the distal end to the proximal end, the arrangement is as follows:
a sealing engagement between the inner wall of the connecting sleeve and the outer sheath 310;
the first vent hole 2102; and
a sealing engagement between the inner wall of the connecting sleeve and the other tube(s) inside the outer sheath 310, approximately at the area where the positioning member 220 restricts the threading channel 201. When the positioning member 220 locks the internal tube(s), it forms a sealing fit with them.

The arrangement mentioned above can ensure that the physiological saline injected from the first vent hole 2102 can only enter the radial gap between the outer sheath 310 and the internal tube(s), flowing distally to expel air.

In this embodiment, an external control valve is omitted. Instead, a one-way valve core that can float under the action of fluid pressure difference is installed inside the first vent channel. When physiological saline is injected, the one-way valve core is pushed open to allow flow through the first vent channel. In the event of backflow, the reverse pressure pushes the one-way valve core to close the first vent channel, preventing fluid leakage. This self-adaptive one-way valve core enhances the overall integration of the system, eliminating the need for cumbersome control valve operations.

In order to connect the first pipe joint 211, a first annular seat 2104 is arranged around the first vent hole 2102. The first pipe joint 211 is inserted and mated with the first annular seat 2104, with an anti-rotation structure provided between them to prevent unnecessary rotation.

One end of the first pipe joint 211 may have a threaded structure to facilitate quick disassembly and assembly with the external pipeline, and the other end is inserted into the first annular seat 2104. A sealing ring 213 arranged around the first vent hole 2102 may be provided in the first annular seat 2104. The end face of the first pipe joint 211 inserted into the first annular seat 2104 abuts against the sealing ring 213 for sealing. The sealing ring 213 is elastic and can ensure the sealing effect.

In order to ensure the strength of the connection with the external pipeline, a first protective sleeve 212 surrounding the first pipe joint 211 is embedded in the housing 200. A pipeline insertion gap is formed between the outer wall of the first pipe joint 211 and the inner wall of the first protective sleeve 212.

The anti-rotation structure can prevent the first pipe joint 211 from rotating unnecessarily when assembling or disassembling the external pipeline. The anti-rotation structure includes:
slots 2111 extending in the insertion direction of the first pipe joint 211 and defined on a side wall of one of the first pipe joint 211 and the first annular seat 2104; and
positioning ribs 2105 engaging with the slots 2111 and provided on the side wall of the other of the first pipe joint 211 and the first annular seat 2104.

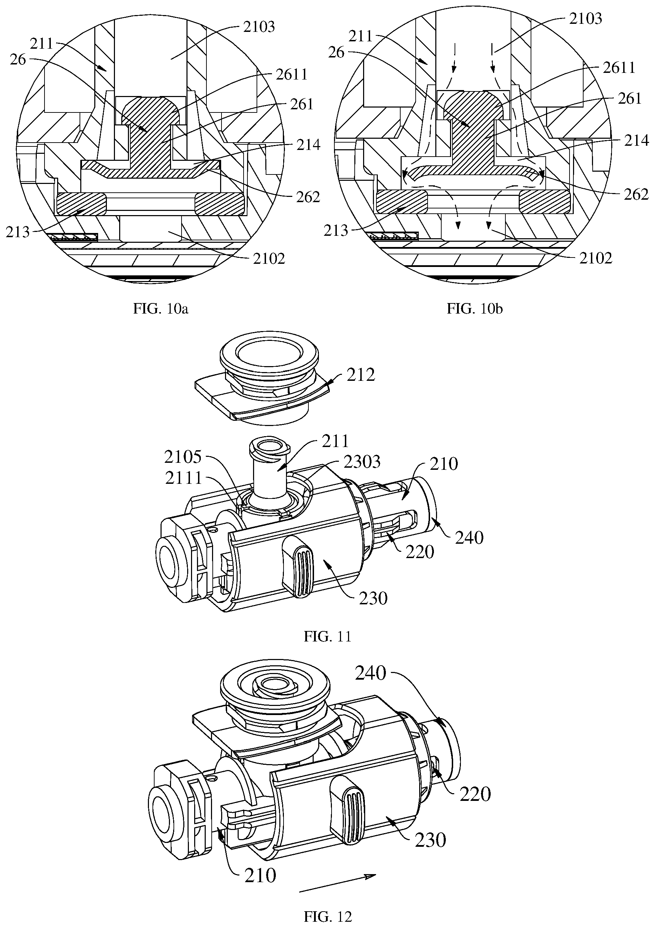

In FIG. 8 and FIG. 11, three slots 2111 are shown on the inner wall of the first annular seat, and three positioning ribs 2105 are provided at corresponding positions on the outer wall of the first pipe joint.

The one-way valve core 26 has a sealed state and an open state, and the one-way valve core 26 specifically includes:
a limiting rod 261 which is slidably mounted in the inner cavity of the first pipe joint 211, with a fluid gap between the outer circumference of the limiting rod and the inner wall of the first pipe joint 211 (the fluid gap also forms part of the first vent channel 2103); and
a sealing sheet 262 connected to the limiting rod 261, which closes the fluid gap in the sealed state, and opens the fluid gap 214 in the open state.

The one-way valve core is overall designed to be floatable. The limiting rod 261 is movably installed and its position is limited by the first pipe joint 211, which can limit the stroke of the sealing sheet 262. Especially in the open state, if the stroke of the sealing sheet 262 is too large, it may directly block the first vent hole 2102. As an example, the inner wall of the first pipe joint 211 is provided with a retaining ring 2112 surrounding the outer periphery of the limiting rod, and the fluid gap is located between the outer periphery of the retaining ring 2112 and the inner wall of the first pipe joint 211.

One end of the limiting rod 261 away from the sealing sheet 262 passes through the retaining ring 2112 and has a head 2611. In the open state, the head 2611 abuts against the retaining ring 2112, and the sealing sheet 262 moves away from the retaining ring 2112 and the fluid gap 214 is opened. In the sealed state, the head moves away from the retaining ring 2112, and the sealing sheet 262 abuts against the retaining ring 2112 and closes the fluid gap 214.

In another embodiment, referring to FIG. 9 to FIG. 10b, the one-way valve core as a whole can also be fixedly arranged. The limiting rod 261 is fixedly installed, and the fluid gap is opened or closed only by the deformation of the edge of the sealing sheet 262. For example, the inner wall of the first pipe joint 211 is provided with a retaining ring 2112 surrounding the outer periphery of the limiting rod, and the fluid gap is located between the outer periphery of the retaining ring 2112 and the inner wall of the first pipe joint 211.

One end of the limiting rod 261 away from the sealing sheet 262 passes through the retaining ring 2112 and has a head 2611. The head 2611 and the sealing sheet are fixed on two opposite sides of the retaining ring 2112. The edge of the sealing sheet 262 can elastically deform to open or close the fluid gap 214.

In the open state, the edge of the sealing sheet 262 moves away from the retaining ring 2112 and the fluid gap 214 is opened (as shown in FIG. 10b, with the fluid flow path indicated by dashed lines).

In the sealed state (FIG. 10a), the edge of the sealing sheet 262 abuts against the retaining ring 2112 and closes the fluid gap 214.

The following embodiment provides the specific structure of the positioning member. For example, referring to FIG. 8 to FIG. 13, the positioning member 220 consists of elastic claws. One end of the elastic claw is fixed to the connecting sleeve 210, and the other end is a free end 2201 that can swing in the radial direction of the connecting sleeve.

The driving member 230 is linked with the free ends 2201 of the elastic claws 221, enabling the free ends 2201 to move inward in the radial direction of the connecting sleeve (i.e., to restrict the threading channel 201 and clamp the internal tube(s)), or releasing the elastic claw 221. The elastic claws 221 have a reset tendency (i.e., moving away from the threading channel and reducing the action on the internal tube(s)), allowing the auxiliary handle 20 to slide relative to the internal tube(s) and adjust the relative position of the distal end of the outer sheath.

The fixed end 2202 of the elastic claw 221 can be fixed to the connecting sleeve 210, or the elastic claw and the connecting sleeve 210 can be formed in one piece.

The connecting sleeve 210 is generally tubular, with U-shaped hollow areas 2204 on its wall. The elastic claw 221 is located within the corresponding U-shaped hollow area. The elastic claw 221 is generally strip-shaped. Relatively, the fixed end 2202 of the elastic claw is at the distal end, and the free end 2201 is at the proximal end. For uniform clamping force, multiple elastic claws can be configured. For instance, there can be 2 to 6 elastic claws (e.g., 4), distributed in the circumferential direction of the connecting sleeve 210.

In order to synchronously drive all elastic claws and simplify the structure, the driving member 230 is cylindrical and slidably fitted over the outer periphery of the connecting sleeve 210. The inner wall of the driving member 230 acts on the elastic claws.

The sliding direction of the driving member 230 corresponds to the length direction of the connecting sleeve 210. When the driving member 230 moves to different positions, the radial force applied to the elastic claws changes, causing the positioning member 220 to switch between the locked position and the unlocked position. In order to enable the elastic claws to obtain a relatively uniform change trend of the radial force, the elastic claws and the driving member 230 can be matched through inclined surfaces. For example, in the radial direction of the connecting sleeve 210, the outer side of the elastic claw 221 is provided with a guide slope 2203, and the inner wall of the driving member 230 acts on the guide slope 2203.

In the radial direction of the connecting sleeve 210, the height of the guide slope 2203 gradually increases from the distal end to the proximal end. Without considering the change of the inner wall of the driving member, when the driving member 230 moves proximally, it causes the free end 2201 of the elastic claw 221 to further move inward, increasing the force exerted on the internal tube(s) until the internal tube(s) are locked. For cushioning, protection, and achieving better clamping and sealing effects, an elastic component can be provided between the elastic claws 221 and the internal tube(s), which can also provide a damping effect when sliding the auxiliary handle 20, improving the operation experience. For example, an elastic liner 240 can be provided inside the connecting sleeve 210, and the elastic claws 221 act on the outer wall of the elastic liner, compressing it to restrict the threading channel 201.

When inserting the internal tube(s), the elastic claws 221 compress the elastic liner 240 inwardly, clamping the internal tube(s) through the elastic liner 240. In order to avoid unnecessary loosening, the elastic liner 240 can be inserted into the connecting sleeve in an interference fit manner. For further axial positioning, the distal end of the elastic liner 240 extends out of the connecting sleeve 210, and the extended portion is provided with a radially outwardly protruding positioning step 2401. In the axial direction, the positioning step 2401 is axially positioned and matched with the end face of the connecting sleeve 210 and/or the inner wall of the housing 200.

In order to guide the movement of the driving member 230, a guiding structure with mutually-matching parts is provided between the inner wall of the driving member 230 and the outer wall of the connecting sleeve 210. The guiding structure includes:
slide grooves 2107 defined on one of the inner wall of the driving member 230 and the outer wall of the connecting sleeve 210; and
guide ribs 2301 engaging with the slide grooves 2107 and provided on the other of the inner wall of the driving member 230 and the outer wall of the connecting sleeve 210.

In FIG. 8, three slide grooves 2107 are defined on the outer wall of the connecting sleeve. The guide ribs 2301 are provided on the inner wall of the driving member 230, with positions and quantities corresponding to the slide grooves.

For ease of operation, referring to FIG. 14 and FIG. 15a, in one embodiment, an operation button 250 is provided on the outer wall of the driving member 230, and a first clearance opening 2302 corresponding to the position of the operation button 250 is opened on the housing 200.

The operation button 250 is exposed through the first clearance opening 2302, and may further extend outward from the first clearance opening 2302 in the radial direction of the connecting sleeve 210. The length of the first clearance opening 2302 is configured according to the sliding range of the driving member 230.

To ensure stable sliding of the driving member 230 and maintain necessary structural strength, it should have sufficient axial length. Considering the potential interference with the first vent channel, the side wall of the driving member 230 defines a second clearance opening 2303. The side wall of the connecting sleeve 210 defines a first vent hole that corresponds to the threading channel 201. In the axial direction of the connecting sleeve 210, the position of the second clearance opening 2303 corresponds to the position of the first vent hole.

The one-way valve core 26 and the first pipe joint 211 among others at the vent hole are all located at the second clearance opening 2303. For ease of assembly, the distal end of the second clearance opening 2303 is an open structure.

In combination with the auxiliary handle 20 of the previous embodiments, referring to FIG. 15b, an embodiment of the present disclosure further provides an intervention control assembly, including:
an outer sheath 310, with the prosthetic implant in the loaded state (invisible in the loaded state) being in the inner lumen of the outer sheath 310; and
a control handle (i.e., the auxiliary handle 20) that directly drives the outer sheath 310, one end of the outer sheath 310 being fixedly connected to the connecting sleeve 210.

In terms of the specific connection method, the proximal end of the outer sheath 310 is an insertion section 3101 (located at the proximal end of the sheath section 3103, or can be regarded as a part of the sheath section 3103) and extends from the distal end of the connecting sleeve 210 and is fixed inside the connecting sleeve (invisible in the assembled state).

Referring to FIG. 9, in order to ensure the connection strength, the side wall of the connecting sleeve 210 defines a reinforcement hole corresponding to the insertion section 3101. The insertion section is fixed via the reinforcement hole 2106 using at least one of dispensing glue, welding, or anchoring.

Regarding the deployment of the prosthetic implant, the following takes the balloon expansion method as an example. This method can also synergize with the separation of the main and auxiliary handles in the present disclosure to achieve better performance. Referring to FIGS. 16a to 17a, in one embodiment, the catheter assembly 30 further includes a balloon catheter 330, and the balloon catheter 330 includes:
a balloon body 331 provided at the distal end of the inner sheath 340 and configured to switch between an inflated state and a folded state under the action of fluid, the prosthetic implant 8 in the loaded state surrounding the balloon body 331; and
a catheter body 332 surrounding the inner sheath 340 or arranged in parallel with the inner sheath 320, the distal end of the catheter body 332 being connected to the balloon body 331, the proximal end of the catheter body 332 being connected to the main handle 10, and the interior of the catheter body 332 serving as a fluid channel.

During operation, the prosthetic implant 8 and the balloon body 331 are enclosed in the outer sheath 310. In some cases, the outer sheath may be withdrawn proximally before reaching the lesion, while the balloon body 331 is still in the folded state, resulting in a protruding step 86 between the outer circumference of the balloon body 331 and the distal end of the prosthetic implant 8. This poses a safety risk of tissue injury during subsequent delivery. This embodiment provides a solution whereby fluid can be injected to inflate the distal portion of the balloon body 331 after it is exposed from the outer sheath 310. Since the proximal portion of the balloon body 331 is still enclosed by the outer sheath, it remains in a folded state. When injecting fluid, if a large part of the prosthetic implant 8 is exposed from the outer sheath 310, the fluid pressure can be adjusted to inflate only the distal portion of the balloon body 331 exposed beyond the prosthetic implant 8, while the other portions remain folded under the constraint of the prosthetic implant 8.

Once the distal portion of the balloon body 331 is inflated, it can eliminate or compensate for the protruding step 86, creating a smooth transition or reducing the height difference between the outer circumference of the balloon body 331 and the end face of the prosthetic implant 8, thereby avoiding potential safety hazards.

The proximal end of the catheter body can be connected to an infusion device to inject fluid (such as saline) into the balloon body 331 to inflate the balloon body 331. In the prior art, during the transition of the balloon body to the inflated state, the outward inflation tendencies vary across different axial sections.

For example, the fluid first enters the proximal portion of the balloon body and gradually inflates it, while the prosthetic implant is still in a compressed state, preventing the fluid from smoothly reaching the distal portion of the balloon body. As a result, the deployment of the distal portion of the prosthetic device is delayed relative to the proximal portion. This may cause the prosthetic device to slide distally relative to the balloon body or even deviate from the balloon body entirely, thereby compromising the deployment performance.

The present disclosure provides an improved approach to this problem. Referring to FIG. 17a, in one embodiment, a fluid guide member 350 is provided around the inner sheath inside the balloon body 331. The fluid guide member 350 defines a diversion channel 354 that connects the distal portion and the proximal portion inside the balloon body.

Referring to FIGS. 17a to 19b, the axial length of the balloon body 331 is slightly larger than that of the prosthetic implant 8 in the loaded state. Under the action of the fluid guide member 350, the fluid can quickly reach the distal portion of the balloon body 331, so the distal portion and the proximal portion of the balloon body 331 are basically inflated synchronously (see the dashed lines in FIG. 19a for the flow path of the fluid) Correspondingly, the two ends of the prosthetic implant 8 are expanded before the middle part. In this process, the prosthetic implant 8 forms a waist-like structure 81 as a whole, which can restrict the axial sliding of the prosthetic implant 8.

The fluid guide member 350 is tubular, and the position of the diversion channel 354 relative to the fluid guide member 350 is at least one of the following:
on the outer wall of the fluid guide member 350;
inside the fluid guide member 350; and
in the side wall interlayer or hollow areas 353 of the fluid guide member 350. The specific structure of the hollow area 353 is described below.

The specific structure of the diversion channel is further described in the following embodiments.

The diversion channel 354 consists of diversion grooves 351 opened on the outer circumferential wall of the fluid guide member.

The radially outwardly protruding groove walls between adjacent diversion grooves 351 can support the inner wall of the balloon body in the folded state, so that the diversion grooves 351 can connect the distal side and the proximal side of the balloon body even in the folded state. To ensure the diversion effect, the fluid guide member 350 should have a sufficient length. For example, the distal side and the proximal side of the fluid guide member 350 are respectively adjacent to the distal side and the proximal side of the balloon body 331.

In one embodiment, there are multiple diversion grooves 351, which are distributed in the circumferential direction of the fluid guide member 350. Each diversion groove 351 extends in the axial direction of the fluid guide member 350 (as shown in FIGS. 20a and 20b) or in a helical pattern (as shown in FIGS. 21a to 22b).

For the helical arrangement, the angle should not be too large to avoid affecting the diversion effect. For example, in the circumferential direction of the fluid guide member 350, the wrap angle of a single diversion groove 351 ranges from 0 to 180 degrees. In FIG. 21a, for instance, the wrap angle (the offset angle between the two ends of the same flow groove 351) of a single diversion groove 351 is about 60 degrees.

Referring to FIGS. 20a to 21b, in one embodiment, a communication groove 352 is defined between two adjacent diversion grooves 351. There are multiple communication grooves 352 arranged at intervals, and those at the same axial position form an annular groove.

The plurality of diversion grooves 351 and communication grooves 352 intersect with each other to form a diversion network.

In another embodiment, a fluid guide member 350 is arranged around the inner sheath 320. The fluid guide member 350 is tubular and is located in the balloon body 331. The tube wall of the fluid guide member 350 has hollow areas 353. These hollow areas 353 extend continuously or intermittently from the distal end of the fluid guide member 350 to the proximal end of the fluid guide member 350.

The hollow area 353 can take various forms, such as those shown in FIGS. 23a to 24b.

In another embodiment, there is a radial gap between the fluid guide member 350 and the inner sheath 320, and this radial gap serves as a diversion channel 354 (as shown in FIG. 17b). In other embodiments, the above-mentioned diversion methods can be combined.

In a complex in vivo environment, for the distal end of the catheter assembly 30 to smoothly advance for intervention or maintain a specific orientation, active bending adjustment is sometimes required. For example, the catheter assembly 30 further includes a deflectable tube 340 and a traction member 341 for driving the deflectable tube 340. The deflectable tube 340 is arranged around the inner sheath 320. The distal ends of the deflectable tube 340 and the traction member 341 are fixedly connected, and the proximal ends of both are connected to the main handle 10 and are in relative sliding fit.

When combined with the balloon catheter, the deflectable tube 340 and the traction member 341 are both located on the outer periphery of the catheter body 332, and the distal end of the deflectable tube 340 avoids the balloon body 331. Under the action of the traction member, the distal end of the deflectable tube 340 can be bent, thereby driving the surrounding tubes to bend together. The distal end of the deflectable tube and other tubes (except the traction member) are not strictly required to be fixed to each other.

Regarding the configuration of the traction member 341, in different embodiments, it can be:
a traction tube, which is located inside or outside the deflectable tube 340 (as shown in FIG. 26a and FIG. 26b); or
a traction wire, which is located inside (as shown in FIG. 27a and FIG. 27b), outside or embedded within the wall of the deflectable tube 340.

The wall of the deflectable tube 340 has an interlayer structure, within which a lining tube is fixed (not visible in the assembled state). The traction member 341 is movably disposed through the lining tube.

Referring to FIGS. 28 to 34, the main handle 10 includes:
a support body 100 having an axial direction, with the proximal end of the deflectable tube 340 fixedly connected to the support body 100, the inner sheath 340 and the catheter body 332 of the balloon catheter both extending and connecting to the proximal side of the support body 100;
a first sliding seat 110 which is axially slidable relative to the support body 100, with the proximal end of the traction member 341 fixed to the first sliding seat 110;
a first driving sleeve 111 rotatably arranged around the support body 100 and around the outer periphery of the first sliding seat 110, the first driving sleeve 111 and the first sliding seat 110 being engaged in a threaded transmission fit;
a housing 120 fixedly arranged around the support body 100, with at least a portion forming an indicating section located outside the first driving sleeve; and
an identification member 122 which is axially slidably installed on the indicating section and is located on the outer periphery of the first driving sleeve 111, the first driving sleeve 111 and the identification member 122 being engaged in a threaded transmission fit.

When the first driving sleeve 111 rotates, it drives the first sliding seat 110 to slide axially, thereby pulling the proximal end of the traction wire (i.e., the traction member). Since the proximal end of the deflectable tube 340 is fixedly connected to the support body 100, the traction wire will pull the distal end of the deflectable tube 340, causing it to change direction. As it is not convenient to observe the bending angle in vivo, the identification member 122 can be used to estimate the bending effect based on the displacement of the first sliding seat 110.

Although the identification member 122 could be directly fixed to the first sliding seat 110, this would require either bypassing the first driving sleeve 111 or adding a viewing window on it for observation, which will cause space waste or weaken the structural strength. In this embodiment, the first driving sleeve 111 is configured to synchronously drive the identification member 122 and the first sliding seat 110, ensuring accurate indication while overcoming these defects. Specifically, the first driving sleeve 111 has internal threads and external threads, the first sliding seat 110 has external teeth that match the internal threads, and the identification member 122 has internal teeth that match the external threads.

The identification member 122 and the first sliding seat 110 move synchronously under the action of the first driving sleeve 111. In order to observe the identification member 122, a viewing window is provided on the sidewall of the indicating section at a corresponding position.

In order to guide the identification member 122, a guiding structure can be provided on the inner edge of the view window or the support body 100 to limit the movement direction of the identification member 122. For example, two slide grooves 1211 are provided on the side wall of the indicating section, and the identification member 122 is located outside the side wall and spans between the two slide grooves 1211. The end of the identification member 122 extends inward through the slide groove 1211, forming inner teeth 1221 that engage with the external threads of the first driving sleeve 111. Referring to FIG. 28, in one embodiment, a transparent cover plate 123 can be provided on the outer side of the identification member 122, fixedly embedded in the outer wall of the indicating section, and a frame 121 can be provided for easy installation.

Once the first driving sleeve reaches the desired position, it needs to be locked in place to avoid interference with other operations. In one embodiment, a locking ring that is axially slidable is provided around the outer periphery of the first driving sleeve, and the first driving sleeve has a locked state in which it is engaged with the locking ring and a free state in which it is disengaged from the locking ring. In the locked state, the locking ring limits the rotation of the first driving sleeve.

In the locked state, the first driving sleeve and the locking ring are meshed together via locking teeth.

To guide the movement of the first sliding seat 110, an installation groove 1001 extending axially can be provided in the support body 100, and a guide cylinder 112 is fixed in the installation groove 1001. The first sliding seat 110 is slidably arranged around the guide cylinder 112, and a guiding structure is provided between the first sliding seat 110 and the outer wall of the guide cylinder 112 to facilitate the axial movement.

For example, as shown in FIGS. 31 and 34, the outer wall of the guide cylinder 112 has an axially extending guide strip 1121, and the inner side of the first sliding seat 110 has a slide groove 1101 that fits with the guide strip 1121. The proximal end of the deflectable tube 340 is inserted into and fixed to the guide cylinder 112.

Referring to FIGS. 35 to 37, for the convenience of exhausting air, the side wall of the guide cylinder 112 is provided with a second vent hole 1122, and the radial gap between the deflectable tube 340 and the catheter body 332 is in communication with the second vent hole 1122. The proximal end of the catheter body 332 passes through the proximal end of the deflectable tube 340 and further extends through the guide cylinder 112;

A second pipe joint 113 is fixedly connected at the second vent hole 1122. The second vent hole 1122 and the second pipe joint 113 define a second vent channel 1131. A one-way valve core 114 is disposed in the second vent channel 1131.

From the distal end to the proximal end, the arrangement is as follows:
a sealing engagement between the proximal end of the deflectable tube 340 and the guide cylinder 112;
the second vent hole 1122; and
a sealing engagement between the inner wall of the guide cylinder and the catheter body 332;

The arrangement mentioned above can ensure that the physiological saline injected from the second vent hole 1122 can only enter the radial gap between the deflectable tube 340 and the catheter body 332, flowing distally to expel air.

In this embodiment, an external control valve is omitted, and the working principle of the one-way valve core 114 is the same as that of the one-way valve core 26 described in the previous embodiment, which will not be further elaborated here.

A second annular seat 1123 is disposed around the second vent hole 1122. The second pipe joint 113 is inserted and mated with the second annular seat 1123, with an anti-rotation structure provided between them to prevent unnecessary rotation.

One end of the second pipe joint 113 may have a threaded structure to facilitate quick disassembly and assembly with the external pipeline, and the other end is inserted into the second annular seat 1123. A sealing ring 115 arranged around the second vent hole 1122 may be provided in the second annular seat 1123. The end face of the second pipe joint 113 inserted into the second annular seat 1123 abuts against the sealing ring 115 for sealing. The sealing ring 115 is elastic and can ensure the sealing effect.

The anti-rotation structure can prevent the second pipe joint 113 from rotating unnecessarily when assembling or disassembling the external pipeline. The anti-rotation structure includes:
slots 1124 extending in the insertion direction of the second pipe joint 113, and defined on a side wall of one of the second pipe joint 113 and the second annular seat 1123; and
positioning ribs 1132 engaging with the slots 1124 and provided on the side wall of the other of the second pipe joint 113 and the second annular seat 1123.

In FIG. 35 and FIG. 36, three slots 1124 are schematically shown on the inner wall of the second annular seat 1123, and three positioning ribs 1132 are provided at corresponding positions on the outer wall of the second pipe joint.

In order to ensure the strength of the connection with the external pipeline, a second protective sleeve 116 is provided around the outer periphery of the second pipe joint 113. A pipeline insertion gap is formed between the outer wall of the second pipe joint 113 and the inner wall of the second protective sleeve 116. The second protective sleeve 116 includes two parts (the first part 116a and the second part 116b as shown in FIGS. 35 and 36 respectively), which are fixedly arranged on the support body 100 and the guide cylinder 112 respectively.

In order to inflate the balloon body 331, an interface connected to the catheter body 332 is provided at the proximal end of the main handle 10. Furthermore, to enable fine axial adjustment of the balloon body 331 in the in vivo environment, referring to FIG. 28 and FIG. 38 to FIG. 41, in one embodiment, the main handle 10 further includes:
a second sliding seat 130 which is axially slidable relative to the support body 100;
a second driving sleeve 131 rotatably arranged around the support body 100 and around the outer periphery of the second sliding seat 130, the second driving sleeve 131 and the second sliding seat 130 being engaged in a threaded transmission fit; and
a multi-way connector 140 connected to the proximal end of the second sliding seat 130. The proximal ends of the balloon catheter 330 and the inner sheath 320 are both connected to the multi-way connector 140. The interior of the inner sheath 320 is a through guidewire channel 321. The multi-way connector 140 has at least two interfaces respectively communicating with the guidewire channel 321 and the fluid channel 322: an interface 141 communicating with the guidewire channel 321 and an interface 142 communicating with the fluid channel 322 as shown in FIG. 39.

When the second driving sleeve 131 rotates, it drives the second sliding seat 130 to slide axially relative to the support body 100 through threaded transmission. Since the multi-way connector 140 is connected to the proximal end of the second sliding seat 130, the balloon catheter 330 and the inner sheath 320 also move. As a further improvement, the multi-way connector 140 and the proximal end of the second sliding seat 130 are rotatably engaged with each other and axially limited relative to each other.

The axial limitation between the multi-way connector 140 and the second sliding seat 130 ensures that the axial movement of the second sliding seat 130 is synchronously transmitted to the balloon body 331 and the prosthetic implant 8. The rotational engagement between the multi-way connector 140 and the second sliding seat 130 allows the balloon body 331 and the prosthetic implant 8 to rotate relative to the support body 100, realizing the precise adjustment of the spatial orientation of the prosthetic implant 8. Specifically, the second sliding seat 130 is generally cylindrical, and partially housed within the second driving sleeve 131. The outer wall of the second sliding seat 130 is in threaded fit with the inner wall of the second driving sleeve 131. The support body 100 is provided with a guide groove 1002 for guiding the second sliding seat 130 to move axially.

Referring to FIGS. 40 and 41, the distal end of the multi-way connector 140 and the proximal end of the second sliding seat 130 are inserted into and rotatably engaged with each other, with an axial limiting structure between them. The axial limiting structure includes:
a latching tooth 1301 which is fixed to one of the two and protrudes radially toward the other; and
a latching groove 1401 which is provided on the other of the two and receives the latching tooth 1301.

The multi-way connector 140 can be configured with multiple interfaces, corresponding to the guidewire channel 321, the fluid channel 322. As a further improvement, a locking wire channel 143 is provided. A locking wire 360 which is directly driven at the proximal end can be threaded through the locking wire channel 143. The locking wire 360 extends to the distal end and is located outside the balloon body 331. Cooperating with the wire loop structures on the balloon body 331, the locking wire 360 can further lock the axial position of the prosthetic implant 8.

For example, in FIG. 42, the distal end of the catheter assembly is provided with a first pull ring 381 and a second pull ring 382, the first pull ring 381 passes through a first eyelet 811 at the distal end of the prosthetic implant 8, and the second pull ring passes through a second eyelet 812 at the proximal end of the prosthetic implant 8.

The distal end of the locking wire 360 passes through the second pull ring 382 and the first pull ring 381 in sequence and then extends into the guide head 370 (with a locking hole for inserting the locking wire) at the distal end of the inner sheath. In this way, the axial position of the prosthetic implant 8 can be further locked. The locking wire can be withdrawn proximally as needed to release the restraint on the prosthetic implant 8.

In the following embodiments, the prosthetic implant takes the prosthetic heart valve as an example. Referring to FIGS. 43 and 44, a prosthetic heart valve is provided in one embodiment, including a stent 800 and leaflets 83. The stent 800 has a mesh structure, the two ends of the stent are respectively an inflow side 834 and an outflow side 833, and the interior of the stent is a blood flow channel 85. There are multiple leaflets 83, which are located in the blood flow channel and cooperate with each other to open or close the blood flow channel 85. The edge of the leaflet 83 includes a fixed edge 831 fixed to the stent 800, and a free edge 832 that cooperates with other leaflets 83 to control the blood flow channel 85.

To prevent paravalvular leakage, an anti-paravalvular leakage component is connected to the stent 800. The anti-paravalvular leakage component is generally formed in one piece, including a base 84 on the inner side of the stent and anti-paravalvular leakage elements 841 fixed to the outer side of the base. The anti-paravalvular leakage elements 841 are spaced apart and block-shaped and aligned with the hollow areas of the stent's cells.

In the expanded state after the stent 800 is released, the anti-paravalvular leakage element 841 extends outward from the corresponding cell in the radial direction of the stent, or is higher than the outer periphery of the stent in the radial direction.

The base 84 and the anti-paravalvular leakage elements 841 may be formed by infiltration bonding. The base 84 may be made of PET, and the anti-paravalvular leakage element 841 may be made of porous material, such as PU, with pores either inherent to the material structure or created via external processing.

Referring to FIG. 43, in one embodiment, depending on the different axial positions of the cells, the anti-paravalvular leakage elements 841 include:
a first row of anti-paravalvular leakage elements 8411, fully covering the respective cells;
a second row of anti-paravalvular leakage elements 8412, adjacent to the inflow side 834 of the first row of anti-paravalvular leakage elements 8411 and covering only partial areas of the respective cells; and
a third row of anti-paravalvular leakage elements 8413, adjacent to the outflow side 833 of the first row of anti-paravalvular leakage elements 8411 and covering only partial areas of the respective cells.

Referring to FIG. 45, in another embodiment, the anti-paravalvular leakage elements 841 include:
a first row of anti-paravalvular leakage elements 8411, fully covering the respective cells; and
a second row of anti-paravalvular leakage elements 8412, adjacent to the inflow side 834 of the first row of anti-paravalvular leakage elements 8411 and covering only partial areas of the respective cells.

Referring to FIG. 46, in another embodiment, the anti-paravalvular leakage elements 841 include:
a first row of anti-paravalvular leakage elements 8411, fully covering the respective cells; and
a third row of anti-paravalvular leakage elements 8413, adjacent to the outflow side 833 of the first row of anti-paravalvular leakage elements 8411 and covering only partial areas of the respective cells.

The axial lengths of the second row of anti-paravalvular leakage elements 8412 and the third row of anti-paravalvular leakage elements 8413 are only half the length of a cell, respectively.

Each anti-paravalvular leakage element, from the outflow side 833 to the inflow side 834, gradually increases in thickness, reaching a maximum outward protrusion (in the radial direction of the stent), and then gradually decreases in thickness. The variation in thickness facilitates the retraction of the maximum outward protrusion. The maximum outward protrusion is closer to the inflow side 834. As can be seen in the figure, the distance between the maximum outward protrusion of the anti-paravalvular leakage element 841 and the inflow side 834 of the corresponding cell is S1, and the distance between the maximum outward protrusion of the anti-paravalvular leakage element 841 and the outflow side 833 of the corresponding cell is S2, where the ratio of S1 : S2 ranges from 0.2 to 0.8. For example, as shown in FIG. 46, S1 : S2=0.8.

The anti-paravalvular leakage element protruding outward can fill the space enclosed by the struts of the corresponding cell. In terms of the protrusion extent, the anti-paravalvular leakage element closely contacts the side edges of the struts of the corresponding cell, ensuring that the lowest position of the anti-paravalvular leakage element is not lower than the outer peripheral surface of the struts of the corresponding cell. This design prevents gaps between the anti-paravalvular leakage elements and the side edges of the struts, thereby avoiding the absorption of deformation by these gaps, which could otherwise reduce the sealing effectiveness. Here, the "side edge of the strut" refers to the side facing the interior of the corresponding cell.

The control handle of the present disclosure can independently operate the outer sheath, so that the movement of the outer sheath is separated from those of other internal tubes while also eliminating restrictions on the main handle's length. To retract the outer sheath, the auxiliary handle can be directly driven, with sufficient travel to ensure that the distal end of the outer sheath disengages from the aortic arch. For example, when the auxiliary handle is retracted proximally to the extreme proximal position, the distal ends of the remaining tubes of the catheter assembly, especially the distal end of the deflectable tube, are exposed (released from the constraint of the outer sheath) for a considerable length, typically ranging from 0 to 20 cm. The overall travel of the auxiliary handle is generally in the range of 10 to 40 cm, in order to avoid the aortic arch or the bent part of the distal end of the catheter assembly, thereby facilitating the adjustment of the prosthetic implant or other operations.

Referring to FIG. 47, the existing interventional delivery system for balloon-expandable valves generally includes an inner shaft 44 and a balloon catheter 42 that are slidably engaged with each other. The distal end of the balloon catheter 42 is a balloon body 423 that is inflated by fluid to deploy the prosthetic implant. After being loaded, the prosthetic implant 41 (i.e., a prosthetic heart valve, hereinafter also referred to as a valve) is crimped to a smaller diameter, enclosing the outer periphery of the balloon body 423, roughly positioned in the middle of its axial length. When the prosthetic implant 41 reaches the intended position in the human body, fluid is injected into the balloon body 423 from the proximal end to inflate it, ultimately achieving balloon expansion-based deployment of the prosthetic implant 41 in the human body.

During surgery, fluid needs to be injected into the balloon body 423. Since the middle part of the balloon body 423 is compressed by the prosthetic implant 41 and basically fits the outer periphery of the inner shaft 44, the flow channel in this part is blocked. Therefore, the fluid generally inflates the proximal part of the balloon body 423 first, which poses a risk of shifting the prosthetic implant 1 towards the distal end, thereby affecting its positional accuracy in the human body.

Referring to FIG. 48, some existing balloon catheters introduce a support member in the middle of the balloon body 423, which can maintain the radial gap between the balloon body 423 and the inner shaft 44 to a certain extent, playing a role in supporting the internal flow channel and alleviating the issue of severe inflation delay at the distal end of the balloon catheter. However, due to the size limitation of the support member, after the fluid enters the balloon body 423, it still primarily inflates the proximal end first, with only a portion of the fluid reaching and inflating the distal end of the balloon body 423 at the same time as or slightly later than the inflation of the proximal end of the balloon body 423. Although this reduces the inflation time difference between the two ends of the balloon body 423, the effect remains suboptimal.

In practical applications, it is desirable for the balloon to form a "dog bone" shape (or "figure-eight" shape), meaning that the two axial ends of the balloon body 423 inflate first, followed by the central portion. This configuration not only aids in positioning the valve-preventing it from sliding on the balloon body 423 during the inflation of the balloon body 423-but also ensures uniform inflation at both ends of the balloon body 423, thereby providing a balanced expansion force to the valve.

Referring to FIGS. 49 to 53, an embodiment of the present disclosure discloses a method for driving a balloon catheter with fluid. The balloon catheter 42 has a distal end 422 and an opposite proximal end 421. The distal end 422 is the end away from the operator, and the proximal end 421 is the end close to the operator.

In this embodiment, the balloon catheter 42 includes a balloon body 423 and a catheter body 424. The catheter body 424 is in communication with the proximal end of the balloon body 423. The balloon body 423 includes a first part 4233, a middle part 4232 and a second part 4231 from the distal end to the proximal end. The inner cavities of these three parts are in communication with each other and form an internal flow channel for fluid injection. The middle part 4232 is where the prosthetic implant 41 is loaded and fixed. Specifically, in the loaded state shown in FIG. 50a, the prosthetic implant 41 is radially compressed, enclosing the outer periphery of the middle part 4232. The catheter body 424 has a main flow channel 4241 therein.

The operation process of the method in this embodiment includes the following steps:
injecting fluid into the main flow channel 4241 from the proximal end of the catheter body 424; and
diverting the fluid output from the main flow channel 4241 to the balloon body 423, allowing only part of the fluid to enter and inflate the second part 4231 while directing the other part of the fluid to pass through the second part 4231 and the middle part 4232 and enter and inflate the first part 4233.

To avoid complexity in the flow channel design, the method ensures that all fluid used for inflating the balloon body 423 originates from the main flow channel 4241 before being diverted. From a fluid supply perspective, all fluid used for inflating the balloon body 423 is supplied from the proximal end of the catheter body 424 through the main flow channel 4241. Only diversion is made for different inflation parts (i.e., the first part 4233 and the second part 4231). Inside the catheter body 424, no other pipelines or flow channels for supplying fluids are configured except the main flow channel 4241. In addition, the interior of the main flow channel 4241 does not need to be partitioned (e.g., a multi-lumen parallel structure).

As shown in FIG. 50b, the fluid in the main flow channel 4241 (illustratively labeled as fluid Q1 and fluid Q2 in the figure to indicate the subsequent fluid diversion, though in reality, they remain mixed before diversion) is diverted when being output to the balloon body 423. A part of the fluid Q1 enters and inflates the second part 4231, and the other part of the fluid Q2 is diverted by an independent flow channel 426 and enters and inflates the first part 4233 after passing through the second part 4231 and the middle part 4232. The other part of the fluid Q2 flows inside the independent flow channel 426, and when passing through the second part 4231 and the middle part 4232, it is isolated from the fluid in these two parts to avoid pressure loss and diffusion. Since all diverted fluid originates from the catheter body 424, the fluid delivery space provided in the catheter body 424 is referred to as the main flow channel.

The material of the balloon body 423 generally exhibits greater compliance or elasticity than that of the catheter body 424, meaning it is more prone to radial expansion when subjected to fluid pressure. Therefore, when the fluid enters the balloon body 423 from the catheter body 424, with the deformation of the balloon body 423, the fluid will present a relatively more obvious turbulent state compared to that in the catheter body 424, and then directly act on the second part 4231 to inflate it. In this embodiment, before the fluid entering the balloon body fully transitions into a turbulent state for inflation, it is partially diverted-allowing at least part of the fluid to directly act on the second part 4231 for inflation.

The fluid diversion in this embodiment is for the fluid from the main flow channel 4241. The radial gap between the catheter body 424 and the inner shaft 44 is the main flow channel 4241. The main flow channel 4241 is a single flow channel without additional partitioning into multiple lumens or parallel pipelines. To simplify the structure, all fluid within the catheter body 424 is output from the main flow channel 4241 to the balloon body 423.

The diverted part of the fluid is directed through an independent flow channel 426, which serves as a relatively enclosed flow guide channel. This design prevents the fluid entering the independent flow channel 426 from participating or excessively participating in the inflation of the second part 4231, reducing unnecessary diffusion while passing through the second part 4231 and the middle part 4232. This part of the fluid can reach the first part 4233 as quickly as possible along the independent flow channel 426 with minimal resistance, so as to reduce the time difference of the inflation deformation between the first part 4233 and the second part 4231.

The primary purpose of the fluid diversion is to partially intercept the fluid from the main flow channel 4241 that will flow into the inner cavity of the second part 4231, that is, to split all the fluid into two streams, inject them into the first part 4233 and the second part 4231 as synchronously as possible, and respectively inflate the first part 4233 and the second part 4231 simultaneously by acting on the inner wall of the balloon body 423, overcoming the defect that the proximal end inflates first in the prior art, and further solving the problem of positional displacement during the deployment of the prosthetic implant.

Referring to FIG. 54, before the fluid 45 in the main flow channel 4241 is injected into the balloon catheter 423, the entire balloon catheter 423 is still in a compressed state because the prosthetic implant 41 is radially compressed, enclosing the outer periphery of the middle part 4232.

Regarding the timing or relative position of fluid diversion, referring to FIGS. 55a to 55b, in one embodiment, the fluid 45 flowing out of the distal end of the main flow channel 4241 and about to flow into the inner cavity of the second part 4231 is partially intercepted, that is, the fluid is split into two streams at the junction of the catheter body 424 and the second part 4231: one stream enters and inflates the second part 4231, while the other stream passes through the second part 4231 and the middle part 4232 via the diversion flow channel formed by the independent flow channel 426, and enters and inflates the first part 4233.

In another embodiment, referring to FIG. 56 and FIG. 57a to FIG. 57c, the fluid 45 (including the fluid Q1 and the fluid Q2) flowing out of the distal end of the main flow channel 4241 and about to flow into the second part 4231 can be entirely intercepted, that is, all the fluid output from the distal end of the main flow channel 4241 enters the independent flow channel 426. The independent flow channel 426 is in communication with a branch flow channel 4261, which is further in communication with the second part 4231. When a part of the fluid Q1 flows through the second part 4231, it enters and inflates the second part 4231 through the branch flow channel 4261, while the other part of the fluid Q2 passes through the second part 4231 and the middle part 4232 through the independent flow channel 426, and enters and inflates the first part 4233. In this embodiment, the proximal end of the independent flow channel can be directly connected to and in communication with the distal end of the catheter body 424, or the independent flow channel and the catheter body 424 can be formed in one piece. This design can further delay the inflation of the second part 4231, helping to maintain synchronization with the deformation of the first part 4233.

After the first part 4233 and the second part 4231 undergo nearly simultaneous inflation and deformation, since the middle part 4232 is more radially constrained by the prosthetic implant and its deformation is more lagging, the balloon body 423 enters a transitional state in which both ends are inflated and the middle part is relatively contracted. In this state, the position of the prosthetic implant can still be adjusted or verified. Further fluid injection into the catheter body 424 continues until the balloon body 423 is fully inflated and the prosthetic implant 41 is radially expanded, as shown in FIG. 58. This process essentially represents a balloon expansion-based deployment method for the prosthetic implant.

As mentioned above, during the diversion process, the fluid is diverted into two streams, which respectively inflate the first part 4233 and the second part 4231. Within each stream, the fluid may be further split into multiple sub-streams. FIG. 59 illustrates an example where the fluid Q2 inflating the first part 4233 is split into three sub-streams (fluid Q21, fluid Q22, and fluid Q23).

These sub-streams within each stream are radially distributed and can be achieved using flow splitters or branch flow channels 4261. This ensures that different circumferential sections of the balloon body 423 expand uniformly or nearly uniformly, reducing the complexity of controlling flow rate or flow velocity.

In order to ensure the synchronous deformation effect, the flow rate ratio of one stream of fluid directly acting on the second part 4231 and the other stream of fluid directly acting on the first part 4233 is set within the range of 1 : 0.6 to 1.5, and the flow rate can be adjusted accordingly by changing the cross-sectional area of the flow channel.

The prosthetic implant 41 is a cylindrical structure. The fluid entering the independent flow channel 426 passes through the middle part 4232 through the interior of the cylindrical structure and enters the first part 4233. This prevents interference with the diversion flow channel caused by deformation of the prosthetic implant.

The balloon body 423 has an axial direction extending between the distal end and the proximal end, as well as the corresponding radial and circumferential directions. After the fluid is output from the diversion flow channel, it enters and inflates the first part 4233 in different directions:
being output in the radial direction of the balloon body 423 from the independent flow channel 426 and inflating the first part 4233; and/or
being output in the axial direction of the balloon body 423 from the independent flow channel 426 and inflating the first part 4233.

The portion of the independent flow channel 426 located within the first part 4233 may be provided with corresponding output holes or flow guides according to the fluid output direction.

Some embodiments below further provide an interventional delivery device based on balloon expansion deployment, which can be used to implement the above method.

Referring to FIG. 60, an embodiment of the present disclosure provides an interventional delivery device based on balloon expansion deployment, having a distal end 422 and a proximal end 421 opposite to each other. The distal end 422 is the end away from the operator, and the proximal end 421 is the end close to the operator. The interventional delivery device includes an inner shaft 44 and a balloon catheter 42 located around the outer periphery of the inner shaft 44, with a radial gap between the inner shaft 44 and the balloon catheter 42, which is an internal channel for fluid injection.

The balloon catheter 42 includes a balloon body 423 and a catheter body 424. The catheter body 424 is in communication with the proximal end of the balloon body 423. The balloon body 423 includes a first part 4233, a middle part 4232 and a second part 4231 from the distal end to the proximal end. The middle part 4232 is where the prosthetic implant 41 is loaded and fixed. The catheter body 424 has a main flow channel 4241. A flow guide tube 425 is provided in the radial gap between the inner shaft 44 and the balloon body 423. At least part of the fluid output from the main flow channel 4241 to the balloon body 423 enters and inflates the first part 4233 after passing through the second part 4231 and the middle part 4232 via the flow guide tube 425. The flow guide tube 425 diverts the fluid output from the distal end of the catheter body 424. The specific principle, process and the corresponding effects can be referred to the above description.

The flow guide tube 425 can divert the fluid from the main flow channel 4241 into two parts: one part enters and inflates the second part 4231, and the other part enters the flow guide tube 425 (corresponding to the independent flow channel 426 mentioned above; since the flow guide tube 425 is located around the outer periphery of the inner shaft 44, the independent flow channel 426 is the radial gap between the flow guide tube 425 and the inner shaft 44) and is directly delivered to the first part 4233 to avoid diffusion in the second part 4231 and the middle part 4232. This embodiment uses a simple tube for fluid diversion, which not only reduces costs and facilitates assembly but also allows for the use of the same material as surrounding components based on strength requirements, thereby reducing material selection difficulties.

Some embodiments below further provide an interventional delivery device based on balloon expansion deployment, which can be used to implement the above method.

Referring to FIGS. 61a to 61b, an embodiment of the present disclosure provides an interventional delivery device based on balloon expansion deployment, having a distal end 422 and a proximal end 421 opposite to each other. The interventional delivery device includes an inner shaft 44 and a balloon catheter 42 located around the outer periphery of the inner shaft 44. The balloon catheter 42 includes a balloon body 423 and a catheter body 424. The catheter body 424 is in communication with the proximal end of the balloon body 423. The balloon body 423 includes a first part 4233, a middle part 4232 and a second part 4231 from the distal end to the proximal end. The middle part 4232 is where the prosthetic implant 41 is loaded and fixed. The proximal ends of the inner shaft 44 and the catheter body 424 can be connected to and controlled by a control handle. As for the control handle itself, either conventional technology or the solutions provided by the embodiments of the present disclosure can be adopted.

A flow guide tube 425 is provided in the radial gap between the inner shaft 44 and the balloon body 423. The distal end of the flow guide tube 425 is fixed to the distal end of the inner shaft 44, and the remaining part of the flow guide tube 425 is suspended between the inner shaft 44 and the balloon catheter 423.

At least part of the fluid output from the inner shaft 44 to the balloon body 423 enters and inflates the first part 4233 after passing through the second part 4231 and the middle part 4232 via the flow guide tube 425.

When the interventional delivery device is delivered in the human body, it will turn and bend to adapt to the physiological structure in the human body, which will cause displacement of the flow guide tube 425. In addition, the action of the fluid may also cause the displacement of the flow guide tube 425, potentially affecting the diversion effect. This embodiment applies the previously described fluid diversion principle using the flow guide tube. The key point is that the distal end of the flow guide tube 425 is fixed relative to the inner shaft 4, which provides overall positional stability of the flow guide tube 425, while allowing relative movement of the remaining part of the flow guide tube 425 through the suspension design. The suspension design means that no connecting or constraining components or matching structures are placed in the radial gap between the flow guide tube 425 and the inner shaft 44, and the flow guide tube 425 and the inner shaft 44 can move relative to each other in the radial direction within the range of the gap.

The suspension design enhances the overall compliance of the balloon catheter. In addition, in the initial stage of balloon expansion, the fluid inlet of the flow guide tube 425 may be partially blocked by the inner wall of the compressed balloon. The suspension design enables the proximal end of the flow guide tube 425 to self-adjust by shifting radially relative to the inner shaft 44, ensuring sufficient exposure of the fluid inlet area.

Referring to FIGS. 62 to 63, the proximal portion of the flow guide tube 425 has an open port serving as a fluid inlet 4254. The fluid (the part that needs to be diverted) output from the catheter body 424 directly enters the interior of the flow guide tube 425 through the fluid inlet 4254. The fluid inlet 4254 can be located at the proximal end of the flow guide tube 425, or an opening on its sidewall. The inlet diameter (cross-sectional area) may be equal to or slightly larger than the inner diameter of the flow guide tube 425, both of which can achieve the diversion effect.

The axial position of the fluid inlet 4254 is adjacent to the junction of the balloon body 423 and the catheter body 424. In the inflated state, the inner diameter of the junction of the balloon body 423 and the catheter body 424 changes significantly. Therefore, this region is also more susceptible to alterations in fluid flow dynamics before inflation. The axial position of the fluid inlet 4254 is adjacent to this region, which can obtain a good diversion timing and effect. The balloon body 423 is inflated after being perfused with fluid. In the inflated state, the fluid inlet 4254 is located at the region where the cross-sectional area of the flow channel of the second part 4231 relative to the catheter body 424 changes abruptly.

At the proximal port of the flow guide tube 425, there is a first gap with a radial span of L1 between the inner shaft 44 and the flow guide tube 425, and the first gap affects the flow rate of the part of the fluid diverted into the flow guide tube 425, i.e., the flow capacity of the diversion flow channel. There is a second gap with a radial span of L2 between the inner shaft 44 and the catheter body 424, and the second gap affects the total flow rate of the fluid. Since the inner diameter of the flow guide tube 425 is relatively small, the fluid stream entering the flow guide tube 425 requires a slightly larger flow rate to achieve the same flow rate as the other fluid stream. Therefore, the radial span ratio of the two gaps satisfies L1 : L2 = 1 : 1.25 to 1.6, ensuring that both fluid streams after diversion achieve approximately the same inflation rate.

In the loaded state, the prosthetic implant is radially compressed, enclosing the outer periphery of the middle part 4232, while the first part 4233 and the second part 4231 remain exposed. This configuration reduces the initial deformation pressure required for expansion and minimizes the risk of prosthetic implant slippage.

In FIG. 63, the proximal end of the flow guide tube has a fluid inlet 4254, which is located in the second part 4231 for fluid diversion. The fluid delivered to the first part 4233 is output through the first fluid outlet 4255 located in the first part 4233.

The flow guide tube 425 includes a distal section 4253, a middle section 4252 and a proximal section 4251 from the distal end to the proximal end, with each corresponding to a different part of the balloon body 423. The distal section 4253 of the flow guide tube 425 has a first fluid outlet 4255 in communication with the first part 4233. The first fluid outlet 4255 is located on the tube wall of the distal section 4253 and/or the end face of the distal section 4253.

To achieve a radial fluid delivery effect, the tube wall of the distal section 4253 is provided with a plurality of first fluid outlets 4255, for example, two to four. The plurality of first fluid outlets are distributed along the circumference of the flow guide tube 425, so that the fluid can flow uniformly from the periphery of the flow guide tube to the first part of the balloon body, and the inflation force applied to the balloon body is more uniform.

In the length direction of the flow guide tube 425, the plurality of first fluid outlets 4255 are located in the middle of the distal section 4253. This configuration shortens the flow path of the fluid along the outer circumference of the flow guide tube, ensuring that the central portion of the first part 4233 of the balloon body receives the initial inflation force first, which helps achieve more uniform balloon inflation.

Referring to FIG. 64, the proximal end of the flow guide tube 425 and the distal end of the catheter body 424 are interconnected and integrated into one piece, and a second fluid outlet 4256 communicating with the second part 4231 is opened on the tube wall of the proximal section 4251.

The fluid output from the distal end of the catheter body 424 can all enter the flow guide tube 425, and then be redistributed through the flow guide tube 425 to enter and inflate different parts of the balloon body 423. This embodiment allows for control of the fluid flow rate through the configuration of the position and effective area of the first fluid outlet 4255 and the second fluid outlet 4256, improving the controllability of the inflation process in both the first part 4233 and the second part 4231.

Referring to FIGS. 65a and 65b, in order to further stabilize the spatial position of the proximal end of the flow guide tube 425, the proximal end of the flow guide tube 425 is provided with a tapered section 4257 with a gradually converging shape. At least a part of the tapered section 4257 extends into the distal end of the catheter body 424.

The tapered section 4257, at least the portion near the tip, can extend into the catheter body 424, thereby limiting the radial spatial position. The distal end of the tapered section 4257 can be opened for direct flow guide. The tapered section 4257 has one or two beveled surfaces. As shown in FIG. 65a, a beveled surface 4258 is provided at the distal end of the tapered section 4257 to guide the fluid output from the distal end of the catheter body 424.

As shown in FIG. 65b, in order to reduce radial eccentricity, two symmetrically shaped beveled surfaces can be used. The inner lumen of the flow guide tube 425 is open to the beveled surfaces 4258 and is simultaneously in communication with the inner lumen of the catheter body 424 and the inner cavity of the second part 4231. This structure can serve both for positioning and direct diversion at the end port. Depending on the bevel angle, different flow rate relationships between two streams can also be achieved.

In some scenarios, in order to maintain the radial relative position of the flow guide tube 425 and the circumferential uniformity of the diversion flow channel, a spacer 441 is provided between the inner shaft 44 and the flow guide tube 425 in the radial direction to maintain the radial gap therebetween.

The spacer 441 may be a separate component or an integrated part of the corresponding component. For example, as shown in FIG. 66, ribs are distributed on the inner wall of the flow guide tube to achieve the purpose of spacing.

The spacer 441 may also be a hollow structural component fixed between the inner shaft 44 and the flow guide tube 425 in the radial direction.

In order to position the distal end of the flow guide tube 425, referring to FIGS. 67a to 67b, a guide head 43 is fixed to the distal end of the inner shaft 44, and the distal end of the flow guide tube 425 is fixed to the proximal side of the guide head 43. After the distal end of the flow guide tube 425 is effectively fixed, the distal end of the flow guide tube 425 is closed by the guide head 43, so that when the fluid enters the distal end of the flow guide tube 425, it can only enter the balloon body from the plurality of fluid outlets, which is conducive to the uniform inflation of the balloon body.

The proximal side of the guide head 43 is provided with a positioning structure 431 adapted to the distal end of the flow guide tube 425. The positioning structure 431 is a coupling groove for inserting the flow guide tube 425 (as shown in FIG. 67a), or a coupling column for inserting the flow guide tube 425 (as shown in FIG. 67b).

The present disclosure achieves synchronous deformation of the balloon body 423 at both ends of the prosthetic implant by distributing the fluid entering the balloon body 423. It reduces the risk of positional offset of the prosthetic implant during deployment and eliminates the need for an axial positioning structure for the prosthetic implant.

FIG. 68 shows an interventional delivery system 51, which includes a control handle 53, and a catheter assembly 54 controllably connected to the control handle 53. A prosthetic implant 55 is loaded onto and delivered by the catheter assembly 54. When balloon expansion is used for deployment, the catheter assembly 54 may include a balloon device, and a limiting mechanism 52 may be applied to the balloon device. The catheter assembly 54 may include an outer sheath that is slidably fitted over of the balloon device and an intermediate shaft in the outer sheath as needed. The interventional delivery system 1 generally has a proximal end 5101 and a distal end 5102 as shown in the figure.

The prosthetic implant has a loaded state in which it is loaded on the catheter assembly 54 and an expanded state. The prosthetic implant can be a heart valve, such as a prosthetic aortic valve, a prosthetic pulmonary valve, or the like. The prosthetic implant hereinafter takes a prosthetic heart valve as an example, which includes a stent and leaflets arranged on the stent. The stent is made of stainless steel material among others and can be deployed via balloon expansion, and the stent itself can be formed by cutting a tube.

Referring to FIGS. 68 to 71, the present disclosure provides a limiting mechanism 52 for an interventional delivery system, including a coupling portion 521 and a deformable portion 523. The coupling portion 521 is used to connect with the interventional delivery system 51, and the coupling portion 521 has the same axial direction as the delivery system 51 and the corresponding circumferential and radial directions. The deformable portion 523 includes a plurality of rods arranged in the circumferential direction. Each rod has a first end 5251 and a second end 5252. The first end 5251 is connected to the coupling portion 521. The second end 5252 is located on one axial side of the coupling portion 521 relative to the first end 5251. The rods are elastically deformable, allowing them to transition between a compressed state, suitable for interventional delivery, and an expanded state. In the expanded state, the second ends 5252 of the rods diverge outward in the radial direction of the coupling portion 521 compared to their positions in the compressed state.

The existing structures have poor blocking effects, especially after the balloon body is slightly inflated, that is, the blocking effect is lost in the initial stage of deployment of the prosthetic implant. In addition, some existing structures are complex and require corresponding unlocking operations.

The rods in this embodiment are generally elongated structures. On the one hand, the rods, especially at the second ends 5251, have less mutual traction, avoiding affecting the overall effect when local failure occurs. This effect is particularly significant when the prosthetic implant is eccentrically positioned. On the other hand, the rods allow for relatively large radial expansion and exhibit good compliance with the balloon folding deformation described below.

The lengths of the rods 525 can have the same or different lengths. The elongated structure is defined as the rod 525 having a length at least five times the rod diameter, preferably twenty times. The length of the rod 525 refers to the straight-line distance between the first end and the second end. If the rod 525 has a circular cross-section, the rod diameter corresponds to the diameter of the circular cross-section. If the cross-section of the rod is of other shapes, the rod diameter refers to the equivalent circular diameter with the same cross-sectional area.

To facilitate gathering and outward expansion, the rods possess elasticity. For example, they can be made of Nitinol memory alloy, which is heat-treated to obtain a preset shape, in which the rod naturally expands without constraints.

The expanded state of the rods refers to the state in which the ends of the rods expand outward to the maximum extent when the limiting mechanism is in use. For example, when the limiting mechanism is used in conjunction with the balloon body, after the balloon body is inflated, the ends of the rods expand outward in the expanded state.

In practical applications, once the balloon body is inflated, the radial constraint on the second ends of the rods may be completely removed, so that the expanded state of the rods is consistent with their own preset shape state. As shown in FIG. 77a, if the rods are not restricted by the inflated balloon body, the expanded state corresponds to the preset shape state.

On the other hand, after the balloon body is inflated, the end shape of the balloon body may still impose some radial constraints on the second ends of the rods, causing the expansion angle of the rods in the expanded state to be smaller than in the preset shape state.

In this embodiment, the expanded state of the rods is consistent with the preset shape state, which is more conducive for the balloon body when being folded and retracted during the retraction of the delivery system to guide the radial retraction of the rods. The compressed state of the rods refers to the radial retraction of the second ends of the rods, which is at least suitable for transcatheter interventional delivery. The object to which the external force is applied to keep the second ends radially retracted can be at least one of the balloon device or the outer sheath.

For example, in another embodiment, in order to obtain a larger expansion range, the distance between the second end of the rod and the axis of the coupling portion in the expanded state is R1; the distance between the second end of the rod and the axis of the coupling portion in the compressed state is R2; and R1 : R2 = 2 - 20 : 1. For example, R1 : R2 = 2 - 10 : 1. In this embodiment, the shape of the rod is not strictly limited, although an elongated structure provides better results.

The coupling portion 521 is connected to the catheter assembly 54. The rods 525 can gather together to form a cylindrical structure with the coupling portion 521. The rods 525 are radially distributed, with their first ends 5251 converging at one axial end of the coupling portion. The rod 525 has an expansion angle α relative to the axial direction. For example, the expansion angle α in the compressed state (FIG. 71) is smaller than the expansion angle α in the expanded state (FIG. 70). In a given state, the expansion angles of the rods can be identical or different. The axial positions of the second ends can be aligned or staggered (meaning the rods may have different lengths). The angle α and rod length can be used to calculate the distance R2 between the second end and the axis of the coupling portion.

The rod 525 extends radially outward relative to the prosthetic implant 55 in the loaded state or at least is not lower than the prosthetic implant 55. The second end 5252 faces the prosthetic implant 55 to prevent it from moving toward the limiting mechanism 52. Limiting mechanisms 52 can be respectively arranged on both axial sides of the prosthetic implant 55, restricting its axial displacement relative to the catheter assembly 54.

The rods 525 can adapt to changes in the surrounding environment. For example, when the prosthetic implant 55 is in a loaded state, the rods 525 can be deformed and radially contracted to facilitate the loading process of the prosthetic implant; and after that, the rods 525 can adaptively deform to restrict the displacement of the prosthetic implant 55.

The limiting mechanism in this embodiment has a simpler structure and does not require unlocking via the control handle, avoiding an increase in the radial size of the distal end of the delivery system.

As shown in FIG. 72, the catheter assembly includes at least one tube, and the coupling portion 521 is connected to the tube (illustrated as the inner shaft 545 below). The coupling portion 521 is a radially deformable annular structure, and the coupling portion 521 is arranged around the inner shaft 545. When assembling the limiting mechanism, the coupling portion 521 is first radially expanded, fitted onto the inner shaft 545 and moved to a predetermined position, and then the coupling portion 521 is radially contracted to conform to the outer surface of the inner shaft 545 to achieve the connection between the two. The radial expansion and contraction of the coupling portion 521 can be achieved by applying an external force or by utilizing the inherent elastic deformation of the coupling portion 521. For instance, once the external force is removed, the coupling portion 521 contracts back due to its elasticity. Accordingly, materials like shape memory alloys can be used for the coupling portion 521 to minimize damage to the inner shaft by the coupling portion during assembly.

The radial deformation of the coupling portion 521 can be implemented by designing it in a spring-like form. As shown in FIG. 73, in one embodiment, the coupling portion 521 has an axially undulating wave structure with alternating peaks 5211 and valleys 5212. The side connected to the first end 5251 of the deformable portion 523 corresponds to the peaks 5211. This wave structure facilitates radial deformation during assembly and the peaks 5121 arranged at intervals further reduce mutual interference between the rods.

The limiting mechanism 52 can be formed in one piece by cutting a single tube, with an inner diameter larger than the outer diameter of the inner shaft. For example, the tube may have an inner diameter of 3.5 - 4.5 mm, while the inner shaft has an outer diameter of 1 - 2 mm, making the tube's inner diameter approximately 2 - times that of the inner shaft.

To reduce potential damage to the balloon body, the rods (especially at the second ends) can have relatively wide dimensions in the circumferential direction. For example, the coupling portion 521 can be cut from a tube with an outer diameter of approximately 5 mm and a wall thickness of 0.3 - 0.7 mm. After compression, it can be mounted onto the inner shaft having an outer diameter of about 1.3 mm. Additionally, the coupling portion 521 can be tightly pressed against the inner shaft's outer surface to increase friction, and adhesive can be applied for further fixation. When assembling the coupling portion 521 with the balloon body, it can be inserted into the balloon body's tubular end and then welded in place, securing it within the balloon body's tubular end.

In FIG. 69, there are two limiting mechanisms for limiting the distal end and the proximal end of the prosthetic implant 55. In some embodiments, when only the distal end or the proximal end of the prosthetic implant needs to be limited, only one limiting mechanism is required.

Regarding the connection between the coupling portion 521 and the deformable portion 523, as shown in FIG. 74, the two can be formed in one or two pieces. Preferably, the two are formed in one piece, and the wave structure and rods 525 are formed in one piece by cutting a tube.

The rods 525 of the deformable portion 523 tend to extend helically around the coupling portion's axis. As shown in FIG. 74, one rod extends along a helical path 5253 (illustrated as a dashed line), with the starting point of the helical path 5253 located at the corresponding peak 5211.

The helical extension is beneficial because it increases the effective length of the rod 525 while maintaining the same axial span. This configuration also facilitates placement within the balloon's folds.

Given that the rods are structurally independent, in another embodiment, when assembling the limiting mechanism 52 in the balloon body 542, the balloon body 542 has a folded state and an inflated state. In the folded state, the balloon body 542 features multiple folds 5424, into which the rods 525 can be inserted.

As shown in FIGS. 77a to 77d, based on the limiting mechanism 52 described in the previous embodiments, an embodiment of the present disclosure further provides a balloon device 541 for delivering a prosthetic implant, including an inner shaft 545 and a balloon body 542 located on the outer periphery of the inner shaft, and the inner shaft 545 is provided with a limiting mechanism 52 located inside the balloon body 542.

The inner shaft can be a hollow structure, for example, it can be used to pass a guide wire. The balloon body 542 can be inflated under the action of fluid to deploy the prosthetic implant, and the fluid used to inflate the balloon body can be delivered via the inner shaft. Optionally, the delivery system further includes an intermediate shaft 543 fitted over the outer periphery of the inner shaft. The distal end of the intermediate shaft 543 can be connected with the proximal end of the balloon body 542, and the two are connected so that the radial gap between the intermediate shaft 543 and the inner shaft 545 is in communication with the proximal end of the balloon body 542 for fluid delivery. The intermediate shaft 543 and the inner shaft 545 are both tubes and can be connected to the control handle and configured with corresponding interfaces.

As shown in FIG. 77a, the catheter assembly of the interventional delivery system may further include an outer sheath 544, the distal end of the outer sheath 544 is slidably fitted over the outer periphery of the balloon body 542, and the proximal end of the outer sheath 544 is controllably connected to the control handle. The outer sheath 544 can act on the balloon body 542 to further compress the rods 525 of the limiting mechanism 52, ensuring that the outer peripheral surface of the distal end of the delivery system remains smooth and streamlined, which facilitates interventional delivery within the human body and minimizes potential injury to surrounding tissues. After the prosthetic implant is delivered to the intended position, the constraint of the outer sheath 544 can be removed. As shown in FIG. 77b and FIG. 77c, the limiting mechanisms at the distal and proximal ends are exposed outside the outer sheath 544. Correspondingly, the rods 525 slightly expand radially due to their inherent elasticity and then return to the expanded state with the inflation of the balloon body 542.

In this embodiment, when the limiting mechanism is enclosed and constrained by the outer sheath 544, the rods are in a compressed state. After the outer sheath is retracted to release the constraint on the limiting mechanism, the rods are only restrained by the balloon body 542, that is, the overall radial constraint force is reduced, so that the second ends can expand radially outward. However, since the balloon body has not yet been inflated, the rods are not fully expanded and remain in a compressed state. To distinguish this from the previously mentioned compressed state, this state is referred to as an "intermediate state."

Referring to FIG. 77d, when the balloon body, especially the prosthetic implant, is exposed from the outer sheath or in the case where the outer sheath and the limiting mechanism are not configured in the system, before the balloon body 542 is inflated, since the axial end face of the prosthetic implant 55 is positioned higher than the outer surface of the balloon body 542 in the radial direction, a significant radial height transition may occur, creating a step 551. This abrupt transition poses a safety risk.

As shown in FIG. 77e, in one embodiment, the number of the limiting mechanism 52 is one and it is disposed at the distal end of the inner shaft 545. The prosthetic implant 55 is located at the proximal end of the limiting mechanism 52. The rods 525 act on the balloon body 542 so that the outer peripheral surface of the balloon body (specifically, the region adjacent to the distal end of the prosthetic implant) is not lower than the outer peripheral surface of the distal end of the prosthetic implant. This compensates for the previously mentioned step, reducing risk and facilitating trans-valve procedures.

Of course, this configuration can also be applied to the proximal end as needed. Although the rod is not strictly limited to an elongated structure in this embodiment, since the rods are independent of each other, they can vary in a relatively large radial deformation range, making them more effective in compensating for the step 551. This design is more adaptable to different diameters of prosthetic implants or balloon bodies. Other structural features of this embodiment can be combined with those in other embodiments described in this disclosure.

The phrase "not lower than" the outer surface of the distal end of the prosthetic implant means that the protruding portion of the balloon body 542 may be slightly higher than or approximately flush with the outer surface of the distal end of the prosthetic implant. When it is "higher," the relatively soft and adaptively, radially deformable nature of the balloon body 542 prevents it from creating safety risks similar to the step 551.

The inner shaft 545 can have a through guidewire channel. The balloon body 542 is located at the distal end of the inner shaft 545 and has a folded state (FIG. 77b) and an inflated state (FIG. 77c). The balloon body 542 includes a distal section 5423, a middle section 5422, and a proximal section 5421 from the distal end to the proximal end. The outer surface of the middle section 5422 is where the prosthetic implant 55 is placed. There are two limiting mechanisms 52, which are respectively located in the distal section 5423 and the proximal section 5421 of the balloon body 542. The deformable portions 523 of the two limiting mechanisms 52 are arranged opposite to each other to limit the axial position of the prosthetic implant 55.

In the folded state, the length of the middle section 5422 is substantially equal to the axial length of the prosthetic implant 55 in the compressed state. The balloon body 542 is wrapped around the outer periphery of the limiting mechanisms. When the balloon body 542 is folded, the rods are gathered. After loading is completed, the limiting mechanisms 52 enter a compressed state, and the prosthetic implant is radially deformed after being crimped and is located on the outer periphery of the balloon body 542.

The rods 525 of the limiting mechanism 52 are placed in the corresponding folds 5424. The balloon body 542 is folded in an orderly manner, reducing the number of layers between the prosthetic implant and the deformable portion 523 caused by disordered folding. For example, in this embodiment, only one layer of the balloon body material exists between the prosthetic implant and the deformable portion, making the radial protrusion effect of the rods 525 more pronounced, thereby improving the blocking effect. The balloon body 542 is wrapped in a first direction 546 in the circumferential direction in the folded state, and the helical direction of the rods is the same as the first direction 546.

The wider the second end 5252 of the rod 525 is, the more beneficial it is to reduce damage to the balloon body 542. However, to prevent interference with the adjacent folds 5424, the width (or diameter) of the second end 5252 of the rod 525 is in the range of 0.7 - 1.8 mm.

The number of rods 525 is a common divisor of the number of folds 5424. Here, the number of rods 525, the expansion angle, and the size of the second end are described below.

The blocking effect is related to the expansion angle. The larger the expansion angle, the better the blocking effect, but it is easy to cause damage to the balloon body. The more rods there are, the better the blocking effect, but correspondingly, the size of the second end is reduced, which is easy to cause damage to the balloon body. In some embodiments, the number of rods 525 is between 4 and 10. Specifically, for example, there are 4 rods 525 in FIG. 79, or 8 rods 525 in FIG. 80. The length of the rod 525 ranges from 7 to 17 mm.

Since the expansion angle of the rod 525 is related to its elastic performance, in one embodiment, the wall thickness of the deformable portion 523 and the coupling portion 521 is in the range of 0.3 - 0.7 mm. The wall thickness of the two can be the same or different.

When the balloon body 542 is in the folded state, the second end 5252 of at least one rod 525 is not lower than the outer surface of the prosthetic implant 55 in the radial direction, and the second end 5252 acts on the balloon body 542 to form a step 5425 that limits the axial movement of the prosthetic implant. The condition of being "not lower than" includes:
when the inner diameter of the outer sheath 544 is close to the outer diameter of the prosthetic implant, the second end 5252 of the rod 525 can be understood to be equal to the outer diameter of the prosthetic implant in the radial direction;
after the outer sheath 544 is retracted, the rod 525 is allowed to expand radially outward, and its second end 5252 is slightly higher than the prosthetic implant 55 in the radial direction.

As shown in FIGS. 78a to 78c, in another embodiment, the delivery system further includes an intermediate shaft 543, which is outside the inner shaft. There are two limiting mechanisms, including a first limiting mechanism 5210 and a second limiting mechanism 5220. The first limiting mechanism 5210 is installed at the distal end of the inner shaft 545 and is located inside the balloon body 542 (represented by bold lines in the figure). The second limiting mechanism 5220 is installed at the distal end of the intermediate shaft 543, positioned outside the balloon body 542, and located on the proximal side of the first limiting mechanism. The prosthetic implant 55 is located between the two limiting mechanisms. The first limiting mechanism 5210 can adopt any of the limiting mechanisms described in the present disclosure. The second limiting mechanism 5220 is used to fill the radial gap between the outer sheath 544 and the intermediate shaft 543 to prevent the prosthetic implant from moving proximally. The second limiting mechanism 5220 can adopt any of the limiting mechanisms described in the present disclosure or other existing limiting mechanisms.

When the outer sheath 544 is retracted, it follows a predefined sliding path and reaches an extreme position, at which the first limiting mechanism 5210 and the prosthetic implant 55 are exposed outside the outer sheath 544, while the second limiting mechanism 5220 remains inside the outer sheath 544. This embodiment enhances safety by preventing the second limiting mechanism 5220 from being exposed and potentially scratching internal tissue. Preferably, the second limiting mechanism 5220 remains completely inside the outer sheath 544.

The control handle injects fluid, namely, an inflation medium (such as saline) into the balloon assembly 55 through the catheter assembly, inflating the balloon body 542 to expand the prosthetic implant.

The injected fluid can pass through the intermediate shaft. Additionally, the intermediate shaft can slide relative to the balloon body, meaning the second limiting mechanism 220 is movable. Its axial position relative to the balloon body can be adjusted according to the timing or stroke requirements to play a blocking role. In this case, to deliver fluid to inflate the balloon body, the proximal end of the balloon body can be connected to a catheter body. The catheter body can be fitted over the inner shaft, with the radial gap between them serving as a fluid channel. Alternatively, the catheter body and the inner shaft may be arranged side by side, either as separate tubes or as a multi-lumen tube. The proximal end of the catheter body is connected to the control handle and is configured with a corresponding fluid interface.

In some embodiments, the middle section 5422 can guide the inflation medium injected into the balloon body 542 from the proximal section 5421 to the distal section 5423, so that the inflation medium is delivered simultaneously to the distal section 5423 and the proximal section 5421 of the balloon body 542, so as to reduce the probability of axial displacement of the prosthetic implant relative to the balloon body 542 when the balloon body 542 expands the prosthetic implant, and to achieve the intended positioning and orientation of the prosthetic implant during the deployment process.

As shown in FIGS. 80 to 83, in some embodiments, the second end 5252 of the rod has a smooth outer contour; and/or
the second end 5252 of the rod is coated with a protective layer; and/or
the portion of the rod close to the second end 5252 is bent radially inwardly so that the second end 5252 tends to bend radially inwardly.

All three designs are intended to prevent the inner peripheral surface of the balloon body 542 from being scratched by the rods 525 of the deformable portion 523 during the folding/inflation process of the balloon body 542.

The second end 5252 can be coated with a polymer layer to form a protective layer. Preferably, as shown in FIG. 80, a slot 5256 is opened in the portion of the rod 525 close to the second end 5252, and the protective layer is partially embedded in the slot 5256 and fixed by dispensing or the like.

In order to improve the compliance near the second end and better fit and shape the balloon body, for example, in FIGS. 81 and 82, the portion of the rod near the second end has a lower radial stiffness than the rest of the rod. Specifically, the abutment portion 5254 is slotted to form a wave-shaped structure, allowing it to undergo elastic deformation under the action of the balloon body 542, such as radial inward bending and/or circumferential twisting, etc. When the force from the balloon body 542 is removed, the abutment portion 5254 elastically deforms so that the rod returns to its original extension trend. The width of the slot is in the range of 0.02 - 0.2 mm.

As shown in FIG. 83, when no external force is applied, the abutment portion 5254 bends radially inward. As shown in FIG. 84, preferably, when the rod 525 is in the expanded state, the end portion 5255 of the abutment portion 5254 close to the first end 5251 is at the maximum radial dimension of the deformable portion 523. That is, during the folding and inflation process of the balloon body 542, the smooth portion where the end portion 5255 is located acts on the balloon body 542, thereby avoiding damage to the balloon body 542.

As shown in FIG. 85a, in another embodiment, an interventional delivery system based on balloon expansion is provided for delivering a prosthetic implant. The interventional delivery system includes a balloon device 54, an adjustment wire 5710, and a locking wire 5720. The balloon device 54 includes an inner shaft and a balloon body 542 located on the outer periphery of the inner shaft 545. A limiting mechanism (which can be any of the limiting mechanisms described above) located inside the balloon body 542 is installed at the distal end of the inner shaft 545. For example, the limiting mechanism can include a plurality of rods 525 arranged in sequence in the circumferential direction, and the specific structure of the rods 525 and other portions can refer to the previous embodiments. The adjustment wire 5710 is used to releasably fix the prosthetic implant 55 on the balloon body 542. One end of the adjustment wire 5710 can remain fixed to the balloon body 542, and the other end can pass through an eyelet 552 on the prosthetic implant 55, with a locking eyelet 5711 on the other end. The locking wire 5720 has a locked state and an unlocked state. In the locked state, the locking wire 5720 passes through the locking eyelets 5711 to restrict the prosthetic implant 55. In the unlocked state, the locking wire 5720 is disengaged from the locking eyelets 5711 to release the prosthetic implant 55.

There is at least one locking eyelet 5711, one of which is arranged at the proximal end of the prosthetic implant 55. There is at least one adjustment wire 5710, one of which is located on the proximal side of the rods 525 and at least partially outside the balloon body 542. The proximal end of the locking wire 5720 is controlled by the control handle. In the locked state, the distal end of the locking wire 5720 is fixed to the distal end of the inner shaft 545, for example, inserted into the guide head. In the unlocked state, the locking wire 5720 is driven by the control handle to move and switch to the unlocked state.

Before unlocking, the locking wire 5720 can provide axial limitation for the prosthetic implant 55 throughout the inflation process of the balloon body. Combined with the compensation provided by the rods 525 for the radial gap between the balloon body and the prosthetic implant 55, this design offers a broader range of applications.

FIG. 85a illustrates an example with a single adjustment wire positioned at the distal end. As shown in FIG. 85b, in other embodiments, two adjustment wires 710 can be used, positioned at both the distal and proximal ends of the prosthetic implant 55, respectively. As shown in FIG. 85c, in another embodiment, the outer sheath 544 can also be used in combination.

During delivery, the prosthetic implant 55 and the balloon body 542 are enclosed by the outer sheath 544. As the procedure progresses, the outer sheath 544 can be withdrawn proximally to expose the prosthetic implant 55 and the balloon body 542, and then the balloon body 542 is inflated to expand and deploy the prosthetic implant.

The limiting mechanism of the present disclosure has the ability of elastic deformation by optimizing the structure of the deformable portion, thereby extending the blocking time for the prosthetic implant and facilitating the assembly of the prosthetic implant in the compressed state. The deformation of the rods adapts to the changes of the balloon body during the folding and inflation process to reduce the damage to the balloon body. In addition, the mutual radial traction can be reduced, further ensuring the precise positioning of the prosthetic implant in an eccentric state.

An embodiment of the present disclosure provides a balloon device 641 for delivering a prosthetic heart valve, which can be used to deliver and expand a prosthetic implant 65a, which may be the prosthetic heart valve 65 or the anchoring stent 656 as described in other embodiments.

The balloon device 641 includes a catheter body 6411 and a balloon body 642. The extension direction of the catheter body is the axial direction. When the catheter body 6411 is straightened, the radial direction perpendicular to the axial direction and the circumferential direction around the axial direction are also determined. The balloon body is in communication with the catheter body, and the balloon body can receive fluid from the catheter body. The balloon body has a folded state and an inflated state under the action of the fluid. As shown in FIG. 127, in the folded state, the balloon body is connected to the distal end of the catheter body, and the prosthetic heart valve 65 is crimped outside the balloon body. As shown in FIG. 128, the proximal end of the catheter body 6411 is provided with a fluid inlet 645. In the inflated state, the fluid can enter the balloon body from the fluid inlet 645 and inflate the balloon body. Based on the change of the balloon body, the prosthetic implant correspondingly expands from the radially compressed state to the radially expanded state, achieving the deployment of the prosthetic implant 65a based on balloon expansion. In some cases, a fluid inlet can also be provided on the distal end or side wall of the balloon body, which is not shown in the figure.

Referring to FIGS. 86 to 89, the balloon device further includes a limiting mechanism 62 for limiting the axial movement of the prosthetic implant 65a. Specifically, it can limit the axial movement of the prosthetic implant in at least one axial direction, for example, the proximal and/or distal axial movement of the prosthetic implant. The limiting mechanism can be located inside the balloon body or outside the balloon body. At least one of the catheter body and the balloon body is directly fixed to the limiting mechanism, or is indirectly fixed to the limiting mechanism through an intermediate piece.

The limiting mechanism includes a limiting body 622, which includes a plurality of rods (such as rods 221). The rods generally extend from a first axial end to a second axial end and are gathered at both ends, forming a hollow cage-like structure with an internal space 62a. One side of the cage-like structure (i.e., the side facing the prosthetic implant in the use state) is used to limit the axial position of the prosthetic implant 65a.

The rods form the side wall of the cage-like structure, and the gaps between the rods form hollow areas on the side wall. For example, a hollow area is formed between the rods 6221a and 6221b in the figure. Thus, the internal space 62a of the cage-like structure is in communication with the external space 62b through the hollow areas, that is, the hollow areas can be used as a fluid channel for inflation.

One part of the cage-like structure is an outward expansion part 623, which has the greatest radial expansion compared to other parts of the cage-like structure. As shown in FIG. 88 (a longitudinal section of the cage-like structure along its axis), from the outer contour of the cage-like structure, the outward expansion part is the area with the maximum radial dimension. In the axial direction, the outward expansion part can be either a single point or a larger sectional region.

Some of the hollow areas are classified as main hollow areas 6222.

For example, the difference between the main hollow areas 6222 and other hollow areas is that these main hollow areas 6222 extend axially across the outward expansion part with the largest outer diameter of the cage-like structure. For example, the hollow area between the rod 623a and the rod 623b in FIG. 86 is a main hollow area 6222, and the hollow area between the rod 623b and the rod 623e is also a main hollow area 6222.

For another example, the main hollow area 6222 can be a hollow area with a larger axial span than other hollow areas. Referring to FIG. 102, some other hollow areas are classified as auxiliary hollow areas 6223, while the main hollow areas 6222 have a larger axial span than the auxiliary hollow areas 6223.

For another example, the main hollow areas are classified as hollow areas spanning the first end and the second end of the limiting body.

In the circumferential direction of the limiting mechanism, the two main hollow areas 6222 are spaced in the circumferential direction in the outward expansion part. In addition, each main hollow area extends continuously without further division by solid elements.

In the use state, the balloon body is wrapped around the outer periphery of the limiting body. The side of the cage-like structure facing the prosthetic implant limits the axial position of the prosthetic implant 65a, and the side of the cage-like structure away from the prosthetic implant plays a role in providing a guide angle for the distal end of the balloon body, making the interventional delivery of the prosthetic implant in the human body smoother. When the fluid enters the balloon body from the fluid inlet 645, the fluid can flow from the internal space 62a to the external space through the main hollow areas 6222, thereby inflating the balloon body.

The balloon device further includes an inner shaft 643 (which can be used as an intermediate piece), a guide head 646 is fixed to the distal end of the inner shaft, and the inner shaft is inserted into the catheter body and the balloon body. For example, the inner shaft can be a guidewire shaft for accommodating a guide wire.

The limiting mechanism 62 further includes a coupling member 621, and the coupling member 621 can be fixed to the inner shaft and connected to the limiting body. It can be understood that when the balloon device is loaded with a prosthetic implant (i.e., in the use state), the coupling member 621 can be fitted over the inner shaft, with their positions fixed relative to each other. In addition, the coupling member and the limiting body can be directly or indirectly connected, so that the positions of the coupling member, the catheter body, and the limiting body are relatively fixed in the use state. Furthermore, when the limiting mechanism of this embodiment is used, the limiting mechanism is assembled into the balloon body 642 of the balloon device. The balloon body 642 has a folded state and an inflated state. As shown in FIG. 97, in the folded state, the balloon body 642 has a plurality of folded portions 6424, and the folded portions 6424 can be placed in the corresponding main hollow areas. Thus, the balloon body 42 can be folded in an orderly manner through the main hollow areas, and the outer periphery of the outward expansion part is only covered with one layer of balloon body material, avoiding an increase in the maximum radial dimension of the limiting body due to disordered folding.

In addition, during the process of loading the prosthetic implant onto the balloon device, different positions around the limiting body experience varying radial constraint forces, with different force directions. The main hollow areas provided in the outward expansion part enable the rods on both sides to adaptively adjust their spacing in the circumferential direction. This means the rods can self-adjust based on the magnitude and direction of the radial constraint forces (that is, the rods can adaptively adjust the size of the main hollowed area), facilitating easier implant loading.

As shown in FIG. 86, the cage-like structure 624 has two gathering (converging) portions (converging portion 624a, converging portion 624b) spaced from each other in the axial direction, and the side wall of the cage-like structure extends between the two converging portions (that is, the rods extend between the two converging portions). The converging portions surround and are adjacent to the inner shaft. The converging portion 624a and the converging portion 624b are respectively the starting point and the end point of the rods' extension path, and both are positioned around and close to the catheter body. In some embodiments, as shown in FIG. 91 and FIG. 92, an additional converging portion may exist between the two converging portions (converging portion 624a, converging portion 624b) on the rods' extension path, such as converging portion 624d and converging portion 624c. Since radial compression of the limiting mechanism is not expected in the state of limiting the prosthetic implant, introducing an additional converging portion can enhance the circumferential strength of the cage-like structure.

In some embodiments, in the axial direction of the limiting mechanism, at least one side of the main hollow area extends adjacent to the coupling member on that side. Here, "adjacent" means that the two are relatively close, and the rods forming the main hollow area can be directly or indirectly connected. For example, as shown in FIG. 87, the coupling member can be arranged inside the cage-like structure 624 and indirectly connect and support the cage-like structure through the rods. As shown in FIG. 88, there is at least one coupling member 621, and it is connected to one of the converging portions. It can be understood that at least one end of the rods converges at the coupling member.

As shown in FIG. 89, there are two coupling members 621, namely a first coupling member 6211 and a second coupling member 6212, which are spaced apart in the axial direction, and the first coupling member 6211 and the second coupling member 6212 are respectively connected to the corresponding converging portions. The main hollow area extends axially across the outward expansion part of the cage-like structure, with its two sides extending toward and close to the first coupling member 6211 and the second coupling member 6212. The main hollow area 6222 corresponds to the span W1 as indicated in the figure.

As shown in FIG. 93, there are two coupling members 621, namely, a first coupling member 6211 and a second coupling member 6212, which are spaced apart in the axial direction. The proximal side (right side in the figure) of the main hollow area 6222 extends adjacent to the first coupling member 6211, and the main hollow area 6222 corresponds to the span W2 in the figure. Furthermore, the above-mentioned coupling members can be connected to the inner shaft by bonding, welding, or snapping. The outer diameter of the outward expansion part of the limiting body is larger than the outer diameter of the coupling member. For example, the outer diameter ratio of the outward expansion part to the coupling member is in the range of 2 to 4 : 1, preferably 3:1.

Referring to FIGS. 93 to 96, the present disclosure further provides a balloon device 641 for delivering a prosthetic implant. The balloon device 641 includes a catheter body 6411, a balloon body 642, and a limiting mechanism 62. The catheter body, balloon body, and limiting mechanism described in this embodiment may be combined with other embodiments.

The limiting mechanism is configured to limit the axial movement of the prosthetic implant at least when the prosthetic implant is installed on the balloon body in a radially compressed state. Specifically, the limiting mechanism includes a limiting body. The limiting body generally has a cage-like structure 624, which can be formed by multiple converging rods. One part of the cage-like structure is an outward expansion part, which has the largest radial expansion relative to other parts of the cage-like structure. The structure of the cage-like structure and the outward expansion part can also be combined with other embodiments.

In the axial direction of the limiting mechanism, one end of the limiting body is a first end 6224 facing the prosthetic implant 65a in the use state, and the other end is an opposite second end 6225. The outward expansion part 623 is adjacent to the first end of the limiting body, as shown in FIG. 95. When the prosthetic implant 65a is loaded on the balloon device, the limiting body is in the use state, the first end 6224 faces the prosthetic implant 65a, and the second end 6225 is axially spaced from the first end 6224. The limiting body has an axial span, defined by the distance between the first end 6224 and the second end 6225. Relative to the second end 6225, the outward expansion part is closer to the first end.

The balloon device 641 further includes an inner shaft 643, which passes through the catheter body 6411 and the balloon body. The limiting mechanism further includes a coupling member 621, which is fixed to the inner shaft and connected to the limiting body.

The outward expansion part 623 being adjacent to the first end 6224 of the limiting body means that the axial distance H2 between the outward expansion part and the second end 6225 is greater than the axial distance H1 between the outward expansion part and the first end 6224.

Overall, the rods 623d between the outward expansion part and the second end extend with a gentler slop, which facilitates smooth guidance for interventional delivery, while the rods 623c between the outward expansion part and the first end have a steeper inclination, providing better blocking effect and reducing the axial misalignment between the limiting body and the prosthetic implant.

Depending on the extension trend of the rods 623c, as shown in FIG. 95, a concave area 62241 for accommodating the end of the prosthetic implant 65a is formed between the side of the limiting body on the first end 6224 and the coupling member on that side (i.e., the first coupling member 6211). The concave area 62241 is roughly frustum-shaped and converges toward the second end 6225.

The cage-like structure is shown in FIG. 96 (a longitudinal section of the cage-like structure along its axis). From the extension direction of the two ends of the outward expansion part, the cage-like structure extends radially inwards from the outward expansion part 623. As shown in the figure, the two ends of the outward expansion part 623 extend radially inward to the first end 6224 and the second end 6225, and the outward expansion part is offset axially toward the first end 6224 and may even extend past the first end, thereby forming the concave area 62241 mentioned above.

Another embodiment of the present disclosure further provides a balloon device for delivering a prosthetic implant. The balloon device 641 includes a catheter body 6411, a balloon body 642, and a limiting mechanism 62. The catheter body, balloon body, and limiting mechanism described in this embodiment can be combined with other embodiments.

As shown in FIGS. 86 and 90, specifically, the limiting mechanism 62 includes a limiting body, which includes a plurality of rods 6221 that generally form a cage-like structure. One part of the cage-like structure is an outward expansion part 623, which has the largest radial expansion relative to other parts of the cage-like structure. As shown in FIG. 86, different parts of the rods 6221a and 6221b have different expansions and are distanced from the central axis of the limiting body. The part of the rod where the distance from the central axis is the largest corresponds to the outward expansion part.

Overall, the above-mentioned main hollow area 6222 is formed between the rods 6221a and 6221b. In the cross section through the outward expansion part, as shown in FIG. 97, the rods 6221 are arranged at intervals in the circumferential direction of the limiting mechanism (such as the rods 6221a and 6221b in the figure), and the region enclosed by the center points of these rods approximates a polygonal shape.

During the loading process of the prosthetic implant, different parts of the limiting body in the circumferential direction have slightly different resistance to radial constraints. The rods can self-adjust in the circumferential direction, that is, they can adapt to the loading process via local deformation, preventing overall deformation of the cage-like structure. In addition, the gaps (corresponding to the main hollow areas mentioned above) between adjacent vertices of the polygon can accommodate the folded portions of the balloon body, so that the balloon body can be folded in an orderly manner, reducing the radial size after loading.

The polygon surrounded by the center points of the rods is a regular polygon, and the number of sides, depending on the number of rods in this part, can be in the range of 4 to 12, and preferably in the range of 6 to 8. This ensures the circumferential strength while taking into account better deformation adaptability.

In the axial direction of the limiting mechanism, one end of the limiting body is a first end 6224 facing the prosthetic implant 65a in the use state, and the other end is an opposite second end 6225. Both ends of the limiting body are converged toward the central axis of the limiting body. Compared with the limiting body with one end open in the prior art, the cage-like structure with both ends converged can provide better radial support force.

In some embodiments, the cage-like structure 624 is formed by a plurality of rods. The rods 6221 can extend on a spherical surface or an ellipsoidal surface, so that the cage-like structure 624 generally forms a sphere or an ellipsoid. The cross-section of the ellipsoid is an ellipsoidal surface, and the major axis of the ellipsoidal surface is in the same direction as the axial direction. As shown in FIG. 86, the circumferentially spaced rods extend on a sphere or ellipsoid (the rods rotate around the axis of the limiting body to form a continuous sphere or ellipsoid, and the radial dimensions of the outward expansion part mentioned above can also be measured with reference to the rotating body constructed in this way). In a radial cross-section, the connection of the center points of the rods still forms a polygon.

In some embodiments, as shown in FIGS. 93 and 95, the cage-like structure is formed by a plurality of rods, and the rods extend on a cone surface 6241. This can be understood as the rods rotating around the axis of the limiting body to form a cone. The cone includes a first cone 62411 and a second cone 62412. The first cone and the second cone can be located on two sides of the outward expansion part respectively, or on the same side of the outward expansion part (the two cones are respectively located on two sides of the outward expansion part in FIG. 93, and the two cones are located on the same side of the outward expansion part in FIG. 95). The first cone and the second cone can have the same taper or different tapers.

When the first cone and the second cone have different tapers, it can be understood that they gradually converge in shape from the outward expansion part, but with different tapering trends. The intersection of the first and second cones marks the position of the outward expansion part (as shown in FIG. 93).

Referring to FIGS. 94 to 99, when the prosthetic implant 65a is loaded on the balloon device, the limiting body is in use. In the axial direction of the limiting body, one end of the limiting body is a first end 6224 facing the prosthetic implant, and the other end is an opposite second end 6225. The first end 6224 is connected to the first cone 62411, and the first cone has a steeper tapering trend.

The deflection direction and radial tapering trend of the two cones affect the overall shape of the cage-like structure. For example, in FIG. 99, the second cone 62412 is deflected in the axial direction toward the second end 6225 and gradually tapers radially, while the first cone 62411 is deflected toward the first end (i.e., in the direction away from the second end) and tapers radially at a steeper rate. For example, in FIG. 96, the second cone 62412 is deflected in the axial direction toward the second end 6225 and gradually tapers radially, while the first cone 62411 is also deflected toward the second end and tapers radially at a steeper rate. For example, the second cone 62412 is deflected in the axial direction toward the second end 6225 and gradually tapers radially, while the first cone 62411 tapers radially.

As shown in FIG. 96, the deflection angle A of the first cone 62411 relative to the radial direction of the limiting body is less than 45 degrees, preferably less than 30 degrees. The deflection angle B of the second cone 62412 relative to the radial direction of the limiting body is greater than 60 degrees.

The first cone and the second cone intersect at the outward expansion part, and the outward expansion part represents the maximum radial dimension of both cones. The included angle C of the two cones at the intersection is in the range of 20 to 120 degrees.

Regarding the connection structure between the coupling member and the delivery system, as shown in FIGS. 127 and 101, the balloon device 641 of the delivery system includes a catheter body 6411, a balloon body 642 and an inner shaft 643. The inner shaft extends through the catheter body and the balloon body. The limiting mechanism further includes a coupling member connected to the inner shaft.

The limiting mechanism 62 is installed inside the balloon body 642 and fitted over the inner shaft 643. The coupling member 621 is a radially compressible tubular structure. "Compressible" means that the coupling member can be radially compressed during the assembly process, but its shape remains fixed in the use state.

For example, the inner diameter of the coupling member 621 before assembly may be larger than the outer diameter of the catheter body. During assembly, the coupling member 621 is radially compressed to tighten around and secure itself to the catheter body.

To achieve radial deformability, the coupling member 621 can adopt a mesh structure or a wave structure. As shown in FIG. 101, the coupling member 621 features an axially undulating wave structure with opposite peaks 6213 and valleys 6214. The valleys are arranged at intervals in the circumferential direction of the coupling member, and the peaks are connected to the limiting body. The wave structure facilitates radial deformation of the coupling member during assembly, and the peaks 6213 arranged at intervals further reduce the mutual traction of the rods.

Regarding the distribution of the rods, the cage-like structure 624 is formed by a plurality of rods 6221, and all the rods define the side wall of the cage-like structure. To ensure the necessary support strength, it is preferred that no rod extends in isolation between the two coupling members. In other words, each individual rod must intersect with at least one adjacent rod during its extension. These intersections between adjacent rods serve as fixed junction points. Compared with the existing limiting mechanisms formed by braided metal wires, although there are also intersection points between the metal wires, the two metal wires at the intersection point can move relative to each other. In the present disclosure, the intersection points of adjacent rods are all fixed nodes, which improves the radial support strength.

As shown in FIG. 95, all the rods extend from the first coupling member 6211 to the second coupling member 6212, and on their extension paths, at least one branch 62211 is present. Alternatively, as shown in FIG. 93, the rods extend from the second coupling member 6212 to the first coupling member 6211, and adjacent rods intersect with each other in the circumferential direction to form a junction point 62212.

In the circumferential direction of the outward expansion part, multiple junction points 62212 are spaced apart on the cage-like structure 624. These multiple branches or intersections increase the contact area between the outward expansion part and the balloon body, allowing the balloon body to expand in the circumferential direction to an ideal shape while preventing insufficient stiffness of the limiting mechanism and easy damage to the balloon body caused by sharp rods.

For wider rods, their stiffness can be adjusted through slits. As shown in FIG. 93, the slits 6226 in the cage-like structure do not expand significantly and are therefore not considered as hollow areas.

Regarding the distribution of the main hollow areas, referring to FIGS. 86 and 102, the number of main hollow areas is in the range of 4 to 12, and preferably, in the range of 6 to 8. The main hollow areas have the same shape and are evenly arranged in the circumferential direction. Preferably, the main hollow areas are elongated.

Referring to FIG. 103, in the axial direction of the limiting body, the projection length of the main hollow area on the axis is L1, and the projection length of the limiting body on the axis is L2. L1 accounts for at least 40% of L2, or even at least 60% or more. Preferably, the projection length L1 of the main hollow area is between 75% and 100% of the total projection length of the limiting body. In the axial direction of the limiting body, the main hollow area can extend toward two sides of the outward expansion part, with the extended length being at least 20% of the total length of the limiting body.

When the main hollow area is projected radially onto the plane where the axis is located, the outward expansion part is the widest part of the projection area. In order to increase the contact area between the outward expansion part and the balloon body, the number of main hollow areas is in the range of 6 to 9.

In some embodiments, the hollowed-out areas of the cage-like structure may include main hollow areas 6222 and corresponding auxiliary hollow areas 6223, wherein the auxiliary hollow areas avoid the outward expansion part 623. For example, two rods can form an auxiliary hollow area 6223 by bifurcating and then intersecting on their extension paths. From the perspective of circumferential span of the main hollow area and the auxiliary hollow area, the maximum circumferential span S1 of the main hollow area corresponds to the location of the outward expansion part, and the maximum circumferential span S2 of the auxiliary hollow area 6223 avoids the outward expansion part and is smaller than the span S1 of the main hollow area. The limiting mechanism can be formed in one piece by cutting a tube. When doing so, the slit in the auxiliary hollow area 6223 may extend to the coupling portion. Referring to FIGS. 102 and 104, the slit extending to the coupling portion is not expanded, so the maximum circumferential span of the auxiliary hollow area is smaller than that of the main hollow area.

When cutting a tube, as shown in FIG. 104, the tube has an initial outer diameter D1, while the outer diameter D2 of the coupling member after assembly is smaller than D1. The tube with a cage-like structure of an outer diameter D1 can provide a longer circumferential distance, allowing for greater flexibility in configuring the number of rods (which can be counted circumferentially along a certain section of the limiting body) and achieving greater rod width and strength. Additionally, it helps prevent the rod width from being too thin, which could easily scratch the balloon.

Referring to FIGS. 105 to 106, an embodiment of the present disclosure further provides a balloon device for delivering a prosthetic implant, which has a distal end and an opposite proximal end. The balloon device 641 includes an inner shaft 643, a balloon body 642, a catheter body 6411, and a limiting mechanism 62. The catheter body, balloon body, and inner shaft mentioned in this embodiment can refer to the previous embodiments.

The limiting mechanism includes a coupling member 621, a limiting body 622, and a guide tube 625. The coupling member is connected to the inner shaft, and the coupling member is also connected to at least one of the limiting body and the guide tube. The coupling member and the limiting body mentioned below can refer to the previous embodiments.

The limiting body is generally a cage-like structure. The cage-like structure can be formed by a plurality of rods extending on a spherical surface or a cone surface. The limiting body includes a plurality of rods arranged at intervals in the circumferential direction, with main hollow areas formed between adjacent rods. The structure of the cage-like structure, the rods and the hollow areas can also refer to the previous embodiments.

The guide tube 625 is connected to the proximal side of the limiting body, and the guide tube 625 and the limiting body are formed in one piece by cutting a tube made of a shape memory alloy material (such as Nitinol alloy). For example, the tube has an initial outer diameter D1, and is cut to form the guide tube and the limiting body having a plurality of rods. The outer diameter of the guide tube is the outer diameter D1 of the tube. The plurality of rods can be made into a cage-like structure using molds and other preforming processes. Due to the shape memory alloy's properties, the limiting body can revert to a tubular shape during the assembly process.

The coupling member has various distribution methods. As shown in FIG. 105, the coupling member 621a can be disposed inside the guide tube and connected to the inner wall of the guide tube; or the coupling member 621b can be disposed at the proximal end of the guide tube; or the coupling member 621d can be disposed at the distal end of the limiting body; or the coupling member 621c can be disposed inside the limiting body. These distribution methods can be used alone or in combination.

As shown in FIG. 106, there are two coupling members: a first coupling member 6211 and a second coupling member 6212, which are spaced apart in the axial direction and respectively connect to the limiting body and the guide tube. Both coupling members are located on the outer periphery of the inner shaft, and at least one coupling member is fixed to the inner shaft. The distal part of the balloon body also wraps the second coupling member.

Referring to FIGS. 107 to 108, the radial gap between the inner shaft 643 and the catheter body 6411 is a fluid channel in communication with the interior of the balloon body. When the prosthetic implant is deployed via balloon expansion, the fluid in the fluid channel enters the balloon body, with the aid of the above-mentioned guide channel, to inflate the balloon body.

The second coupling member 6212 is located at the proximal end of the guide tube 625 and is adjacent to the connection between the balloon body 642 and the catheter body 6411. The second coupling member 6212 can divert the fluid in the fluid channel. Before the balloon body is inflated, the second coupling member 6212 diverts part of the fluid to the interior of the flow guide tube, and this part of the fluid eventually inflates the first part through the hollow areas of the cage-like structure.

The proximal end of the guide tube 625 has a diameter-reduced section 6252 and is connected to the corresponding coupling member through the diameter-reduced section. This design allows the connection between coupling members with different diameters and the guide tube.

The guide tube has fluid inlets 6251, which are distributed on the diameter-reduced section and/or the circumferential wall of the flow guide tube. Preferably, the circumferential wall of the guide tube is provided with hollowed-out gaps, which can be elongated as shown in FIG. 105, or circular as shown in FIG. 110. When the prosthetic implant is still in the crimped state, the gap between the middle part of the balloon body and the catheter body is effectively utilized to allow the fluid to pass through the gap more rapidly, so that the fluid can simultaneously inflate the first and second parts of the balloon body. In some embodiments, the guide tube is connected to the proximal side of the limiting body. During use, the limiting body limits the distal end of the prosthetic implant. The interior of the guide tube serves as a flow channel for the inflation fluid in the delivery system, playing a role in guiding fluid.

Referring to FIG. 111, in order to further limit the proximal end of the prosthetic implant, there are two limiting bodies, namely a limiting body 622a located at the proximal end of the guide tube and a limiting body 622b located at the distal end of the guide tube.

The limiting body 22a and the limiting body 22b are both cage-like structures, and the two cage-like structures are independent of each other. For example, the two cage-like structures in FIG. 111 are formed by a plurality of rods extending on a spherical surface, and the two cage-like structures in FIG. 112 are formed by a plurality of rods extending on a cone surface; the limiting body 622a in FIG. 113 is formed by a plurality of rods extending on a spherical surface, and the limiting body 22b is formed by a plurality of rods extending on a cone surface; the first cones 62411 of the two cage-like structures in FIG. 114 have different deflection tendencies (i.e., deflection angle) relative to the radial direction of the corresponding limiting body.

The interventional delivery system includes a control handle 63 and a catheter assembly 64 controllably connected to the control handle 63. The prosthetic implant 65a is loaded onto the catheter assembly 64. In an interventional delivery system based on balloon expansion, the catheter assembly 64 can include a balloon device 641 and an outer sheath 644 that is slidably fitted over the balloon device.

The balloon device 641 includes a balloon body 642, which includes a first part 6421, a middle part 6422 and a second part 6423 from the distal end to the proximal end, and the middle part 6422 is used for loading and fixing the prosthetic implant 65a.

The limiting mechanism is generally disposed in the balloon body 642. As shown in FIG. 107, the limiting body is located in the first part 6421. The guide tube 625 is fitted over the inner shaft, and extends from the proximal side of the limiting body to the middle part and the second part of the balloon body. A guide channel for fluid to pass through is defined between the guide tube 625 and the inner shaft 643. Preferably, the length of the guide tube ranges from 36 mm to 50 mm, and the inner diameter ranges from 1.6 mm to 2.3 mm. The proximal end of the guide tube is fixed relative to the inner shaft, and the improved fixing method of the two will be provided below.

The limiting mechanism of the present disclosure has a simple structure and can be formed in one piece by cutting a tube. The specific structures of the coupling member and the limiting body can refer to the previous embodiments. For example, as shown in FIG. 115, the tube has an initial outer diameter D1, which is cut to form two wave structures, a plurality of rods, and a guide tube with hollowed-out gaps, corresponding to the two coupling members, the limiting body, and the guide tube respectively. The outer diameter of the guide tube is the outer diameter D1 of the tube. The plurality of rods can be made into a cage-like structure using molds and other forming processes. At least one coupling member undergoes radial compression before use, resulting in an outer diameter D2 smaller than D1.

When the limiting mechanism is not provided on the delivery system, the prosthetic implant is crimped on the catheter (such as the balloon body) of the delivery system. Since the outer diameter of the prosthetic implant in the crimped state is larger than the outer diameter of the catheter, steps will be formed between the proximal and distal ends of the prosthetic implant and the catheter, posing a certain risk. The limiting body of the limiting mechanism can fill the step, especially the distal step, which is beneficial for implantation. For example, when the prosthetic implant is a prosthetic heart valve, this design is beneficial for trans-valve procedures. In addition, the circumferential strength and blocking effect of the limiting body (the outward expansion part forms a blocking step for the prosthetic heart valve) are related to the wall thickness of the tube, and the wall thickness of the tube is selected within the range of 0.1 mm to 0.25 mm.

Referring to FIGS. 107 to 116, an embodiment of the present disclosure further provides a balloon device 641 for delivering a prosthetic implant, including an inner shaft 643, a catheter body 6411, and a balloon body 642. The balloon body 642 includes a first part 6421, a middle part 6422, and a second part 6423 from the distal end to the proximal end, and the middle part 6422 is used for loading and fixing the prosthetic implant 65a. The inner shaft 643 is provided with a limiting mechanism 62 as described in the previous embodiments inside the balloon body, and the limiting mechanism includes coupling members 621, a limiting body 622, and a guide tube 625. The structures of the coupling member, limiting body, and guide tube mentioned below can refer to other embodiments.

The coupling members include a first coupling member 6211 (located at the proximal end) and a second coupling member 6212 (located at the distal end) spaced apart in the axial direction. The two coupling members are fitted over the inner shaft 643. The coupling members can be bonded to the inner shaft 643. Alternatively, as shown in FIG. 116, a sleeve 62121 can be fitted over the coupling member, to facilitate using a tool 66 to push the blocking member into the balloon body.

The limiting body is used to fill the first part 6421 of the balloon body 642 without interfering with the loading area in the middle of the balloon body, and it provides a guide angle for the distal end of the balloon body. The limiting body includes a plurality of rods arranged at intervals in the circumferential direction, with main hollow areas formed between adjacent outward expansion parts. The structure of the rods and the hollow areas can refer to the previous embodiments.

The balloon body 642 has a folded state and an inflated state. As shown in FIG. 97, in the folded state, the balloon body 642 has a plurality of folded portions 6424. The folded portions 6424 are embedded in the cage-like structure, i.e., in the corresponding main hollow areas. The outer periphery of the outward expansion part is only covered with a layer of balloon body material. Without the main hollow areas, the folded portions 6424 may form a three-layer covering, resulting in an increase in the size of the outward expansion part of the limiting body, which is not conducive to loading. The guide tube 625 runs through the loading area in the middle of the balloon body, and the guide tube 625 may be provided with fluid inlets 6251 on the circumferential wall, so that when the balloon body is inflated, the first part 6421 and the second part 6423 can be inflated at the same time.

The limiting mechanism of this embodiment is formed in one piece by cutting a tube. The tube can be made of materials such as memory alloy, and is pre-formed into a cage-like structure before being assembled into the balloon body. During assembly, the blocking member can be straightened to facilitate assembly.

The assembly process of the balloon device is as follows:
1. first fixing the first coupling member 6211 on the inner shaft 643 by gluing, and straightening the limiting mechanism to form a straight-tube shape;
2. pushing the limiting mechanism 62 and the inner shaft 643 into the balloon body 642 from the distal tubular end of the balloon body, the limiting body returning to the preset cage-like structure; optionally, applying axial pushing to shape the limiting body, as shown in FIG. 116, where a tool 66 is used to apply force to the limiting mechanism for pushing or compressing; and
3. after pushing the limiting mechanism 62 and the inner shaft 643 into the balloon body, fixing the second coupling member 6212 on the inner shaft 643 by bonding, or welding the second coupling member 6212 to the distal tubular end of the balloon body, or adding a sleeve 62121 to the second coupling member 6212 to fix it to the distal tubular end of the balloon body.

Referring to FIG. 117, the present disclosure further provides a transcatheter implant system, including a balloon device 641 and a prosthetic implant 65a. The balloon device 641 includes a limiting mechanism 62, a catheter body 6411 for delivering fluid, and a balloon body 642 in communication with the catheter body. In the loaded state, the prosthetic implant 65a is radially compressed and installed on the balloon body. The prosthetic implant 65a is blocked by the limiting mechanism in the axial direction, and at least the distal end of the prosthetic implant 65a is blocked by the limiting mechanism. The catheter body, the balloon body, and the limiting mechanism can refer to the previous embodiments.

The balloon body 642 has a folded state and an inflated state. When the prosthetic implant is delivered during intervention, the balloon body is in the folded state. The operator injects fluid, i.e., an inflation medium (e.g., saline), into the balloon body 642 to inflate the balloon body 642, so as to expand and deploy the prosthetic implant 65a. During the deployment of the prosthetic implant, i.e., during the inflation of the balloon body, the radial gap between the catheter body 6411 and the inner shaft 643 is a fluid channel in communication with the interior of the balloon body. A guide channel for the fluid to pass through is defined between the guide tube 625 and the inner shaft 643. The guide tube 625 can divert the fluid in the fluid channel, and inflate the first and second parts of the balloon body at the same time through the guide channel. When the prosthetic implant is completely deployed, the balloon body is in the inflated state. During the delivery process, the limiting body is used to fill the first part of the balloon body 642. The side of the limiting body facing the first end blocks the distal end of the prosthetic implant 5a, so as to position the prosthetic implant during the inflation process. The side of the limiting body facing the second end plays a role in providing a guide angle for the distal end of the balloon body, making the interventional delivery of the prosthetic implant in the human body smoother. To achieve effective guidance, the radial dimension of the limiting body should be larger than the radial dimension of the prosthetic implant crimped on the balloon body but not excessively large to avoid assembly difficulties. Preferably, the radial dimension of the limiting body ranges from 7.5 mm to 9.5 mm.

During the delivery process, a blocking member 67 can be provided outside the proximal end of the balloon body to further limit the proximal end of the prosthetic implant. Alternatively, a conventional locking wire structure in the art can be used to further limit the proximal end of the prosthetic implant.

The transcatheter implant system according to the above-mentioned embodiment further includes a control handle 63 and an outer sheath 644. The proximal end of the control handle has an interface 631 (as shown in FIG. 127) in communication with the catheter body for injecting fluid.

The outer sheath 644 is slidably fitted on the outer periphery of the balloon device. The proximal end of the outer sheath is connected to the control handle. The outer sheath and the balloon body are movable relative to each other, allowing the outer sheath to either cover or expose the prosthetic implant. During the interventional delivery of the prosthetic implant, the outer sheath 644 is fitted over the balloon device and the prosthetic implant, providing protection. After the prosthetic implant is delivered to the intended position, the outer sheath 644 can be removed by operating the control handle.

During the implantation process, the limiting body includes a loaded state, an intermediate state, and an expanded state. In the loaded state, the limiting body is located inside the balloon body and the outer sheath, and is subjected to radial forces from both the balloon body and the outer sheath. The outer sheath here can be an outer sheath 644 (i.e., a catheter sheath), or a device independent of the delivery system, such as a catheter sheath or an outer sheath of a sheath protector.

In the intermediate state, the limiting body is released from the radial constraint of the outer sheath and is only subjected to the radial force of the balloon body. In this state, the limiting body expands slightly compared to the loaded state but does not fully expand due to the radial force of the balloon body, so it is called the intermediate state.

When the delivery system and the prosthetic implant reach the appropriate position in the human body, the fluid can be injected into the balloon body to inflate it. When the balloon is inflated to the point where it no longer exerts a radial force on the limiting body, the limiting body enters the expanded state in which the limiting body is fully expanded.

Some embodiments of the present disclosure improve the structure of the prosthetic heart valve to increase the compatibility of the prosthetic heart valve with the interventional delivery system and enhance the control effect.

Referring to FIGS. 118 to 127, some embodiments of the present disclosure provide a prosthetic heart valve 65, which can be deployed by means of balloon expansion or self-expansion. Specifically, the prosthetic heart valve includes a first stent 651 and a plurality of leaflets 652. The first stent is a radially deformable cylindrical structure. The interior of the first stent 651 is a blood flow channel. The leaflets 652 cooperate with each other to control the blood flow channel. As shown in FIG. 118, the fixed edges 6521 of the leaflets are sewn and fixed to the first stent, and the free edges 6522 of the leaflets cooperate with each other to control the blood flow channel. The leaflets can be made of biological materials (such as porcine pericardium or bovine pericardium) or synthetic materials.

The first stent can be in the shape of a hollow straight cylinder, with the side wall of the hollow straight cylinder forming struts 6511 by carving. A plurality of cells 6512 are defined by the struts. The cell can be diamond-shaped, hexagonal, fishbone-shaped, etc., and the intersection of adjacent struts is a node 65111.

The first stent has an axial direction (the axis direction, such as the X direction shown in FIG. 119), a radial direction perpendicular to the axial direction, and a circumferential direction around the axis. The first stent has a radially expanded state (as shown in FIG. 119) and a compressed state (as shown in FIG. 120). During the radial compression process, the cells and the struts undergo corresponding deformation, allowing the first stent to be loaded into a transcatheter implantation system in the compressed state.

In the axial direction of the first stent, taking the prosthetic heart valve 65 as an aortic valve or a pulmonary valve example, depending on the normal blood flow direction in the human body, one side is the inflow side 6513 and the other side is the outflow side 6514. The first stent has ends on both sides, which correspond to the outermost nodes 65111. For example, as shown in FIG. 119, the projection point P of the node 65111a is the projection of the end on the outflow side of the first stent. Similarly, the end on the inflow side corresponds to the projection point Q of the node 65111b.

The inflow side and/or outflow side of the first stent has an eyelet structure 653 protruding from the corresponding end. Taking the outflow side as an example, as shown in FIG. 120, in the compressed state, the highest point N of the projection of the eyelet structure 653a protrudes from the end (projection point P) by a distance of L1. As shown in FIG. 119, in the expanded state, the highest point M of the projection of the eyelet structure 653 protrudes from the end (projection point P) by a distance of L2, and L1 > L2 ≥ 0. It can be understood that in the expanded state, through the deformation and expansion of the struts, the eyelet structure is basically flush with the surrounding nodes, resulting in a smooth end of the first stent. In addition, the eyelet structure in this embodiment is not a fully enclosed hole. As shown in FIG. 119, the eyelet structure 653a is generally arc-shaped, with an opening facing the inflow side. As the first stent transitions from expanded state to compressed state (as shown in FIG. 120), the opening contracts. When the first stent undergoes significant compression, the sides of the opening may come into contact, forming an almost closed circular structure (for example, see the eyelet structure 653 in FIG. 120). In the compressed state shown in FIG. 120, the eyelet structure 653 is significantly raised compared to the surrounding nodes.

In one embodiment, L1 : L2 is approximately in the range of 1.2 - 1.6 : 1. Specifically, L1 may be in the range of 0.6 - 0.8 mm, preferably 0.7 mm; L2 may be in the range of 0.4 - 0.6 mm, preferably 0.5 mm.

The prosthetic heart valve is usually implanted using a delivery system. When a balloon expansion-based interventional delivery system is used, the first stent is installed on the balloon body 642 in a radially compressed state. The protruding eyelet structure facilitates the connection and control of the locking wire structure of the delivery system, making it easier to pass the locking wire structure through the eyelet structure and axially position the prosthetic heart valve on the delivery system. When the first stent is deformed and expanded, the eyelet structure is also deformed and expanded and basically aligns with the end of the first stent, preventing the risk of the protruding eyelet structure puncturing blood vessels after implantation of the prosthetic heart valve.

The prosthetic heart valve of this embodiment can be a prosthetic pulmonary valve. Preferably, it is deployed by means of balloon expansion. In the compressed state, the eyelet structure 653a is more prominent, which facilitates axial positioning with the balloon body, while in the expanded state, the protrusion is reduced, minimizing risks.

In some embodiments, the first stent has a mesh structure formed by struts, and the eyelet structure is defined by the struts at the end. For example, in FIG. 121, the cell 6512c is formed by struts 6511a, 6511b, 6511c, 6511d, 6511e, and 6511f at the end, and the eyelet structure 653a is defined by the struts 6511a and 6511b. Preferably, the struts can be bent into an arc shape to outlet the edge of the eyelet structure 653a.

Both axial ends of the first stent have eyelet structures. As shown in FIG. 119, an eyelet structure 653a is provided on the outflow side and an eyelet structure 653b is provided on the inflow side. The eyelet structures at both ends can be aligned circumferentially (as shown in FIG. 119, where eyelet structures 653a and 653b are arranged opposite to each other) or staggered.

In some embodiments, the first stent 651 is in the shape of a straight cylinder and includes multiple rows of cells in the axial direction. The shape of the cell is roughly rhombus or rhombus-like. As shown in FIG. 122, the cell with the eyelet structure 653 is the first cell 654, and the cell circumferentially adjacent to the first cell is the second cell 655. In the first cell 654, the struts around the eyelet structure include:
an arc segment 65112, defining the edge of the eyelet structure; and
connecting segments 65113, extending from two ends of the arc segment 65112 in opposite directions to the nodes on both circumferential sides of the first cell 654 (i.e., the node 65111b and the node 65111c in the figure), respectively.

As shown in FIG. 123, the angle between the connecting segments 65113a and 65113b on both sides of the arc segment is A1, and the inner angle of the node 65111a at the axial end of the second cell is A2. In the expanded state, A1 is greater than A2 to adapt to the fact that the end of the eyelet structure has a greater axial convergence trend, which is conducive to the further axial protrusion of the eyelet structure in the compressed state. The central angle C corresponding to the arc segment is in the range of 150° - 210°.

Referring to FIG. 124, the span of the arc segment 65112 in the circumferential direction of the first stent is L1, and the span of the first cell in the circumferential direction is L2, and L1 accounts for 1/4 to 1/2 of L2, for example, about 1/3.

In some embodiments, in the first cell 654, the strength of the struts around the eyelet structure is less than that of other struts of the first cell, making the eyelet structure more deformable. Specifically, for example, in the first cell 654 as shown in FIG. 123, the strength of the connecting segments 65113c and 65113d is greater than the strength of the connecting segments 65113a and 65113b. Strength reduction of the struts can be achieved by reducing the amount of metal, thickness, etc., allowing a larger angle A1 between the connecting segments 65113a and 65113b after expansion, making the eyelet structure more flexible.

The first stent includes multiple rows of cells, for example, 3 to 6 rows. For example, there are 5 rows of cells in FIG. 122. The two axially adjacent rows are staggered in the circumferential direction and share some struts. In the expanded state of the first stent, the inner angle of the node of each cell is in the range of 75° to 105°. For example, when the inner angle is 90°, the cell is basically a square.

Referring to FIG. 125, the present disclosure further provides a prosthetic heart valve, including a first stent 651, leaflets 652, a skirt 657, and a sealing member 659 to enhance the anti-paravalvular effect. The first stent 651 and leaflets 652 can refer to other embodiments.

The skirt 657 is connected to and surrounded by the inner wall of the first stent, and one axial side of the skirt is connected to the leaflets 652. The skirt can be sewn or bonded to the stent and the leaflets, in order to close the blood flow channel.

The sealing member 659 is fixed to the radial outer side of the skirt 657 and protrudes from the radial inner side of the first stent to the radial outer side of the first stent through the corresponding cells. The sealing member is made of foam material, providing adaptive sealing to reduce paravalvular leakage.

In one embodiment, the skirt and the sealing member are formed in one piece or adhesively fixed together. For example, the skirt may be made of PET material, and the sealing member may be made of PU foam material. For another example, the skirt and the sealing member are both made of PU material.

On the one hand, the PU skirt offers advantages such as high elongation and thin thickness, which help prevent the risk of tearing. Due to its high elongation, the PU skirt can adapt to various cell shapes and sizes, including smaller diamond-shaped cells with less deformation and larger fishbone-shaped cells with greater deformation.

On the other hand, the PU sealing member is compressible and self-expandable. When subjected to uneven external forces, the PU sealing member can automatically adjust the foam height and thickness to closely fit the valve annulus, thereby preventing paravalvular leakage. Additionally, the PU sealing member does not significantly increase the size of the delivery system.

When the sealing member and the skirt are made of the same PU material (when processed separately, it is not required that the molecular weight or hardness ratio is strictly the same), they can be bonded or integrally processed to achieve high peel strength. This ensures that even if the sealing member is exposed and comes into contact with the catheter assembly of the interventional delivery system or body tissues, it is not easy to detach, thereby avoiding safety risks.

As shown in FIG. 125, the sealing member 659 may include a plurality of sealing blocks arranged in the circumferential direction of the first stent, and the sealing blocks are made of foam material and embedded in corresponding cells.

In another embodiment, the skirt may also be made of biological pericardial material, such as porcine pericardium or bovine pericardium. To facilitate the installation of the plurality of sealing blocks, the sealing member 659 further includes an inner lining film 658. The inner lining film and the sealing blocks may be made of the same material to facilitate bonding or integral processing of the two. As shown in FIG. 126, the sealing blocks and the inner lining film are formed in one piece. When the prosthetic heart valve is in use, the inner lining film 658 is located on the inner side of the first stent and sewn to the skirt, while the sealing blocks are fixed on the inner lining film and embedded in the corresponding cells, protruding outward. The inner lining film is designed to be very thin, minimizing the impact on the diameter of the crimped prosthetic implant.

In some embodiments, the transcatheter implant system can limit the axial movement of the prosthetic heart valve by threading the locking wire structure 68 connected to the balloon device through the eyelet structures, that is, limit the proximal and distal ends of the first stent. The locking wire structure 68 can include an adjustment wire and a locking wire, and has a locked state for limiting the first stent and an unlocked state for releasing the first stent. The details will be described later.

The present disclosure further provides an embodiment of a transcatheter implant system. For example, FIG. 127 shows a transcatheter implant system 61, including a prosthetic heart valve 65 and an interventional delivery system for delivering and expanding the prosthetic heart valve. The interventional delivery system includes a control handle 63, and a catheter assembly 64 controllably connected to the control handle 63. The prosthetic heart valve 65 is loaded on the catheter assembly 64. The catheter assembly includes a plurality of controlled components, and the distal ends of the controlled components cooperate with each other to manipulate the prosthetic heart valve, such as releasing, retrieving, locking in position, bending, and adjusting spatial orientation. The controlled components can include a hollow tube, a solid rod, a flexible wire, or a combination of various forms. There are a plurality of controlled components, and at least two of them (taking the proximal end as an example) can slide relative to each other in the axial direction or rotate relative to each other around the axial direction. The force-applying components (that users directly operate and touch) on the control handle used to operate the controlled components are either directly fixed to the corresponding controlled components for transmission or use mechanisms such as threads or gear racks or the like for transmission. The prosthetic heart valve may be a prosthetic aortic valve, a prosthetic pulmonary valve, a prosthetic mitral valve, a prosthetic tricuspid valve, etc., and has a loaded state (i.e., a compressed state) loaded on the catheter assembly 64 and an expanded state.

The transcatheter implant system 61 has a proximal end 6101 and an opposite distal end 6102. When indicating direction, the term "proximal" generally refers to the side close to the operator (e.g., a physician), while "distal" refers to the side far away from the operator. Along the interventional path, each component has its distal and proximal ends. Theoretically, when the catheter assembly and the control handle are fully straightened, the proximal and distal ends form a straight line, defining the axial direction, as well as the radial direction perpendicular to it and the circumferential direction around it. When used to refer to a structure, the term "end" in this disclosure refers to the endpoint of the structure, or a point or area on that side, or a specific structure connected to that point or area. Unless otherwise specified, the axial direction mentioned in the embodiments above and below refers to the direction along the straight line between the proximal and distal ends that is theoretically determined when the catheter assembly and the control handle are completely straightened. Correspondingly, the radial direction perpendicular to the axial direction and the circumferential direction around the axial direction can be determined.

The catheter assembly 64 generally includes a balloon device 641. An outer sheath 644 that is slidably fitted over the balloon device can be configured as needed. As shown in FIG. 127, the outer sheath 644 slides proximally and exposes the balloon device. The balloon device 641 mainly includes a catheter body 6411 and a balloon body 642. The extension direction of the catheter body is the axial direction. When the catheter body 6411 is straightened, a radial direction perpendicular to the axial direction and a circumferential direction around the axial direction are also determined. The balloon body is in communication with the catheter body, and can receive fluid from the catheter body. The balloon body has a folded state and an inflated state under the action of the fluid. As shown in FIG. 127, in the folded state, the balloon body is connected to the distal end of the catheter body, and the prosthetic heart valve 65 is crimped on the outer surface of the balloon body. As shown in FIG. 128, the proximal end of the catheter body 6411 is provided with a fluid inlet 645, and in the inflated state, the fluid can enter the balloon body from the fluid inlet 645 and inflate the balloon body. The prosthetic heart valve is expanded from the radially compressed state to the radially expanded state based on the change of the balloon body, thereby achieving deployment of the prosthetic heart valve 65 based on balloon expansion. In some cases, the distal end and side wall of the balloon body can also be provided with fluid inlets, which is not shown in the figure.

The transcatheter implant system may further include a limiting mechanism 62 and a locking wire structure 68 for limiting the movement of the prosthetic heart valve. For example, the locking wire structure is arranged outside the balloon device, and the limiting mechanism 62 can be arranged inside or outside the balloon device, both of which can limit the axial movement of the prosthetic heart valve 65. Some of the following embodiments provide improvements for the limiting mechanism and the locking wire structure.

Referring to FIGS. 129 to 131, in the existing prosthetic heart valve 7200, the top side of the leaflet 7100 is a free edge 7120, the bottom side is a fixed edge 7130, and the intersection of the free edge 7120 and the fixed edge 7130 is a commissure tab 7140. During assembly, the commissure tabs 7140 of the two adjacent leaflets are first sutured together with sutures, and then connected to the stent 7210. However, the tightened sutures during suturing may cause a certain deformation of the free edges, potentially leading to poor closure near the stent, thereby compromising the sealing of the blood flow channel. Referring to FIGS. 132 to 133, an embodiment of the present disclosure provides a leaflet 7100 for a prosthetic heart valve, and a prosthetic heart valve with the leaflet. Depending on the processing method or storage conditions, the prosthetic heart valve of the embodiments of the present disclosure can be a wet valve stored in a solution or a dry valve that has undergone dehydration treatment.

The leaflet 7100 includes a leaflet body 7110, which has a transverse direction and a longitudinal direction in a flattened state. The outer edge of the leaflet body 7110 includes a free edge 7120 and a fixed edge 7130 distributed on two opposite sides in the longitudinal direction. The free edge 7120 is used to control the blood flow channel in the prosthetic heart valve by deformation. The commissure tabs 7140 are arranged on two opposite sides in the transverse direction of the leaflet body 7110. The free edge 7120 and the fixed edge 7130 meet at the commissure tab 7140 on the corresponding side. The junction of the commissure tab 7140 with the free edge 7120 is provided with an outward-protruding portion 7150 extending longitudinally relative to the free edge 7120. The transverse direction and the longitudinal direction referred to in this disclosure can refer to the X direction and the Y direction in FIG. 132. Specifically, the X direction is the transverse direction and the Y direction is the longitudinal direction. The leaflet 7100 is generally symmetrical, and the following description and some of the figures use one side as an example, with the other side being similar.

During suturing, the commissure tabs 7140 of the two leaflets 7100 are folded and aligned for stitching, and finally the sutures are tightened and knotted for fixation, wherein the suture knots are specifically placed on the outward-protruding portions 7150. When the sutures are tightened and knotted, the outward-protruding portions 7150 are deformed and absorb stress, which greatly reduces the impact on the free edges 7120. This prevents the free edges 7120 from being noticeably affected by the tightening of the sutures, thereby addressing the issue of improper closure of the blood flow channel caused by the deformation of the free edges 7120 during the suturing of the leaflets 7100. The outward-protruding portion 7150 also increases the suture area, enhancing the structural strength.

In this embodiment, the outward-protruding portion 7150 intersects with the extension line of the fixed edge 7130. The inner side of the extension line basically defines the working area of the leaflet 7100. Positioning the outward-protruding portion 7150 in this manner ensures both adequate suture strength and an appropriate opening size of the leaflet 7100. For example, at least 1/4 of the area of the outward-protruding portion 7150 is located inside the extension line (refer to L in FIG. 132) of the fixed edge 7130. For another example, at least 1/2, or even 3/4 of the area of the outward-protruding portion 7150 is located inside the extension line of the fixed edge 7130.

The outward-protruding portion 7150 is strip-shaped, approximately rectangular, and the length direction of the strip is consistent with the transverse direction of the leaflet body. Therefore, even after being folded into a double-layer structure, it maintains sufficient transverse span to facilitate suturing operations and absorb deformation caused by compression of the knot.

The outward-protruding portion 7150 has an inner edge 7151 and an opposite outer edge 7152. The outer edge 7152 transitions to the top side of the commissure tab 7140, forming a noticeable corner and making the outer edge 7152 roughly parallel to the outer side of the commissure tab 7140.

The outward-protruding portion 7150, after being folded in half, can provide sufficient structural strength around the suture hole 7173 mentioned below. To minimize folding and suturing operations on the commissure tab 7140, the outer side of the commissure tab 7140 exceeds the outer edge 7152 by at least 2 mm in the transverse direction of the leaflet body, for example, by 2.5 to 5 mm. The junction of the commissure tab 7140 with the fixed edge 7130 has an opening area 7131. Since the commissure tab 7140 needs to be folded during suturing, the opening area 7131 can reduce the traction and deformation of the leaflet body 7110 and the commissure tab 7140 during folding, especially release the tensile stress during longitudinal deflection.

Furthermore, the transverse positions of the outward-protruding portion 7150 and the opening area 7131 are aligned. In the flattened state, the outward-protruding portion 7150 is roughly rectangular, the transverse length of the outward-protruding portion 7150 is greater than the transverse length of the opening area 7131, and the opening area 7131 is located below the center line of the rectangle (L1 is the center line as shown in FIG. 133). After folding, the outward-protruding portion 7150 forms a double-layer structure, which further improves the strength. In this embodiment, the transverse length of the outward-protruding portion 7150 is in the range of 1 - 3 mm, and the longitudinal length is in the range of 0.2 mm - 0.8 mm. For another example, the transverse length of the outward-protruding portion 7150 is in the range of 1.08 mm - 2.43 mm, and the longitudinal length is in the range of 0.29 mm - 0.44 mm.

In one embodiment, the commissure tab 7140 has:
a first folded portion 7171 which is bent from the junction of the commissure tab 7140 with the leaflet body 7110 toward the transverse inner side of the leaflet body 7110 and attaches to the leaflet body 7110 to form a joint 7170 of a double-layer structure; and
a second folded portion 7172 which is bent from the end of the first folded portion 7171 toward the transverse outer side of the leaflet body 7110.

The inner side and the outer side referred to above are relative to the leaflet body 7110, the side close to the center line of the leaflet body 7110 is the inner side, and the side away from the center line of the leaflet body 7110 is the outer side. To facilitate suturing with the stent 7210, the joints 7170 of two adjacent leaflets 7100 are aligned and sutured together (the leaflets 7100 can be preassembled into a cylindrical preform by pre-fixing them in pairs during assembly), and are then secured against the radial inner side of the stent 7210 (the joints 7170 can also be sutured to the stent 7210). The second folded portion 7172 of each leaflet 7100 extends to the radial outer side of the stent 7210, wrapping around the corresponding part of the stent 7210 and being sutured in place.

Referring to FIG. 134, the joints 7170 of two adjacent leaflets 7100 are fixed together by one or more first sutures 7174. All the knots 7180 of the first suture 7174 are located at the top side of the outward-protruding portions 7150, which is convenient for the outward-protruding portions 7150 after deformation to absorb the stress and deformation from the knots. Specifically, the first suture 7174 is first passed through the two joints 7170. As can be seen in the figure, the needle penetration point is located at the intersection of the outward-protruding portion 7150 and the extension line of the free edge 7120. Then the first suture 7174 is first knotted to pre-fix the two joints 7170, facilitating subsequent suturing.

After the first suture 7174 passes through all the suture holes 7173, it returns to the top side of the outward-protruding portion 7150 and is knotted for the second time to complete the fixation of the two joints 7170. As can be seen in the figure, the first suture 7174 has two knots 7180, which are arranged side by side on the top sides of the two adjacent outward-protruding portions 7150. At this point, the knots 7180 only interfere with the outward-protruding portions 7150, causing certain deformation of the outward-protruding portions 7150. However, the deformation of the outward-protruding portions 7150 has minimal interference with the free edges 7120, thereby reducing the impact on the free edges 7120.

Referring to FIGS. 135a, 135b, 135c and 136, to facilitate the fixation of the knot 7181, in the circumferential direction of the stent 7210, at least one side of the support bar 7220 is provided with a recessed area 7221, and the knot 7181 of the second suture 7176 is placed in the recessed area 7221. The recessed area 7221 can be formed on the support bar 7220 by structural bending, cutout, or branch structure of the cell. The recessed area 7221 helps secure the knot 7181, minimizing protrusion, and prevents the knot 7181 from sliding or detaching axially.

To reinforce the connection strength between the joints 7170 and the support bar 7220, the second suture 7176 is looped around the joints 7170 and the support bar 7220, with each loop containing one knot 7181. Each loop of the second suture 7176 passes through the joints 7170 of two adjacent leaflets 7100 on the radial inner side of the stent 7210, securing the adjacent joints 7170 tightly together. Each knot 7181 is placed within the corresponding recessed area 7221.

The second folded portions 7172 of two adjacent leaflets 7100 wrap around the support bar 7220 from two opposite sides of the support bar 7220 to improve the connection strength, and the second folded portions 7172 wrap around the knot(s) 7181 of the second suture(s) 7176, making the whole structure more concise and reducing the number of exposed knots. The second folded portions 7172 are connected to the support bar 7220 through the third suture 7178, and in the circumferential direction of the stent 7210, all the knot(s) 7182 of the third suture(s) 7178 are located on the side of the support bar 7220. Preferably, in order to facilitate knotting and positioning, all the knot(s) 7182 of the third suture(s) 7178 are located on the same side of the support bar 7220. In other words, for the same support bar 7220, on two opposite sides in the circumferential direction of the stent 7210, one side defines a first recessed area 7222 for all the knot(s) 7181 of the second suture(s) 7176 to be placed, while the other side defines a second recessed area 7223 for all the knot(s) 7182 of the third suture(s) 7178 to be placed. All the knot(s) 7180 of the second suture(s) 7176 and the third suture(s) 7178 are placed in the corresponding recessed areas. The first recessed area 7222 and the second recessed area 7223 can be arranged on the support bar 7220 symmetrically or staggered.

There are multiple recessed areas on the same side of the support bar 7220 arranged along the extension direction of the support bar 7220. The second sutures 7176 and the third sutures 7178 are respectively looped around the support bar 7220 for multiple times, with each loop containing a knot positioned within the corresponding recessed area. Each loop of the third suture 7178 passes through the joints 7170 and the second folded portions 7172 of two adjacent leaflets 7100 on the radial inner side of the stent 7210. In other words, for the same leaflet 7100, the second folded portion 7172 and the joint 7170 are attached to each other to form a three-layer structure, and the third suture 7178 passes through the three-layer structure. To minimize the number of suture holes, the third suture 7178 can be threaded through the suture holes 7175 of the second suture 7176. The suture holes 7175 are arranged axially, with a total of three holes, all located on the joint 7170. These three holes are specifically designated as suture holes 7175a, 7175b, and 7175c.

Referring to FIGS. 137 to 139, the present disclosure further provides a transcatheter implant system, including a balloon device and a prosthetic heart valve as described in the previous embodiments. The prosthetic heart valve 65 includes a first stent 651 and leaflets 652. Both axial ends of the first stent have eyelet structures, which include an eyelet structure 653a close to the limiting body 622a and an eyelet structure 653b close to the limiting body 622b, and the axial positions of the eyelet structures at both ends are the same.

The balloon device 641 includes:
a catheter body 6411; and
a balloon body 642 in communication with the catheter body, which can receive fluid from the catheter body; the first stent 651 in the loaded state is radially compressed and installed on the balloon body; when the balloon body enters the inflated state from the folded state, the first stent is expanded from the radially compressed state to the radially expanded state based on the change of the balloon body.
an inner shaft which extends through the catheter body and the balloon body, with a guide head 646 fixed at its distal end; and
a locking wire structure 68 which is used to limit the axial position of the prosthetic heart valve relative to the balloon body.

The locking structure 68 includes:
a first adjustment wire 681, used to releasably fix the prosthetic heart valve 65 to the balloon body, one end of the first adjustment wire can be fixed to the catheter body 6411, and the other end can pass through the eyelet structure and has a first locking eyelet 6811;
a locking wire 682 which has a locked state and an unlocked state, in the locked state, the locking wire 682 passes through locking eyelets to restrict the prosthetic heart valve, and in the unlocked state, the locking wire 682 is disengaged from the locking eyelets to release the prosthetic heart valve; the guide head 646 is provided with an insertion hole, in the locked state, the end of the locking wire 682 extends into the insertion hole; and
a second adjustment wire 683, one end of which is connected to the guide head 646, and the other end can pass through the eyelet structure and has a second locking eyelet 6831; there are at least two junctions between the adjustment wires and the locking wire, at least one junction limits the movement of the prosthetic heart valve in the distal direction, and at least one junction limits the movement of the prosthetic heart valve in the proximal direction.

The first adjustment wire and the second adjustment wire are matched in length to simultaneously adjust the proximal and distal positions of the first stent, so that the first stent is approximately located in the middle of the balloon body in the axial direction. After the relative positions of the first stent and the balloon body are adjusted, they are locked in place.

The transcatheter implant system further includes the control handle 63 and the outer sheath 644 mentioned above, and when in use, the operation includes the following steps:
driving the outer sheath 644 to move toward the proximal side of the delivery system through the control handle to expose the prosthetic heart valve 65;
pulling the locking wire 682 out of the locking eyelets of the adjustment wires (the first adjustment wire and the second adjustment wire);
inflating the balloon body 642 to drive the prosthetic heart valve 65 to deform to an expanded state, and pulling the adjustment wires out of the prosthetic heart valve; and
deflating the balloon body 642 and driving the balloon device to move toward the proximal side of the transcatheter implant system and separate from the prosthetic heart valve.

In some embodiments, the transcatheter implant system can limit the axial movement of the prosthetic heart valve by a limiting mechanism(s), and specifically limit the distal end and/or proximal end of the first stent.

Referring to FIGS. 140 to 142, the present disclosure further provides a transcatheter implant system for delivering and expanding the prosthetic heart valve 65 of the previous embodiments. The transcatheter implant system includes a balloon device capable of limiting the axial movement of the prosthetic heart valve. The balloon device 641 specifically includes:
a catheter body 6411;
a balloon body 642 in communication with the catheter body, the balloon body 642 includes a first part 6421, a middle part 6422, and a second part 6423 from the distal end to the proximal end, and the middle part 6422 is used for loading and fixing the prosthetic heart valve 65; and
a limiting mechanism 62 which is configured to limit the axial movement of the prosthetic heart valve at least when the prosthetic implant is installed on the balloon body in a radially compressed state; specifically, the limiting mechanism includes a limiting body, which includes a plurality of rods; the plurality of rods generally extend from a first axial end to a second axial end, and converge at the first end and the second end respectively to form a hollow cage-like structure.

The catheter body and the balloon body can be combined with the limiting mechanism of the previous embodiments, and some embodiments below further improve the limiting mechanism. Referring to FIG. 141, in some embodiments, the limiting mechanism 62 includes at least one limiting body 622, and at least the distal end of the first stent is blocked by the limiting body 622.

The catheter body 6411 is used to deliver fluid. The balloon body 642 has a folded state (as shown in FIG. 140) and an inflated state (as shown in FIG. 141). When the prosthetic heart valve is delivered during intervention, the balloon body is in the folded state. The operator injects fluid, i.e., an inflation medium (e.g., saline), into the balloon body 642 to inflate the balloon body 642, so as to expand and deploy the prosthetic implant 65.

During the delivery process, the limiting body is used to fill the first part of the balloon body 642, and the side of the limiting body facing the proximal end blocks the distal end of the prosthetic heart valve 65. In addition, the limiting mechanism can fill the step between the balloon body and the prosthetic heart valve, which is helpful for delivery and reduces risks.

After the inflation is completed, the first stent is in an expanded state, so that the prosthetic heart valve 65 can be deployed via balloon expansion.

In some embodiments, the limiting mechanism includes a limiting body, a guide tube, and a coupling member. The limiting body is generally a cage-like structure. The guide tube is connected to the limiting body and they are formed in one piece by cutting a tube made of shape memory alloy. The coupling member is connected to the inner shaft, and the coupling member is also connected to at least one of the limiting body and the guide tube. The specific structures of the limiting body, the guide tube, and the coupling member can refer to the embodiments below.

Referring to FIG. 142, there are two limiting bodies, namely a limiting body 622a located at the proximal end of the guide tube and a limiting body 622b located at the distal end of the guide tube. During the delivery process, the distal end and the proximal end of the first stent are blocked by the corresponding limiting bodies. Preferably, the limiting body defines a concave area (see FIG. 95) for accommodating the end of the prosthetic heart valve 65. The concave area 62241 is roughly frustum-shaped. Both ends of the first stent are accurately limited by the corresponding concave areas.

In some embodiments, referring to FIGS. 143 to 144, the balloon device is configured with both a locking wire structure 68 and a limiting mechanism 62 for axial limiting for the prosthetic heart valve.

In some embodiments, the limiting mechanism 62 can include a limiting body to limit the axial movement of the distal end of the first stent. The proximal and distal ends of the first stent both have eyelet structures protruding from the corresponding ends, and one or both ends can be limited by the locking wire structure.

Referring to FIG. 143, the proximal end is fixed by a locking wire, and the distal end is fixed by a limiting mechanism. The locking wire structure 68 includes a first adjustment wire 681, which is used to releasably fix the prosthetic heart valve on the balloon body 642. One end of the first adjustment wire can be fixed to the catheter body, and the other end can pass through the eyelet structure and has a first locking eyelet 6811. The first locking eyelet 6811 is used to limit the eyelet structure at the proximal end of the prosthetic heart valve.

Referring to FIG. 144, the proximal end is fixed by a locking wire, and the distal end is fixed by a combination of a limiting mechanism and a locking wire. In addition to the first adjustment wire 681 shown in FIG. 143, the locking wire structure 68 further includes a second adjustment wire 683 with a second locking eyelet 6831 for passing through the eyelet structure at the distal end of the first stent.

In the embodiment shown in FIGS. 143 and 144, the locking wire structure further includes a locking wire 682, which has a locked state and an unlocked state. The locking wire 682 passes through one or two locking eyelets. In the locked state, the locking wire passes through the locking eyelet(s) to limit the prosthetic heart valve. In the unlocked state, the locking wire is disengaged from the locking eyelet(s) to release the prosthetic heart valve.

In some embodiments, the limiting mechanism 62 can include two limiting bodies respectively located at the proximal end and the distal end of the balloon body to limit the axial movement of the distal end and the proximal end of the first stent.

In the present disclosure, improvements are made to the prosthetic heart valve of the transcatheter implant system.

The present disclosure further provides a prosthetic heart valve assembly, including a prosthetic pulmonary valve (refer to the afore-mentioned prosthetic heart valve 65) and an anchoring stent 656. The prosthetic pulmonary valve includes a first stent 651 and a plurality of leaflets 652. The first stent 651 is a radially deformable cylindrical structure, the interior of the first stent is a blood flow channel, and the leaflets cooperate with each other to control the blood flow channel.

Referring to FIGS. 145 to 150, the first stent and the anchoring stent both have an expanded state and a compressed state. Taking the anchoring stent as an example, FIG. 150 is a schematic view of the anchoring stent in a compressed state, and FIG. 145 is a schematic view of the anchoring stent 656 in an expanded state after being assembled and fitted over the first stent. In the expanded state, the first stent and the anchoring stent are in contact.

The anchoring stent also has a pre-expanded state before assembly (as shown in FIG. 146). The anchoring stent has a first diameter D2 in the pre-expanded state and a second diameter D3 in the expanded state, and the second diameter D3 is larger than the first diameter D2.

In addition to the prosthetic pulmonary valve mentioned above, the prosthetic heart valve assembly can also be applied for aortic valves, mitral valves, and tricuspid valves, etc. In the expanded state, the axial length S2 of the anchoring stent is greater than the length S1 of the first stent, S2 is approximately 1.5 to 2 times of S1, and the first stent is approximately located in the middle of the anchoring stent in the axial direction.

As shown in FIG. 146, the side wall of the anchoring stent can also be formed by cutting to create struts 6561 and a plurality of cells 6562 formed by the struts. Preferably, the anchoring stent can be cut from a cobalt-chromium alloy tube, reducing costs while significantly improving fatigue resistance. As shown in FIG. 148, the cells include:
a middle part 65621 which includes two rows of fishbone-shaped cells 6563; the fishbone-shaped cells are concave hexagons with opposite convex tips 65631 and concave tails 65632; the concave tails of the two rows of fishbone-shaped cells are symmetrical and face each other, while the convex tips face outward; as shown in FIG. 149, adjacent fishbone-shaped cells share a strut 65633, which not only eliminates the shrinkage effects caused by the middle diamond-shaped cell (i.e., formed by the concave tails facing each other), but also avoids scratching the blood vessel due to an excessive shrinkage, while maintaining the overall radial strength; and
end parts 65622 including two rows of diamond-shaped cells 6564, which are respectively located on both sides of the middle part in the axial direction, and each diamond-shaped cell is located between the convex tips of two adjacent fishbone-shaped cells 6563.

In some embodiments, the axial position of the first stent does not exceed the middle part, that is, the two ends of the first stent are located between the two convex tips of the two rows of fishbone-shaped cells.

In the diamond-shaped cell at the end part of the anchoring stent, the two struts facing away from the middle part have lower strength than the two struts shared with the middle part. That is, as shown in FIG. 149, the struts constituting the diamond-shaped cell 6564 are strut 65641, strut 65642, strut 65643, and strut 65644, respectively. The angle B1 between strut 65641 and strut 65642 is larger than the other angles in the diamond-shaped cell. The large angle design not only increases the deformation capacity, but also has an anti-warping effect, reducing the risk of outward eversion and enhancing safety.

The strength of struts 65641 and 65642 is lower than that of the other two struts. The strength of the struts can be reduced by reducing metal content, further increasing the angle B1 formed after the expansion of the diamond-shaped cell 6564.

In addition, by adjusting metal content at different positions of the anchoring stent, issues such as different metal contents of the anchoring stent in the axial direction and inconsistent crimping diameters at different positions of the anchoring stent when crimped on the balloon can be avoided. In conjunction with FIGS. 149 and 150, the angle B2 at the concave tail 65632 of the fishbone-shaped cell is designed to be greater than the angle B3 at the convex tip 65631 to eliminate the problem of uneven radial forces caused by different metal amounts in the diamond-shaped cell, making the overall radial force more uniform.

In some embodiments, as shown in FIG. 149, the diamond-shaped cell 6564 of the anchoring stent also has an eyelet structure 653 protruding from the end, which can refer to the eyelet structure of the first stent described in the above embodiments.

The above-mentioned prosthetic pulmonary valve and anchoring stent can be deployed via balloon expansion or self-expansion.

Preferably, to ensure proper contact between the prosthetic pulmonary valve and the anchoring stent in the expanded state, the anchoring stent can be first deployed via balloon expansion or self-expansion, and then the prosthetic pulmonary valve can be deployed via balloon expansion.

To facilitate the cooperation with the prosthetic heart valve assembly in the above-mentioned embodiment, referring to FIGS. 151 to 153, the present disclosure further provides a transcatheter implant system, including:
a prosthetic heart valve as described in the previous embodiments; and
an anchoring stent fitted over the prosthetic heart valve; the prosthetic heart valve and the anchoring stent both have an expanded state and a compressed state, and in the expanded state, the first stent of the prosthetic heart valve contacts the anchoring stent.

The transcatheter implant system first deploys the anchoring stent, and after the deployment is completed, the anchoring stent has a first diameter in a pre-expanded state. For example, the anchoring stent in this state may be smaller than or equal to its nominal diameter.

Then, the prosthetic heart valve is deployed, ensuring it contacts the anchoring stent. In this state, the anchoring stent has a second diameter, and the second diameter is greater than the first diameter. The second diameter may be greater than or equal to the nominal diameter of the anchoring stent.

In one embodiment, two delivery systems may be provided, one for implanting the prosthetic heart valve and one for implanting the anchoring stent.

As shown in FIG. 151, the transcatheter implant system further includes a first delivery system 611 having a first balloon body 642 for delivering and expanding a prosthetic heart valve.

The prosthetic heart valve and the anchoring stent can refer to the previous embodiments, and the delivery system further includes the catheter body for delivering fluid into the first balloon body and the control handle mentioned above.

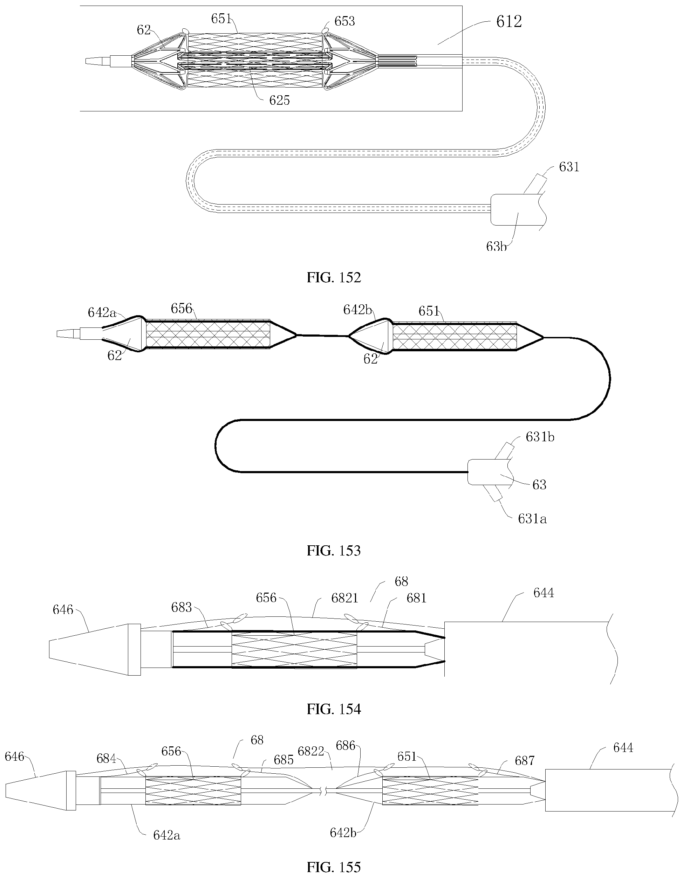

As shown in FIG. 152, when the anchoring stent is deployed via balloon expansion, the transcatheter implant system further includes a second delivery system 612 for expanding the anchoring stent. The second delivery system is configured in the same manner as the first delivery system, including a balloon body for delivering and expanding the prosthetic heart valve and a control handle. In other words, two delivery systems are provided for the prosthetic heart valve and the anchoring stent respectively. Each delivery system has its own control handle (such as control handles 63a and 63b in the figure), and the interventional delivery can be performed using the same or different interventional paths.

Alternatively, the anchoring stent can be deployed by self-expansion. For example, the anchoring stent is made of shape memory metal material. Accordingly, the second delivery system can be a delivery system for delivering the self-expandable stent, which can be based on existing technology and is not described in detail here.

In another embodiment, one delivery system can be provided and shared by the prosthetic heart valve and the anchoring stent.

When the anchoring stent is deployed via balloon expansion, as shown in FIG. 153, the first delivery system can be configured with two independently controlled balloon bodies: a first balloon body 642b for delivering and expanding the prosthetic heart valve, and a second balloon body 642a for delivering and expanding the anchoring stent. Each balloon body is equipped with a separate fluid channel. For example, the control handle 63 is provided with two interfaces 631a and 631b, corresponding to the first balloon body 642a and the second balloon body 642b, respectively, to avoid mutual interference. The two balloon bodies are arranged in the axial direction, and are respectively loaded with a prosthetic heart valve and an anchoring stent. The anchoring stent 656 to be deployed first is located at the distal end of the prosthetic heart valve.

Alternatively, when the anchoring stent is self-expandable, the delivery system can include only one balloon device for expanding the prosthetic heart valve (not shown). The delivery system may further include a sheath for accommodating the anchoring stent in a compressed state in the sheath.

In the above embodiment, when the anchoring stent is deployed via balloon expansion, a limiting mechanism(s) 62 can be provided at the distal end or both ends of the anchoring stent during the process of delivering and expanding the anchoring stent. If two independent delivery systems are used, the balloon body of the second delivery system can be provided with a limiting mechanism(s) to limit the axial movement of the distal and/or proximal ends of the anchoring stent. If only one delivery system is used, both the first balloon body and the second balloon body are provided with limiting mechanisms to limit the axial movement of the distal and/or proximal ends of the prosthetic heart valve and the anchoring stent. The specific structure of the limiting mechanism can refer to the following embodiments.

In some embodiments, the transcatheter implant system can also include a locking wire structure to limit the axial movement of the prosthetic heart valve assembly. Referring to FIGS. 154 to 156, the first delivery system and the second delivery system (i.e., the second balloon body) can be provided with a locking wire structure 68 to limit the axial movements of two ends of the anchoring stent.

Alternatively, both the prosthetic heart valve and the anchoring stent are provided with a locking wire structure 68 for axial limiting. Specifically, if a shared delivery system is used, both balloon bodies of the delivery system can be provided with a locking wire structure. Alternatively, if two delivery systems are used, the balloon body of each delivery system can be provided with a locking wire structure.

As shown in FIG. 154, when the locking wire structure is only configured for the anchoring stent, both ends of the anchoring stent have eyelet structures protruding from the corresponding ends, and the locking wire structure includes:
a first adjustment wire 681, used to releasably fix the first stent on the balloon body, one end of the first adjustment wire can be fixed to the catheter body, and the other end can pass through the corresponding eyelet structure and has a first locking eyelet;
a second adjustment wire 683, one end of which is connected to the guide head and the other end can pass through the corresponding eyelet structure and has a second locking eyelet; and
a first locking wire 6821, which has a locked state and an unlocked state; in the locked state, the locking wire passes through the locking eyelets to restrict the first stent; in the unlocked state, the locking wire is disengaged from the locking eyelets to release the anchoring stent.

When the locking wire structure is configured for the prosthetic heart valve and the anchoring stent, especially in the case where only one delivery system is provided and shared by the first stent and the anchoring stent, as shown in FIG. 155, the first balloon body 642b and the second balloon body 642a respectively and independently limit the axial positions of the prosthetic heart valve and the anchoring stent, and the locking wire structure specifically includes:
a third adjustment wire 684 and a fourth adjustment wire 685, used to releasably fix the anchoring stent on the first balloon body, with locking eyelets that can pass through the corresponding eyelet structures;
a fifth adjustment wire 686 and a sixth adjustment wire 687, used to releasably fix the prosthetic heart valve on the second balloon body, with locking eyelets that can pass through the corresponding eyelet structures; and
a second locking wire 6822, which has a locked state and an unlocked state; in the locked state, the locking wire passes through the locking eyelets to restrict the prosthetic heart valve and the anchoring stent; in the unlocked state, the locking wire is disengaged from the locking eyelets to release the prosthetic heart valve and the anchoring stent. The two balloon bodies are arranged in the axial direction, respectively loaded with the prosthetic heart valve and the anchoring stent, and the anchoring stent 656 to be deployed first is located at the distal end of the first stent.

In other embodiments, the locking wire may further include a first locking wire and a third locking wire (not shown). In the locked state, the first locking wire passes through the corresponding locking eyelets to restrict the prosthetic heart valve, and the third locking wire passes through the corresponding locking eyelets to restrict the anchoring stent.

In some embodiments, whether two independently configured delivery systems or a shared delivery system is used, both the locking wire structure and the limiting mechanism can be configured for the anchoring stent, or for both the prosthetic heart valve and the anchoring stent.

When the transcatheter implant system employs two independently configured delivery systems, a locking wire structure and a limiting mechanism(s) can be configured for the anchoring stent in the second delivery system, or both the first delivery system and the second delivery system can be each configured with a locking wire structure and a limiting mechanism(s). The detail refers to the following embodiments.

When the transcatheter implant system employs a shared delivery system, a locking wire structure and a limiting mechanism(s) can be configured in the second balloon body for the anchoring stent, or a locking wire structure and a limiting mechanism(s) can be configured in both the first balloon body and the second balloon body. The detail refers to the previous embodiments.

The present disclosure further provides a releasing method for a prosthetic heart valve assembly from an interventional delivery system, which is applicable to the prosthetic heart valve assembly and interventional delivery system in the above-mentioned embodiments.

The prosthetic heart valve assembly includes a prosthetic heart valve and an anchoring stent, both of which have an expanded state and a compressed state. The specific structure of the prosthetic heart valve assembly refers to the previous embodiments.

The interventional delivery system includes at least an inner shaft 643 for interventional delivery, to which both the prosthetic heart valve and the anchoring stent are connected in the compressed state.

The method releasing for the prosthetic heart valve assembly from the interventional delivery system mentioned above includes the following steps:
step S100, releasing the connection between the anchoring stent and the inner shaft, allowing the anchoring stent to enter an expanded state;
step S200, delivering the prosthetic heart valve to the interior of the anchoring stent; and
step S300, releasing the connection between the prosthetic heart valve and the inner shaft, allowing the prosthetic heart valve to enter an expanded state and fit tightly against the inner wall of the anchoring stent.

This releasing method can be implemented not only in product testing and ex vivo simulation training but also in interventional surgical procedures.

In some embodiments, when the prosthetic heart valve assembly is deployed via self-expansion, the interventional delivery system further includes an outer sheath 644, which is slidably installed on the outer periphery of the inner shaft. The anchoring stent 656 is connected to the inner shaft 643 and enclosed by the outer sheath.

When releasing the connection between the anchoring stent and the inner shaft in step S100, the outer sheath and the inner shaft are first moved relative to each other to expose the anchoring stent from the outer sheath, thereby allowing the anchoring stent to enter the expanded state in a self-expanding manner.

In some embodiments, when the prosthetic heart valve assembly is deployed via balloon expansion, the transcatheter implant system for delivering the prosthetic heart valve assembly includes only one inner shaft. This corresponds to the scenario in which the transcatheter implant system described above consists only of the first delivery system, which includes a first balloon body for delivering and expanding the prosthetic heart valve, and a second balloon body for delivering and expanding the anchoring stent-meaning that a single delivery system is used for both the prosthetic heart valve and the anchoring stent.

Preferably, the interventional delivery system further includes:
two balloon bodies (i.e., a first balloon body and a second balloon body) which are arranged in sequence along the inner shaft; the prosthetic heart valve and the anchoring stent are respectively connected to the outer peripheries of the corresponding balloon bodies; and
a locking wire which is used to simultaneously limit the axial positions of the prosthetic heart valve and the anchoring stent relative to the corresponding balloon bodies.

When releasing the connection between the anchoring stent and the inner shaft in step S100, an additional step of moving the locking wire is included before or after inflating the balloon body corresponding to the anchoring stent to release the axial limitation on the anchoring stent while maintaining the axial limitation on the prosthetic heart valve.

When releasing the connection between the prosthetic heart valve and the inner shaft in step S300, an additional step of further moving the locking wire along the original movement direction before or after inflating the balloon body corresponding to the prosthetic heart valve to release the axial limitation on the prosthetic heart valve.

In some embodiments, when the prosthetic heart valve is a prosthetic pulmonary valve, during ex vivo simulation or interventional surgery, the interventional path of the interventional delivery system needs to make 2 turns near the tricuspid valve. It can be understood that the inner shaft should be at least S-shaped to reach the deployment position.

Referring to FIG. 157, in order to meet the above-mentioned bending requirements, the interventional delivery system further includes a deflection assembly, which can include a deflection module 613 of the handle of the delivery system and a deflectable tube 614 in the catheter assembly. The deflection assembly can at least act on the inner shaft to change the orientation of the distal end of the inner shaft. The specific implementation of the deflection module can refer to existing technologies, which will not be described in detail.

Before the prosthetic heart valve assembly is detached from the inner shaft, under the action of the deflection assembly, the inner shaft has a smooth bent section, or has at least two smooth bent sections, and the two bent sections have opposite curvatures. As shown in FIG. 158, the deflectable tube 614 has two smooth bent sections 6141, forming a roughly S-shaped curve. The part of the inner shaft corresponding to the bent section can be made of a metal cutting tube to adapt to the bending shape.

The technical features of the previous embodiments can be arbitrarily combined, and not all possible combinations of the technical features of the previous embodiments have been described for the sake of brevity of description. However, as long as there is no contradiction in the combination of these technical characteristics, such combination should be regarded as falling into the scope of this specification. When the technical features in different embodiments are shown in the same drawing, it can be considered that the drawing also discloses a combined embodiment of various embodiments involved.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure.

## Claims

1. An interventional delivery system, comprising a catheter assembly and a control handle connected to the catheter assembly, wherein the catheter assembly has a distal end and a proximal end opposite to each other and comprises an inner sheath and an outer sheath slidably engaged with each other, the outer sheath comprises a loading section and a sheath section sequentially from a distal end to a proximal end, and the loading section is configured to enclose an interventional device and keep the interventional device in a compressed state;
the control handle comprising:
a main handle, to which a proximal end of the inner sheath is connected; and
an auxiliary handle, slidably fitted over the inner sheath, to which the proximal end of the outer sheath is connected.

2. The interventional delivery system of claim 1, wherein the auxiliary handle comprises:
a housing having an axial direction, with a distal end and a proximal end opposite to each other in the axial direction, the housing having an axially through threading channel;
a connecting sleeve fixed in the housing and arranged around the threading channel, with a distal end of the connecting sleeve configured for insertion of the outer sheath;
a positioning member movably mounted in the housing, the positioning member having a locked position for restricting the threading channel and an unlocked position for releasing the threading channel; and
a driving member which is in transmission cooperation with the positioning member to drive the positioning member to switch positions.

3. The interventional delivery system of claim 1, wherein a sliding range of the auxiliary handle relative to the main handle is more than 10 to 40 cm.

4. The interventional delivery system of claim 2, wherein a side wall of the connecting sleeve defines a first vent hole in communication with the threading channel, a first pipe joint is fixedly connected to the first vent hole, the first vent hole and the first pipe joint define a first vent channel, and a one-way valve core is provided in the first vent channel.

5. The interventional delivery system of claim 4, wherein a first annular seat is arranged around the first vent hole, and the first pipe joint is inserted and mated with the first annular seat, with an anti-rotation structure provided between the first pipe joint and the first annular seat.

6. The interventional delivery system of claim 4, wherein, optionally, a first protective sleeve arranged around the first pipe joint is embedded in the housing, and a pipeline insertion gap is defined between an outer wall of the first pipe joint and an inner wall of the first protective sleeve.

7. The interventional delivery system of claim 5, wherein, optionally, the anti-rotation structure comprises:
a slot extending in an insertion direction of the first pipe joint, wherein the slot is provided on a side wall of one of the first pipe joint and the first annular seat; and
a positioning rib engaging with the slot, wherein the positioning rib is provided on a side wall of the other of the first pipe joint and the first annular seat.

8. The interventional delivery system of claim 4, wherein the one-way valve core has a sealed state and an open state, and the one-way valve core specifically comprises:
a limiting rod which is slidably mounted in an inner cavity of the first pipe joint, with a fluid gap between an outer periphery of the limiting rod and an inner wall of the first pipe joint; and
a sealing sheet connected to the limiting rod, which closes the fluid gap in the sealed state and opens the fluid gap in the open state.

9. The interventional delivery system of claim 8, wherein the inner wall of the first pipe joint is provided with a retaining ring surrounding the outer periphery of the limiting rod, and the fluid gap is located between an outer periphery of the retaining ring and the inner wall of the first pipe joint;
an end of the limiting rod away from the sealing sheet passes through the retaining ring and has a head; in the open state, the head abuts against the retaining ring, and the sealing sheet is away from the retaining ring and opens the fluid gap; in the sealed state, the head is away from the retaining ring, and the sealing sheet abuts against the retaining ring and closes the fluid gap.

10. The interventional delivery system of claim 8, wherein the inner wall of the first pipe joint is provided with a retaining ring surrounding the outer periphery of the limiting rod, and the fluid gap is located between an outer periphery of the retaining ring and the inner wall of the first pipe joint;
an end of the limiting rod away from the sealing sheet passes through the retaining ring and has a head restricted by the retaining ring, the head and the sealing sheet are fixed on two opposite sides of the retaining ring, and an edge of the sealing sheet is elastically deformable to open or close the fluid gap.

11. The interventional delivery system of claim 2, wherein the positioning member comprises an elastic claw, one end of the elastic claw is fixed to the connecting sleeve, and the other end is a free end that is capable of swinging in a radial direction of the connecting sleeve.

12. The interventional delivery system of claim 11, wherein the elastic claw and the connecting sleeve are formed in one piece, and the positioning member comprises two to six elastic claws distributed in a circumferential direction of the connecting sleeve.

13. The interventional delivery system of claim 11, wherein the driving member is cylindrical and slidably fitted over the connecting sleeve; in the radial direction of the connecting sleeve, an outer side of the elastic claw is provided with a guide slope, and an inner wall of the driving member acts on the guide slope.

14. The interventional delivery system of claim 11, wherein an elastic liner is provided inside the connecting sleeve, and the elastic claw acts on an outer wall of the elastic liner to restrict the threading channel by compressing the elastic liner.

15. The interventional delivery system of claim 14, wherein a distal end of the elastic liner extends out of the connecting sleeve, and the extended portion is provided with a radially outwardly protruding positioning step; in the axial direction, the positioning step is axially positioned and matched with an end face of the connecting sleeve and/or an inner wall of the housing.

16. The interventional delivery system of claim 2, wherein the driving member is cylindrical and slidably fitted over the connecting sleeve, and a guiding structure is provided between an inner wall of the driving member and an outer wall of the connecting sleeve; the guiding structure comprises:
a slide groove defined on one of the inner wall of the driving member and the outer wall of the connecting sleeve; and
a guide rib engaging with the slide groove and provided on the other of the inner wall of the driving member and the outer wall of the connecting sleeve.

17. The interventional delivery system of claim 1, wherein the catheter assembly further comprises a balloon catheter, and the balloon catheter comprises:
a balloon body provided at a distal end of the inner sheath and configured to switch between an inflated state and a folded state under an action of fluid, the interventional device in a loaded state being located around the balloon body; and
a catheter body fitted over the inner sheath or arranged in parallel with the inner sheath, wherein a distal end of the catheter body is in communication with the balloon body, a proximal end of the catheter body is connected to the main handle, and the catheter body contains a fluid channel.

18. The interventional delivery system of claim 17, wherein a fluid guide member is provided in the balloon body and is located around the inner sheath, and the fluid guide member defines a diversion channel that communicates a distal end and a proximal end of the balloon body.

19. The interventional delivery system of claim 18, wherein the fluid guide member is tubular, and the diversion channel is positioned in at least one of the following ways relative to the fluid guide member:
on an outer wall of the fluid guide member;
inside the fluid guide member; and
in a side wall interlayer or hollow area of the fluid guide member.

20. The interventional delivery system of claim 19, wherein the diversion channel comprises a plurality of diversion grooves opened on an outer circumferential wall of the fluid guide member and distributed in a circumferential direction of the fluid guide member, and each diversion groove extends in an axial direction of the fluid guide member or in a helical pattern.

21. The interventional delivery system of claim 20, wherein in the circumferential direction of the fluid guide member, a wrap angle of each diversion groove is in a range of 0 to 180 degrees.

22. The interventional delivery system of claim 20, wherein a communication groove is defined between two adjacent diversion grooves, and a plurality of communication grooves arranged at intervals are provided, while the communication grooves at a same axial position form an annular groove.

23. The interventional delivery system of claim 19, wherein a fluid guide member is provided around the inner sheath, the fluid guide member is tubular and is located inside the balloon body, a tube wall of the fluid guide member defines a hollow area, and the hollow area extends continuously or intermittently from a distal side of the fluid guide member to a proximal side of the fluid guide member.

24. The interventional delivery system of claim 19, wherein there is a radial gap between the fluid guide member and the inner sheath and serves as the diversion channel.

25. The interventional delivery system of claim 17, wherein the catheter assembly further comprises a deflectable tube and a traction member for driving the deflectable tube, the deflectable tube is fitted over the inner sheath, distal ends of the deflectable tube and the traction member are fixedly connected, proximal ends of the deflectable tube and the traction member are connected to the main handle and are in relative sliding fit, and the deflectable tube and the traction member are both located on an outer periphery of the catheter body.

26. The interventional delivery system of claim 25, wherein the traction member is a traction tube and is located inside or outside the deflectable tube.

27. The interventional delivery system of claim 25, wherein the traction member is a traction wire, and is located inside, outside or embedded within a wall of the deflectable tube.

28. The interventional delivery system of claim 26, wherein a wall of the deflectable tube has an interlayer structure, within which a lining tube is fixed, and the traction member is movably arranged in the lining tube.

29. The interventional delivery system of claim 17, wherein the main handle comprises:
a support body having an axial direction, wherein the inner sheath and the catheter body of the balloon catheter both extend to and are connected with a proximal side of the support body;
a second sliding seat axially slidably engaged with the support body;
a second driving sleeve rotatably fitted over the support body and located around the second sliding seat, wherein the second driving sleeve and the second sliding seat are engaged in a threaded transmission fit; and
a multi-way connector connected to a proximal side of the second sliding seat, wherein proximal ends of both the balloon catheter and the inner sheath are connected to the multi-way connector, an interior of the inner sheath is a through guidewire channel, and the multi-way connector has at least interfaces respectively connected to the guidewire channel and the fluid channel.

30. The interventional delivery system of claim 29, wherein the multi-way connector and the proximal side of the second sliding seat are rotatably engaged with each other and axially limited relative to each other.

31. The interventional delivery system of claim 29, wherein the second sliding seat is generally cylindrical and at least partially housed within the second driving sleeve, an outer wall of the second sliding seat is in threaded fit with an inner wall of the second driving sleeve, and the support body has a guide groove for guiding the second sliding seat to move axially.

32. The interventional delivery system of claim 29, wherein a distal end of the multi-way connector and a proximal end of the second sliding seat are inserted into and rotatably engaged with each other, with an axial limiting structure between the multi-way connector and the second sliding seat, and the axial limiting structure comprises:
a latching tooth, fixed to one of the multi-way connector and the second sliding seat and protruding radially toward the other; and
a latching groove, which is provided on the other of the multi-way connector and the second sliding seat and receives the latching tooth.

33. The interventional delivery system of claim 1, wherein the catheter assembly further comprises a deflectable tube and a traction member for driving the deflectable tube, the deflectable tube is fitted over the inner sheath, distal ends of the deflectable tube and the traction member are fixedly connected, and proximal ends of the deflectable tube and the traction member are connected to the main handle and are in relative sliding fit.

34. The interventional delivery system of claim 33, wherein the main handle comprises:
a support body having an axial direction, wherein the proximal end of the deflectable tube is fixedly connected to the support body, and the inner sheath extends to and is connected with a proximal side of the support body;
a first sliding seat axially slidably engaged with the support body, wherein the proximal end of the traction member is fixed to the first sliding seat;
a first driving sleeve, which is rotatably fitted over the support body and is located around the first sliding seat, wherein the first driving sleeve and the first sliding seat are engaged in a threaded transmission fit;
a housing fixedly fitted over the support body, with at least a portion being an indicating section located outside the first driving sleeve; and
an identification member which is axially slidably installed on the indicating section and is located on an outer periphery of the first driving sleeve, wherein the first driving sleeve and the identification member are engaged in a threaded transmission fit.

35. The interventional delivery system of claim 34, wherein the first driving sleeve has an internal thread and an external thread, the first sliding seat has an external tooth that matches with the internal thread, and the identification member has an internal tooth that matches with the external thread; and wherein the identification member and the first sliding seat are configured to move synchronously under an action of the first driving sleeve.

36. The interventional delivery system of claim 34, wherein a view window corresponding to the identification member in position is provided on a side wall of the indicating section.

37. The interventional delivery system of claim 34, wherein an installation groove extending axially is provided in the support body, a guide cylinder is fixed in the installation groove, the first sliding seat is slidably fitted over the guide cylinder, and a guiding structure is provided between the first sliding seat and an outer wall of the guide cylinder to guide the axial movement; wherein the proximal end of the deflectable tube is inserted into and fixed to the guide cylinder.

38. The interventional delivery system of claim 33, wherein when the auxiliary handle slides proximally to an extreme position, the distal end of the deflectable tube is exposed from the outer sheath, with an exposure length ranging from 0 to 20 cm.

39. The interventional delivery system of claim 37, wherein a second vent hole is defined on a side wall of the guide cylinder, a radial gap between the deflectable tube and the catheter body is in communication with the second vent hole, and a proximal end of the catheter body passes through the proximal end of the deflectable tube and further extends through the guide cylinder;
a second pipe joint is fixedly connected at the second vent hole, the second vent hole and the second pipe joint define a second vent channel, and a one-way valve core is provided in the second vent channel.

40. The interventional delivery system of claim 39, wherein the one-way valve core has a sealed state and an open state, and the one-way valve core specifically comprises:
a limiting rod which is slidably mounted in an inner cavity of the second pipe joint, with a fluid gap between an outer periphery of the limiting rod and an inner wall of the second pipe joint; and
a sealing sheet connected to the limiting rod, which closes the fluid gap in the sealed state and opens the fluid gap in the open state.

41. The interventional delivery system of claim 40, wherein a second annular seat is provided around the second vent hole, and the second pipe joint is inserted and mated with the second annular seat, with an anti-rotation structure provided between the second pipe joint and the second annular seat.

42. The interventional delivery system of claim 41, wherein a second protective sleeve is provided around the second pipe joint, and a pipeline insertion gap is defined between an outer wall of the second pipe joint and an inner wall of the second protective sleeve.

43. The interventional delivery system of claim 41, wherein the anti-rotation structure comprises:
a slot extending in an insertion direction of the second pipe joint, wherein the slot is provided on a side wall of one of the second pipe joint and the second annular seat; and
a positioning rib engaging with the slot, wherein the positioning rib is provided on a side wall of the other of the second pipe joint and the second annular seat.

44. The interventional delivery system of claim 40, wherein the inner wall of the second pipe joint is provided with a retaining ring surrounding the outer periphery of the limiting rod, and the fluid gap is located between an outer periphery of the retaining ring and the inner wall of the second pipe joint.

45. The interventional delivery system of claim 44, wherein an end of the limiting rod away from the sealing sheet passes through the retaining ring and has a head restricted by the retaining ring, the head and the sealing sheet are fixed on two opposite sides of the retaining ring, and an edge of the sealing sheet is elastically deformable to open or close the fluid gap.

46. The interventional delivery system of claim 1, wherein the interventional delivery system is configured to deliver a prosthetic implant, the prosthetic implant comprises a stent and leaflets, the stent has a cell structure, two ends of the stent are respectively an inflow side and an outflow side, and an interior of the stent is a blood flow channel;
the leaflets are located in the blood flow channel and cooperate with each other to open or close the blood flow channel, and an edge of the leaflet comprises a fixed edge fixed to the stent and a free edge that cooperates with other leaflets to control the blood flow channel;
the stent is connected with an anti-paravalvular leakage component, which is formed in one piece and comprises a base inside the stent and anti-paravalvular leakage elements fixed to an outside of the base, and the anti-paravalvular leakage elements are spaced apart and block-shaped and aligned with hollow areas of the stent's cells.

47. The interventional delivery system of claim 46, wherein the base is made of PET material.

48. The interventional delivery system of claim 47, wherein the anti-paravalvular leakage element is made of PU material.

49. The interventional delivery system of claim 46, wherein depending on different axial positions of corresponding cells, the anti-paravalvular leakage elements comprise a first row of anti-paravalvular leakage elements and at least one of a second row of anti-paravalvular leakage elements and a third row of anti-paravalvular leakage elements, wherein the first row of anti-paravalvular leakage elements cover entire corresponding cells;
the second row of anti-paravalvular leakage elements and the third row of anti-paravalvular leakage elements cover partial areas of corresponding cells.

50. The interventional delivery system of claim 49, wherein the second row of anti-paravalvular leakage elements are adjacent to an inflow side of the first row of anti-paravalvular leakage elements.

51. The interventional delivery system of claim 49, wherein the third row of anti-paravalvular leakage elements are adjacent to an outflow side of the first row of anti-paravalvular leakage elements.

52. The interventional delivery system of claim 49, wherein axial lengths of the second row of anti-paravalvular leakage elements and the third row of anti-paravalvular leakage elements are only half a length of a cell respectively, and are adjacent to the first row of anti-paravalvular leakage elements.

53. The interventional delivery system of claim 49, wherein each anti-paravalvular leakage element, from an outflow side thereof to an inflow side thereof, gradually increases in thickness, and then gradually decreases in thickness after reaching a maximum outward protrusion.

54. The interventional delivery system of claim 53, wherein for each anti-paravalvular leakage element, the maximum outward protrusion is closer to an inflow side of a corresponding cell.

55. The interventional delivery system of claim 54, wherein for each anti-paravalvular leakage element, a distance from the maximum outward protrusion to the inflow side of the corresponding cell is S1, and a distance from the maximum outward protrusion to an outflow side of the corresponding cell is S2, wherein S1 : S2 is in a range of 0.2 to 0.8.

56. The interventional delivery system of claim 1, wherein a diameter of the loading section is larger than a diameter of the sheath section.

57. The interventional delivery system of claim 1, wherein a distal end of the inner sheath is provided with a guide head, an outer circumference of the guide head is provided with an annular step, and a distal end surface of the loading section abuts against the annular step for position limitation.

58. An interventional delivery device based on balloon expansion deployment, which has opposite distal and proximal ends and comprises an inner shaft and a balloon catheter located around the inner shaft, wherein the balloon catheter comprises a balloon body and a catheter body in communication with a proximal end of the balloon body, the balloon body comprises a first part, a middle part and a second part from a distal end to a proximal end, and the middle part is configured for loading and fixing a prosthetic implant;
a flow guide tube is arranged in a radial gap between the inner shaft and the balloon body, a distal end of the flow guide tube is fixed to a distal end of the inner shaft, while the remaining part of the flow guide tube is suspended between the inner shaft and the balloon catheter;
at least a part of fluid output from the catheter body to the balloon body passes through the second part and the middle part via the flow guide tube and then enters and inflates the first part.

59. The interventional delivery device of claim 58, wherein a radial gap between the inner shaft and the catheter body serves as a main flow channel, and the main flow channel is a single flow channel.

60. The interventional delivery device of claim 58, wherein a part of the fluid output from the main flow channel to the balloon body enters and inflates the second part, while the other part enters the flow guide tube, passes through the second part and the middle part, and then enters and inflates the first part.

61. An interventional delivery device based on balloon expansion deployment, which has opposite distal and proximal ends and comprises an inner shaft and a balloon catheter located around the inner shaft, wherein the balloon catheter comprises a balloon body and a catheter body in communication with a proximal end of the balloon body, the balloon body comprises a first part, a middle part and a second part from a distal end to a proximal end, the middle part is configured for loading and fixing a prosthetic implant, and the catheter body has a main flow channel;
a flow guide tube is provided in a radial gap between the inner shaft and the balloon body, and at least a part of fluid output from the main flow channel to the balloon body passes through the second part and the middle part through the flow guide tube and then enters and inflates the first part.

62. The interventional delivery device of claim 61, wherein a proximal end of the flow guide tube has an open port serving as a fluid inlet.

63. The interventional delivery device of claim 62, wherein an axial position of the fluid inlet is adjacent to a junction of the balloon body and the catheter body.

64. The interventional delivery device of claim 62, wherein the balloon body is in an inflated state after being infused with fluid, and in the inflated state, the fluid inlet is located at a position where a cross-sectional area of a flow channel of the second part changes abruptly relative to the catheter body.

65. The interventional delivery device of claim 61, wherein in a loaded state, the prosthetic implant is radially compressed and encloses the middle part, while the first part and the second part are exposed from both ends of the prosthetic implant; a proximal end of the flow guide tube has a fluid inlet, and the fluid inlet is located in the second part.

66. The interventional delivery device of claim 61, wherein at a proximal port of the flow guide tube, there is a first gap with a radial span of L1 between the inner shaft and the flow guide tube, and there is a second gap with a radial span of L2 between the inner shaft and the catheter body, and wherein L1 : L2 = 1 : 1.25 to 1.6.

67. The interventional delivery device of claim 61, wherein the flow guide tube comprises a distal section, a middle section and a proximal section from a distal end to a proximal end corresponding to different parts of the balloon body respectively, and the distal section of the flow guide tube has a first fluid outlet in communication with the first part, and wherein the first fluid outlet is located on a tube wall of the distal section and/or an end face of the distal section.

68. The interventional delivery device of claim 67, wherein a plurality of first fluid outlets are provided on the tube wall of the distal section.

69. The interventional delivery device of claim 68, wherein the plurality of first fluid outlets are distributed in a circumferential direction of the flow guide tube.

70. The interventional delivery device of claim 68, wherein the plurality of first fluid outlets are located in a middle of the distal section in a length direction of the flow guide tube.

71. The interventional delivery device of claim 61, wherein a proximal end of the flow guide tube and a distal end of the catheter body are interconnected and integrated into one piece, and a tube wall of the proximal section is provided with a second fluid outlet in communication with the second part.

72. The interventional delivery device of claim 61, wherein a proximal end of the flow guide tube has a tapered section with a gradually converging shape, and at least a part of the tapered section extends into a distal end of the catheter body.

73. The interventional delivery device of claim 72, wherein the tapered section has one or two beveled surfaces.

74. The interventional delivery device of claim 73, wherein an inner lumen of the flow guide tube is open to the beveled surface and is simultaneously in communication with an inner lumen of the catheter body and an inner cavity of the second part.

75. The interventional delivery device of claim 61, wherein a spacer is provided between the inner shaft and the flow guide tube in a radial direction to maintain a radial gap between them.

76. The interventional delivery device of claim 75, wherein the spacer comprises a rib located on an inner wall of the flow guide tube.

77. The interventional delivery device of claim 75, wherein the spacer is a hollow structural component fixed between the inner shaft and the flow guide tube in the radial direction.

78. The interventional delivery device of claim 61, wherein a guide head is fixed to a distal end of the inner shaft, and a distal end of the flow guide tube is fixed to a proximal side of the guide head.

79. The interventional delivery system of claim 78, wherein the distal end of the flow guide tube is closed by the guide head.

80. The interventional delivery device of claim 78, wherein the proximal side of the guide head is provided with a positioning structure adapted to the distal end of the flow guide tube, and the positioning structure is a coupling groove for inserting the flow guide tube or a coupling column for inserting the flow guide tube.

81. A method for driving a balloon catheter with fluid, wherein the balloon catheter has a distal end and a proximal end opposite to each other and comprises a balloon body and a catheter body, a distal end of the catheter body is connected to a proximal end of the balloon body, the balloon body comprises a first part, a middle part and a second part from a distal end to a proximal end, the middle part is configured for loading and fixing a prosthetic implant, the catheter body has a main flow channel, and the method comprises steps of:
injecting fluid into the main flow channel from a proximal end of the catheter body;
diverting the fluid output from the main flow channel to the balloon body, allowing only a part of the fluid to enter and inflate the second part; and
directing the other part of the fluid to pass through the second part and the middle part, and then enter and inflate the first part.

82. The method of claim 81, wherein all fluid for inflating the balloon body is supplied from the main flow channel before being diverted.

83. The method of claim 81, wherein the diverting step comprises:
splitting the fluid into two streams at a junction of the catheter body and the second part, one of which enters and inflates the second part, and the other enters and inflates the first part after passing through the second part and the middle part through an independent flow channel.

84. The method of claim 83, wherein all the fluid enters the independent flow channel from the main flow channel, and the independent flow channel is in communication with a branch flow channel, wherein a part of the fluid enters and inflates the second part through the branch flow channel when flowing through the second part, and the other part enters and inflates the first part after passing through the second part and the middle part through the independent flow channel.

85. The method of claim 81, further comprising:
controlling the fluid so that the first part and the second part are inflated before the middle part, and the balloon body enters a transitional state in which both ends are inflated while the middle part is relatively contracted; and continuously injecting the fluid into the catheter body until the balloon body is fully inflated and the prosthetic implant is radially expanded.

86. The method of claim 81, wherein the fluid is split into two streams, with each stream comprising one or more sub-streams.

87. The method of claim 86, wherein the sub-streams of the same stream are radially distributed.

88. The method of claim 81, wherein the prosthetic implant is a cylindrical structure, and the fluid entering the independent flow channel passes through the middle part through an interior of the cylindrical structure.

89. The method of claim 81, wherein the balloon body has an axial direction extending between the distal end and the proximal end and corresponding radial and circumferential directions, and the step of inflating the first part comprises:
outputting the fluid in the radial direction of the balloon body through the independent flow channel and inflating the first part; and/or
output the fluid in the axial direction of the balloon body through the independent flow channel and inflating the first part.

90. A limiting mechanism for an interventional delivery system, comprising:
a coupling portion for connecting with the interventional delivery system, the coupling portion having an axial direction and corresponding circumferential and radial directions; and
a deformable portion comprising a plurality of elongated rods arranged in sequence in the circumferential direction, each of which has:
a first end connected to the coupling portion; and
a second end located on one axial side of the coupling portion relative to the first end; wherein the rods are elastically deformable, and have a compressed state suitable for interventional delivery and an expanded state, and wherein the second ends of the rods diverge outward in the radial direction of the coupling portion in the expanded state relative to the compressed state.

91. The limiting mechanism of claim 90, wherein the coupling portion is in a straight cylindrical shape, and the rods are distributed radially, with the first ends of the rods converging at one axial end of the coupling portion.

92. The limiting mechanism of claim 91, wherein the coupling portion is a radially deformable annular structure.

93. The limiting mechanism of claim 92, wherein the coupling portion is an axially undulating wave structure with opposite peaks and valleys, and the first ends of the rods are connected to corresponding peaks.

94. The limiting mechanism of claim 90, wherein the coupling portion and the deformable portion are formed in one piece.

95. The limiting mechanism of claim 94, wherein the coupling portion and the deformable portion are formed in one piece by cutting a tube.

96. The limiting mechanism of claim 90, wherein the second ends of the rods are configured to move independently.

97. The limiting mechanism of claim 90, wherein the rods tend to extend helically around an axis of the coupling portion.

98. The limiting mechanism of claim 90, wherein the rods have a number in a range of 4 to 10.

99. The limiting mechanism of claim 90, wherein the second end of the rod has a smooth outer contour and/or is coated with a protective layer.

100. The limiting mechanism of claim 90, wherein a portion of the rod close to the second end is bent radially inwardly.

101. The limiting mechanism of claim 90, wherein a portion of the rod close to the second end has a lower radial stiffness than the rest of the rod.

102. A limiting mechanism for an interventional delivery system, which is configured to be installed in a balloon body, wherein the balloon body has a folded state and an inflated state, and in the folded state, the balloon body has a plurality of folds; the limiting mechanism comprises:
a coupling portion for connecting with the interventional delivery system, the coupling portion having an axial direction and corresponding circumferential and radial directions; and
a deformable portion comprising a plurality of rods arranged in sequence in the circumferential direction, wherein the rods are elastically deformable, and have a compressed state suitable for interventional delivery and an expanded state, and in the compressed state, the rods are placed in corresponding folds.

103. The limiting mechanism of claim 102, wherein the balloon body is wrapped in a first direction in the circumferential direction in the folded state, and a helical direction of the rods is the same as the first direction.

104. The limiting mechanism of claim 102, wherein when the balloon body is in the folded state, a second end of at least one rod is not lower than a prosthetic implant in the radial direction.

105. A limiting mechanism for an interventional delivery system, comprising:
a coupling portion for connecting with the interventional delivery system, the coupling portion having an axial direction and corresponding circumferential and radial directions; and
a deformable portion comprising a plurality of rods arranged in sequence in the circumferential direction, each of which has:
a first end connected to the coupling portion; and
a second end located on one axial side of the coupling portion relative to the first end; wherein the rods are elastically deformable, and have a compressed state suitable for interventional delivery and an expanded state, and the second ends of the rods diverge outward in the radial direction of the coupling portion in the expanded state relative to the compressed state; the rods are made of memory alloy, distances from the second end of the rod to an axis of the coupling portion in the expanded and compressed states are R1 and R2 respectively, and R1 : R2 = 2 - 20 : 1.

106. A balloon device for delivering a prosthetic implant, comprising an inner shaft and a balloon body located around the inner shaft, wherein the inner shaft is provided with a limiting mechanism of any one of claims 90 to 105 located inside the balloon body.

107. An interventional delivery system based on balloon expansion for delivering a prosthetic implant, wherein the interventional delivery system comprises a balloon device and an outer sheath, the balloon device comprises an inner shaft and a balloon body located around the inner shaft, a plurality of rods arranged in sequence in a circumferential direction are provided at a distal end of the inner shaft, the rods are elastically deformable and have a compressed state suitable for interventional delivery and an expanded state, and each rod has:
a first end connected to the inner shaft; and
a second end which is located at one side of the first end and is suspended relative to the inner shaft, wherein the second ends of the rods diverge outward in a radial direction in the expanded state relative to the compressed state;
wherein the prosthetic implant is configured to be radially compressed and installed on the balloon body in a loaded state and located at a proximal end of the rods, and the rods act on the balloon body so that an outer peripheral surface of the balloon body is not lower than an outer peripheral surface of a distal end of the prosthetic implant.

108. The interventional delivery system of claim 107, wherein the rods act on the balloon body so that the outer peripheral surface of the balloon body is roughly flush with the outer peripheral surface of the distal end of the prosthetic implant.

109. An interventional delivery system based on balloon expansion for delivering a prosthetic implant, wherein the interventional delivery system has opposite distal and proximal ends and an axial direction extending between the proximal end and the distal end, and the interventional delivery system comprises:
a balloon device, comprising an inner shaft and a balloon body located around the inner shaft, wherein a first limiting mechanism located inside the balloon body is installed at a distal end of the inner shaft;
an intermediate shaft, which is located outside the inner shaft, wherein a second limiting mechanism outside the balloon body is installed at a distal end of the intermediate shaft, the second limiting mechanism is located proximal to the first limiting mechanism, the prosthetic implant is configured to be radially compressed and installed on the balloon body in a loaded state and located between the first limiting mechanism and the second limiting mechanism, and the two limiting mechanisms are configured to limit axial movement of the prosthetic implant relative to the balloon body; and
an outer sheath which is slidably fitted over the balloon device, wherein the outer sheath has an extreme position of proximal movement on its sliding path, and in this extreme position, the first limiting mechanism and the prosthetic implant are exposed outside the outer sheath, while the second limiting mechanism remains inside the outer sheath.

110. An interventional delivery system based on balloon expansion for delivering a prosthetic implant, wherein the interventional delivery system comprises:
a balloon device, comprising an inner shaft and a balloon body located around the inner shaft, wherein a plurality of rods arranged in sequence in a circumferential direction inside the balloon body are installed at a distal end of the inner shaft, the rods are elastically deformable and have a compressed state suitable for interventional delivery and an expanded state, each rod has a first end connected to the inner shaft and a second end located on one side of the first end and suspended relative to the inner shaft, the second ends of the rods diverge outward in a radial direction in the expanded state relative to the compressed state, and the prosthetic implant is configured to be radially compressed and installed on the balloon body in a loaded state and is located at a proximal end of the rods;
an adjustment wire for releasably fixing the prosthetic implant on the balloon body, wherein one end of the adjustment wire is fixed to the balloon body, while the other end passes through the prosthetic implant and has a locking eyelet; and
a locking wire having a locked state and an unlocked state, wherein in the locked state, the locking wire passes through the locking eyelet to restrict the prosthetic implant, and in the unlocked state, the locking wire is disengaged from the locking eyelet to release the prosthetic implant.

111. The interventional delivery system of any one of claims 107 to 110, wherein the interventional delivery system further comprises a control handle, and the control handle is connected to and configured to control the balloon device.

112. The interventional delivery system of claim 111, wherein a fluid guide member is provided in the balloon body and is located around the inner shaft, and the fluid guide member defines a diversion channel that communicates a distal end and a proximal end of the balloon body.

113. A balloon device for delivering a prosthetic heart valve, comprising:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body; and
a limiting mechanism which is configured to limit movement of the prosthetic heart valve in the axial direction at least when the prosthetic heart valve is mounted on the balloon body in a radially compressed state, wherein the limiting mechanism specifically comprises:
a limiting body comprising a plurality of rods, which generally extend from a first end in the axial direction to a second end in the axial direction, converging at the first end and the second end respectively to form a hollow cage-like structure.

114. The balloon device of claim 113, wherein the plurality of rods are configured to form a side wall of the cage-like structure, and gaps between two adjacent rods on the side wall form hollow areas.

115. The balloon device of claim 114, wherein the hollow areas comprise main hollow areas and auxiliary hollow areas, and a span of the main hollow area in the axial direction is greater than a span of the auxiliary hollow area in the axial direction.

116. The balloon device of claim 115, wherein the main hollow area spans the first end and the second end.

117. The balloon device of claim 115, wherein the cage-like structure has an outward expansion part, the outward expansion part has a maximum outer diameter of the cage-like structure, and the main hollow area extends across the outward expansion part.

118. The balloon device of claim 115, wherein the main hollow areas and the auxiliary hollow areas are distributed in sequence at intervals in a circumferential direction.

119. The balloon device of claim 113, wherein the limiting mechanism is located inside or outside the balloon body.

120. The balloon device of claim 113, wherein at least one of the catheter body and the balloon body is directly fixed to the limiting mechanism, or is indirectly fixed to the limiting mechanism through an intermediate piece.

121. The balloon device of claim 113, wherein the balloon device further comprises an inner shaft which extends through the catheter body and the balloon body;
the limiting mechanism further comprises at least one coupling member which is fixed to the inner shaft and connected to the converging portion of the limiting body.

122. The balloon device of claim 121, wherein the coupling member is located inside or outside the limiting body.

123. The balloon device of claim 121, wherein the coupling member is located at at least one end of the limiting body in the axial direction.

124. The balloon device of claim 121, wherein the cage-like structure has an outward expansion part, the outward expansion part has a maximum outer diameter of the cage-like structure, and an outer diameter ratio of the outward expansion part to the coupling member is in a range of 2 - 4 : 1.

125. The balloon device of claim 124, wherein the outer diameter ratio of the outward expansion part to the coupling member is 3 : 1.

126. The balloon device of claim 121, wherein the coupling member is in a radially compressible tubular shape.

127. The balloon device of claim 126, wherein the coupling member has a wave structure that undulates in the axial direction or has a deformable mesh structure.

128. The balloon device of claim 126, wherein the limiting mechanism is formed in one piece by cutting a tube, the tube has an initial outer diameter D1, the coupling member has an outer diameter D2, and the outer diameter D2 of at least one coupling member is smaller than D1.

129. The balloon device of claim 121, wherein there are two coupling members, the cage-like structure is formed by a plurality of rods, all the rods define a side wall of the cage-like structure, and all the rods extend from one coupling member to the other coupling member and have at least one branch on their extension path, or intersect with adjacent rods.

130. The balloon device of claim 129, wherein the cage-like structure has an outward expansion part, the outward expansion part has a maximum outer diameter of the cage-like structure, and a plurality of junction points of two rods are distributed at intervals in a circumferential direction of the outward expansion part.

131. The balloon device of claim 115, wherein the main hollow areas have a number in a range of 4 to 12.

132. The balloon device of claim 131, wherein the number of the main hollow areas is in a range of 6 to 8.

133. The balloon device of claim 115, wherein the main hollow areas have the same shape and are evenly arranged in a circumferential direction.

134. The balloon device of claim 115, wherein the main hollow area is elongated.

135. The balloon device of claim 115, wherein in the axial direction, a length of the main hollow area is at least 40% of a total length of the limiting body.

136. The balloon device of claim 135, wherein the length of the main hollow area is at least 60% of the total length of the limiting body.

137. The balloon device of claim 116, wherein a length of the main hollow area is 75% to 100% of a total length of the limiting body.

138. The balloon device of claim 115, wherein the main hollow area is diamond-shaped.

139. The balloon device of claim 117, wherein in the axial direction, the main hollow area extends to both sides of the outward expansion part, and the extended length is at least 20% of a total length of the limiting body.

140. The balloon device of claim 139, wherein the auxiliary hollow area avoids the outward expansion part.

141. The balloon device of claim 113, wherein one end of the limiting body is the first end facing the prosthetic heart valve when in use, and the other end is the opposite second end, and both ends of the limiting body are converged toward a central axis of the limiting body.

142. The balloon device of claim 141, wherein the rods extend from the first end to the second end to form a sphere or an ellipsoid.

143. The balloon device of claim 141, wherein a cross-section of the ellipsoid is an elliptical surface, and a major axis of the elliptical surface is consistent with the axial direction.

144. The balloon device of claim 113, wherein the cage-like structure is formed by a plurality of rods, and the rods extend from the first end to the second end to form a cone.

145. The balloon device of claim 144, wherein the cage-like structure has an outward expansion part, the outward expansion part has a maximum outer diameter of the cage-like structure, and the cone comprises a first cone and a second cone which are located on both sides or on a same side of the outward expansion part.

146. The balloon device of claim 145, wherein the first cone and the second cone are located on both sides of the outward expansion part, and the first cone and the second cone gradually converge in shape from the outward expansion part, with different converging trends.

147. The balloon device of claim 146, wherein one end of the limiting body is the first end facing the prosthetic heart valve when in use, and the other end is the opposite second end, wherein the first cone is connected to the first end, and the first cone has a steeper converging trend.

148. The balloon device of claim 146, wherein the first cone and the second cone intersect at the outward expansion part, and an angle at the intersection is in a range of 20 to 120 degrees.

149. The balloon device of claim 145, wherein the first cone and the second cone are located on the same side of the outward expansion part, one end of the limiting body is the first end facing the prosthetic heart valve when in use, and the other end is the opposite second end, wherein the first cone is connected to the first end, and the first cone is converged in a direction away from the first end.

150. A balloon device for delivering a prosthetic heart valve, comprising:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body; and
a limiting mechanism which is configured to limit movement of the prosthetic heart valve in the axial direction at least when the prosthetic heart valve is mounted on the balloon body in a radially compressed state, wherein the limiting mechanism specifically comprises:
a limiting body which is generally a cage-like structure, wherein a part of the cage-like structure is an outward expansion part, and the outward expansion part has the largest radial expansion relative to other parts of the cage-like structure; in the axial direction, one end of the limiting body is a first end facing the prosthetic heart valve when in use, and the other end is an opposite second end, and the outward expansion part is adjacent to the first end of the limiting body.

151. The balloon device of claim 150, wherein the balloon device further comprises an inner shaft which extends through the catheter body and the balloon body;
the limiting mechanism further comprises a coupling member fixed to the inner shaft and connected to the limiting body.

152. The balloon device of claim 151, wherein a concave area for accommodating an end of the prosthetic heart valve is defined between a side of the limiting body on the first end and the coupling member on that side.

153. A balloon device for delivering a prosthetic heart valve, comprising:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body; and
a limiting mechanism which is configured to limit movement of the prosthetic heart valve in the axial direction at least when the prosthetic heart valve is mounted on the balloon body in a radially compressed state, wherein the limiting mechanism specifically comprises:
a limiting body comprising a plurality of rods that generally form a cage-like structure, wherein a part of the cage-like structure is an outward expansion part, and the outward expansion part has the largest radial expansion relative to other parts of the cage-like structure; on a cross-section of the outward expansion part, the rods are arranged at intervals in a circumferential direction of the limiting mechanism, enclosing an area that approximates a polygon.

154. The balloon device of claim 153, wherein the polygon is a regular polygon with 4 to 12 sides.

155. The balloon device of claim 154, wherein the regular polygon has 6 to 8 sides.

156. A balloon device for delivering a prosthetic heart valve, having opposed distal and proximal ends and comprising:
a catheter body, with an extension direction of the catheter body defined as an axial direction;
a balloon body in communication with the catheter body, wherein the balloon body is configured to receive fluid from the catheter body to transition from a folded state to an inflated state, thereby enabling the prosthetic heart valve to expand from a radially compressed state to a radially expanded state based on changes of the balloon body;
an inner shaft which extends through the catheter body and the balloon body; and
a limiting mechanism which is configured to limit movement of the prosthetic heart valve in the axial direction at least when the prosthetic heart valve is mounted on the balloon body in a radially compressed state, wherein the limiting mechanism specifically comprises:
a limiting body which is generally a cage-like structure;
a guide tube connected to the limiting body, wherein the guide tube and the limiting body are formed in one piece by cutting a tube made of shape memory alloy; and
a coupling member connected to the inner shaft and at least one of the limiting body and the guide tube.

157. The balloon device of claim 156, wherein the guide tube is connected to a proximal side of the limiting body.

158. The balloon device of claim 156, wherein there are two limiting bodies, which are respectively connected to a proximal side and a distal side of the guide tube.

159. The balloon device of claim 158, wherein the cage-like structures of the two limiting bodies are independent of each other.

160. The balloon device of claim 156, wherein the balloon body comprises a first part, a middle part and a second part from a distal end to a proximal end, and the middle part is configured for loading and fixing the prosthetic heart valve;
the guide tube is arranged in a radial gap between the inner shaft and the balloon body, and a guide channel for fluid to pass through is defined between the guide tube and the inner shaft.

161. The balloon device of claim 156, wherein a first coupling member and a second coupling member located at a distal end of the first coupling member are provided and located around the inner shaft, at least one coupling member is fixed to the inner shaft, and a distal part of the balloon body wraps the second coupling member.

162. The balloon device of claim 161, wherein the second coupling member is located at a proximal end of the guide tube and is adjacent to a connection of the balloon body and the catheter body.

163. The balloon device of claim 156, wherein a proximal end of the guide tube has a diameter-reduced section and is connected to the corresponding coupling member through the diameter-reduced section.

164. The balloon device of claim 163, wherein the guide tube has a fluid inlet, and the fluid inlet is defined on the diameter-reduced section and/or a circumferential wall of the guide tube.

165. The balloon device of claim 156, wherein a circumferential wall of the guide tube has a hollowed-out gap.

166. The balloon device of claim 156, wherein the limiting mechanism is formed in one piece by cutting a tube; the tube has an initial outer diameter D1, the guide tube has an outer diameter D1, the coupling member has an outer diameter D2, and the outer diameter D2 of at least one coupling member is smaller than D1.

167. A transcatheter implant intervention system, comprising a prosthetic implant and a balloon device of any one of claims 106, 113 to 166, wherein the balloon device comprises:
a catheter body;
a balloon body in communication with the catheter body; and
a limiting mechanism, wherein the prosthetic implant is radially compressed and installed on the balloon body in a loaded state and is blocked by the limiting mechanism in an axial direction.

168. The transcatheter implant intervention system of claim 167, wherein the transcatheter implant intervention system further comprises an outer sheath that is slidably fitted over the balloon device, and a limiting body of the limiting mechanism has a loaded state, an intermediate state, and an expanded state;
wherein in the loaded state, the limiting body is located inside the balloon body and the outer sheath, and is subjected to radial forces from both the balloon body and the outer sheath;
in the intermediate state, the limiting body is free from a radial constraint from the outer sheath and is subjected to the radial force from the balloon body; and
in the expanded state, the balloon body is inflated and the limiting body is expanded.

169. The transcatheter implant intervention system of claim 167, wherein the prosthetic implant is a prosthetic heart valve.

170. A prosthetic heart valve, comprising a first stent and a plurality of leaflets, wherein the first stent is a radially deformable cylindrical structure, an interior of the first stent is a blood flow channel, and the leaflets cooperate with each other to control the blood flow channel;
the first stent has a cell structure formed by struts, the first stent is in a straight cylindrical shape, the cell structure comprises diamond-shaped cells, an end of the first stent has an eyelet structure protruding from the end, and the eyelet structure is formed by struts at the end; the first stent has an expanded state and a compressed state, wherein in the compressed state, the eyelet structure protrudes from the end by a distance of L1, and in the expanded state, the eyelet structure protrudes from the end by a distance of L2, and L1 > L2 ≥ 0.

171. The prosthetic heart valve of claim 170, wherein the cell structure of the first stent forms a plurality of nodes at the end; in the expanded state, the eyelet structure is basically flush with surrounding nodes, and in the compressed state, the eyelet structure is higher than the surrounding nodes.

172. The prosthetic heart valve of claim 170, wherein L1 : L2 is in a range of 1.2 to 1.6 : 1.

173. The prosthetic heart valve of claim 170, wherein L1 is in a range of 0.6 to 0.8 mm, and L2 is in a range of 0.4 to 0.6 mm.

174. The prosthetic heart valve of claim 170, wherein in the expanded state, the eyelet structure is generally an arc-shaped structure, and in the compressed state, the eyelet structure approximates a circular structure.

175. The prosthetic heart valve of claim 170, wherein the prosthetic heart valve is a prosthetic pulmonary valve or a prosthetic aortic valve.

176. The prosthetic heart valve of claim 170, wherein in the expanded state, the struts at the end are bent into an arc shape and define an edge of the eyelet structure.

177. The prosthetic heart valve of claim 170, wherein the first stent has eyelet structures at both axial ends, and circumferential positions of the eyelet structures at both ends are the same or staggered.

178. The prosthetic heart valve of claim 170, wherein a cell with the eyelet structure is a first cell, a cell adjacent to the first cell in a circumferential direction is a second cell, and within the first cell, the struts around the eyelet structure comprise:
an arc segment defining an edge of the eyelet structure; and
connecting segments extending from two ends of the arc segment in opposite directions to nodes on both sides of the first cell in the circumferential direction.

179. The prosthetic heart valve of claim 178, wherein an angle between the connecting segments on both sides of the arc segment is A1, an inner angle of a node located at an axial end of the second cell is A2, and A1 is greater than A2 in the expanded state.

180. The prosthetic heart valve of claim 178, wherein in the expanded state, a central angle corresponding to the arc segment is in a range of 150 to 210 degrees.

181. The prosthetic heart valve of claim 178, wherein in the expanded state, a span of the arc segment in the circumferential direction of the first stent accounts for 1/4 to 1/2 of a span of the first cell.

182. The prosthetic heart valve of claim 178, wherein in the first cell, a strength of the struts around the eyelet structure is less than that of other struts of the first cell.

183. The prosthetic heart valve of claim 170, wherein the cell structure of the first stent comprises a plurality of rows of cells, and two axially adjacent rows are staggered in a circumferential direction and share some struts;
in the expanded state, an inner angle of a node of each cell is in a range of 75 to 105 degrees.

184. The prosthetic heart valve of claim 170, wherein the prosthetic heart valve further comprises a skirt connected to and surrounded by an inner wall of the first stent, and one axial side of the skirt is connected to the leaflets.

185. The prosthetic heart valve of claim 184, wherein the prosthetic heart valve further comprises a sealing member arranged in a circumferential direction of the first stent, the sealing member is fixed to the skirt and protrudes from a radial inner side of the first stent to a radial outer side of the first stent via corresponding cells.

186. The prosthetic heart valve of claim 185, wherein the skirt and the sealing member are formed in one piece or separate pieces which are fixed by bonding, and both the skirt and the sealing member are made of PU material.

187. The prosthetic heart valve of claim 185, wherein the sealing member comprises:
a plurality of sealing blocks arranged in the circumferential direction of the first stent, wherein the sealing blocks are made of foam material and embedded in corresponding cells.

188. The prosthetic heart valve of claim 187, wherein the sealing member further comprises:
an inner lining film, wherein the plurality of sealing blocks are fixed on one side of the inner lining film, and the inner lining film and the plurality of sealing blocks are made of a same material.

189. The prosthetic heart valve of claim 188, wherein the skirt is made of biological pericardial material, and the inner lining film is sutured to the skirt.

190. The prosthetic heart valve of claim 170, wherein at least parts of the stent are configured as support bars, in a circumferential direction of the stent, at least one side of the support bar is provided with a recessed area, the leaflets are fixed to the support bars by sutures, and all knots of the sutures are located in the corresponding recessed areas.

191. The prosthetic heart valve of claim 190, wherein the leaflet comprises:
a leaflet body having a transverse direction and a longitudinal direction in a flattened state, wherein an outer edge of the leaflet body comprises a free edge and a fixed edge distributed on two opposite sides in the longitudinal direction, the plurality of leaflets are fixed to the stent through their respective fixed edges, and the free edges of the plurality of leaflets cooperate with each other to control the blood flow channel in the prosthetic heart valve; and
commissure tabs arranged at two opposite sides of the leaflet body in the transverse direction, wherein the free edge and the fixed edge meet at the commissure tabs on corresponding sides;
wherein commissure tabs between two adjacent leaflets are connected to each other through first sutures, and are also connected to the corresponding support bars through second sutures and third sutures, wherein all knots of the second sutures and the third sutures are located in corresponding recessed areas.

192. The prosthetic heart valve of claim 191, wherein a joint of the commissure tabs between two adjacent leaflets connected through the first sutures contacts a radial inner side of the support bar;
the commissure tabs between two adjacent leaflets cooperate with each other to wrap the support bar.

193. The prosthetic heart valve of claim 192, wherein the knots of the second sutures are wrapped by the commissure tabs, and the knots of the third sutures are exposed from the commissure tabs.

194. The prosthetic heart valve of claim 190, wherein two axial sides of the stent are opposite inflow and outflow sides, and a row of hexagonal cells is arranged circumferentially on the outflow side, with the support bar serving as a common side between two adjacent cells.

195. The prosthetic heart valve of claim 190, wherein an extension direction of the support bar is consistent with an axial direction of the stent.

196. The prosthetic heart valve of claim 190, wherein two sides of the support bar are respectively in an M-shape, and an undulating structure of the M-shape forms the recessed area;
the M-shapes on the two sides are symmetrically arranged or arranged in a staggered manner in an axial direction.

197. A transcatheter implant system, comprising a balloon device and the prosthetic heart valve of any one of claims 170 to 196, wherein the balloon device comprises:
a catheter body;
a balloon body in communication with the catheter body, wherein the balloon body is configured to receive fluid from the catheter body to transition from a folded state to an inflated state, thereby enabling the prosthetic heart valve to expand from a compressed state to an expanded state based on changes of the balloon body; and
an inner shaft which extends through the catheter body and the balloon body, and is provided with a guide head at a distal end thereof;
wherein the transcatheter implant system further comprises a locking wire structure for limiting an axial position of the prosthetic heart valve relative to the balloon body, and the locking wire is configured to pass through the eyelet structure of the first stent.

198. The transcatheter implant system of claim 197, wherein the locking wire structure comprises:
a first adjustment wire for releasably fixing the prosthetic implant on the balloon body, wherein one end of the first adjustment wire is fixed to the catheter body, and the other end passes through a corresponding eyelet structure and has a first locking eyelet;
a second adjustment wire, one end of which is connected to the guide head and the other end of which passes through a corresponding eyelet structure and has a second locking eyelet; and
a locking wire having a locked state and an unlocked state, wherein in the locked state, the locking wire passes through the locking eyelets to restrict the prosthetic implant, and in the unlocked state, the locking wire is disengaged from the locking eyelets to release the prosthetic implant;
wherein the guide head is provided with an insertion hole, and an end of the locking wire extends into the insertion hole in the locked state.

199. The transcatheter implant system of claim 198, wherein there are at least two junctions between the adjustment wires and the locking wire, at least one junction limits movement of the prosthetic implant in a distal direction, and at least one junction limits movement of the prosthetic implant in a proximal direction.

200. The transcatheter implant system of claim 199, wherein the first adjustment wire and the second adjustment wire are matched in axial length so that the first stent is maintained in a middle of the balloon body.

201. The transcatheter implant system of claim 197, wherein the balloon device is the balloon device of any one of claims 106, 113 to 166.

202. A prosthetic heart valve assembly, comprising:
a prosthetic heart valve comprising a first stent and a plurality of leaflets, wherein the first stent is a radially deformable cylindrical structure, an interior of the first stent is a blood flow channel, and the leaflets cooperate with each other to control the blood flow channel; and
an anchoring stent, which is fitted over a radial outer side of the prosthetic heart valve after assembly, wherein the prosthetic heart valve and the anchoring stent both have an expanded state and a compressed state, and in the expanded state, the prosthetic heart valve and the anchoring stent contact each other;
wherein the anchoring stent has a cell structure formed by struts, and the cell structure comprises in an axial direction:
a middle part comprising two rows of fishbone-shaped cells; and
end parts comprising two rows of diamond-shaped cells, which are respectively located on both sides of the middle part in the axial direction;
wherein the anchoring stent also has a pre-expanded state before assembly, the anchoring stent has a first diameter in the pre-expanded state, and has a second diameter in the expanded state, and the second diameter is greater than the first diameter.

203. The prosthetic heart valve assembly of claim 202, wherein in the expanded state, an axial length of the anchoring stent is greater than a length of the first stent.

204. The prosthetic heart valve assembly of claim 202, wherein the first stent is located in a middle of the anchoring stent in the axial direction.

205. The prosthetic heart valve assembly of claim 202, wherein the fishbone-shaped cell is a concave hexagon with opposite convex tips and concave tails, the concave tails of two rows of fishbone-shaped cells are symmetrical and face each other, while the convex tips face outward;
the two rows of diamond-shaped cells are respectively located on two sides of the middle part in the axial direction, and each diamond-shaped cell is located between the convex tips of two adjacent fishbone-shaped cells.

206. The prosthetic heart valve assembly of claim 202, wherein the first stent does not axially exceed the middle part.

207. The prosthetic heart valve assembly of claim 202, wherein the first stent has a denser cell than that of the anchoring stent.

208. The prosthetic heart valve assembly of claim 202, wherein a number of circumferential cells of the first stent is 1.2 to 2 times a number of circumferential cells of the anchoring stent.

209. The prosthetic heart valve assembly of claim 202, wherein within a diamond-shaped cell at an end of the anchoring stent, two struts facing away from the middle part have lower strength than two struts shared with the middle part.

210. The prosthetic heart valve assembly of claim 202, wherein the prosthetic heart valve and the anchoring stent are both configured to be deployed based on balloon expansion.

211. The prosthetic heart valve assembly of claim 202, wherein the prosthetic heart valve is configured to be deployed based on balloon expansion, and the anchoring stent is configured to be deployed based on self-expansion.

212. The prosthetic heart valve assembly of claim 202, wherein the prosthetic heart valve is the prosthetic heart valve of any one of claims 170 to 196.

213. A transcatheter implant system, comprising:
the prosthetic heart valve assembly of any one of claims 202 to 212; and
a first delivery system having a first balloon body for delivering and expanding the prosthetic heart valve.

214. The transcatheter implant system of claim 213, further comprising:
a second delivery system for expanding the anchoring stent.

215. The transcatheter implant system of claim 214, wherein the first delivery system and the second delivery system are configured to implement interventional delivery via a same or different interventional paths.

216. The transcatheter implant system of claim 213, wherein the first delivery system further comprises a second balloon body for expanding the anchoring stent; or
the first delivery system further comprises an outer sheath, the anchoring stent is accommodated in the outer sheath in the compressed state, and the anchoring stent is configured to be deployed based on self-expansion.

217. The transcatheter implant system of claim 216, wherein the first balloon body and the second balloon body are respectively provided with fluid channels, the two balloon bodies are arranged in an axial direction and are respectively loaded with the prosthetic heart valve and the anchoring stent, and the anchoring stent to be deployed first is located at a distal end of the prosthetic heart valve.

218. The transcatheter implant system of claim 213, wherein a distal end or both ends of the prosthetic heart valve are each provided with a limiting mechanism in the first balloon body;
the limiting mechanism is configured to limit movement of the prosthetic heart valve in an axial direction at least when the prosthetic heart valve is mounted on the first balloon body in a radially compressed state.

219. The transcatheter implant system of claim 217, wherein a distal end or both ends of the anchoring stent are each provided with a limiting mechanism in the second balloon body;
the limiting mechanism is configured to limit movement of the anchoring stent in an axial direction at least when the anchoring stent is installed on the corresponding balloon body in a radially compressed state.

220. The transcatheter implant system of claim 213, wherein the first stent of the prosthetic heart valve and/or the anchoring stent have eyelet structures protruding from two axial ends respectively, the transcatheter implant system is configured with a locking wire structure for the prosthetic heart valve and/or the anchoring stent, and the locking wire structure comprises:
a first adjustment wire, one end of which is fixed to the delivery system, and the other end of which passes through a corresponding eyelet structure at a corresponding end and has a first locking eyelet;
a second adjustment wire, one end of which is fixed to the delivery system, and the other end of which passes through a corresponding eyelet structure at a corresponding end and has a second locking eyelet; and
a first locking wire having a locked state and an unlocked state, wherein in the locked state, the locking wire passes through the locking eyelets to restrict the prosthetic heart valve and/or the anchoring stent, and in the unlocked state, the locking wire is disengaged from the locking eyelets to release the prosthetic heart valve and/or the anchoring stent.

221. A releasing method for a prosthetic heart valve assembly from an interventional delivery system, wherein the prosthetic heart valve assembly comprises a prosthetic heart valve and an anchoring stent, both of which have an expanded state and a compressed state;
the interventional delivery system comprises at least an inner shaft for interventional delivery, and both the prosthetic heart valve and the anchoring stent are connected to the inner shaft in a compressed state;
the releasing method comprises steps of:
step S100, releasing a connection between the anchoring stent and the inner shaft, allowing the anchoring stent to enter the expanded state;
step S200, delivering the prosthetic heart valve to an interior of the anchoring stent; and
step S300, releasing a connection between the prosthetic heart valve and the inner shaft, allowing the prosthetic heart valve to enter the expanded state and fit tightly against an inner wall of the anchoring stent.

222. The releasing method of claim 221, wherein the interventional delivery system further comprises an outer sheath, the outer sheath is slidably mounted around the inner shaft, and the anchoring stent is connected to the inner shaft and enclosed by the outer sheath;
the step of releasing the connection between the anchoring stent and the inner shaft comprises first moving the outer sheath and the inner shaft relative to each other to expose the anchoring stent from the outer sheath, thereby allowing the anchoring stent to enter the expanded state via self-expansion.

223. The releasing method of claim 221, wherein one inner shaft is provided, and the interventional delivery system further comprises:
two balloon bodies arranged in sequence along the inner shaft, wherein the prosthetic heart valve and the anchoring stent are respectively connected to outer peripheries of the corresponding balloon bodies; and
a locking wire for simultaneously limiting axial positions of the prosthetic heart valve and the anchoring stent relative to the corresponding balloon bodies;
wherein the step of releasing the connection between the anchoring stent and the inner shaft further comprises moving the locking wire before or after inflating the balloon body corresponding to the anchoring stent to release axial limitation on the anchoring stent while maintaining axial limitation on the prosthetic heart valve; and
the step of releasing the connection between the prosthetic heart valve and the inner shaft further comprises moving the locking wire along the original movement direction before or after inflating the balloon body corresponding to the prosthetic heart valve to release axial limitation on the prosthetic heart valve.

224. The releasing method of claim 221, wherein the interventional delivery system further comprises a deflection assembly, and the deflection assembly can at least act on the inner shaft to change an orientation of a distal end of the inner shaft;
before the prosthetic heart valve assembly is separated from the inner shaft, the inner shaft, under an action of the deflection assembly, has a smooth bent section or at least two smooth bent sections, and the two bent sections have opposite curvatures.

225. The releasing method of claim 224, wherein a portion of the inner shaft corresponding to the bent section is made of a metal cutting tube.
